# EUROPEAN PATENT APPLICATION

(11) **EP 4 520 333 A1**
(43) Date of publication of application: **12.03.2025**
(21) Application number: 23195633.5
(22) Date of filing: 06.09.2023
(51) Int. Cl.: A61K 31/5377, A61K 31/675, A61K 45/06, A61P 35/00, A61P 37/00, C07K 16/28

(54) **SYK INHIBITORS FOR USE IN THE TREATMENT OF IMMUNE CHECKPOINT INHIBITION INDUCED ADVERSE EVENTS**

(71) Applicant: Albert-Ludwigs-Universität Freiburg, 79098 Freiburg (DE)
(72) Inventor: Zeiser, Robert, 79100 Freiburg (DE)
(74) Representative: Hertin und Partner Rechts- und Patentanwälte PartG mbB

(57) **Abstract**

The present invention is directed to an inhibitor of Spleen tyrosine kinase (Syk-inhibitor) for use in the treatment or prevention of immune checkpoint inhibition (ICI)-induced immune-related adverse events (irAEs) in a subject, wherein irAEs preferably comprise or consist of central nervous system immune-related adverse events (CNS-irAEs), preferably wherein the checkpoint blockade therapy comprises administration of at least one antibody, such as an anti- programmed cell death-1 (PD-1) antibody.

## Description

The present invention lies in the field of biochemistry and targeted kinase inhibitor therapy, particularly in the field of treatment and prevention of cancer immunotherapy-induced immune-related adverse events.

The present invention is directed to an inhibitor of Spleen tyrosine kinase (Syk-inhibitor) for use in the treatment or prevention of immune checkpoint inhibition (ICI)-induced immune-related adverse events (irAEs) in a subject, wherein irAEs preferably comprise or consist of central nervous system immune-related adverse events (CNS-irAEs), preferably wherein the checkpoint blockade therapy comprises administration of at least one antibody, such as an anti- programmed cell death-1 (PD-1) antibody.

### BACKGROUND OF THE INVENTION

Immune checkpoint inhibition (ICI) has proven clinical benefit in the treatment of multiple cancers. Immune checkpoint blockade increases antitumor immunity by blocking negative regulators of immunity. Anti PD-1 therapy for the treatment of peripheral malignancies improves anti-tumor efficacy through systemic immune activation, predominantly via augmenting T cell responses (15). However, broad evidence suggests that expression of PD-1 is not exclusive to T cells, but is also expressed by macrophages and microglia, as the resident CNS immune cell (16). Microglia play a central role in multiple inflammatory diseases affecting the CNS, such as multiple sclerosis (17) and graft-versus-host disease (GVHD) (18). In GVHD, microglia-derived TNF-mediated neurotoxic effects led to neurological deficits in mice (18). Blockade of PD-1 has been shown to mediate a pro-inflammatory phenotype in myeloid cells evidenced by upregulation of MHC class II (19, 20). In a mouse model of ischemic stroke, deficiency of PD-1 promoted microglia activation and exacerbated cognitive deficits. These findings support the hypothesis that blockade of PD-1 signaling can contribute to disease progression (21).

Immunotherapeutic approaches that rely on immune checkpoint inhibition (ICI) targeting programmed cell death protein/ligand 1 (PD-1/PD-L1) have significantly improved the treatment efficacy for multiple cancer entities. However, patients treated with ICIs develop different types of autoimmune-like diseases summarized as immune related adverse events (irAEs) (1, 2).

Treatment of irAEs includes cessation of ICI-therapy and/or administration of glucocorticosteroids, adapted to the severity of the irAEs (Haanen et al., 2017). These measures incur the risk that lack of ICI-therapy and immunosuppression reduce anti-tumor immune responses. So far, no randomized trial has analyzed the impact of glucocorticosteroids on the anti-tumor immune effect in ICI treated patients and retrospective data are controversial (Luo et al., 2017; Horvat et al., 2015; Marschner et al., 2020). Additionally, glucocorticosteroids cause major side-effects including hyperglycemia, fluid retention, psychological disorders, iatrogenic adrenal insufficiency if the glucocorticoids are tapered too fast and infectious complication (Postow et al., 2018).

Also, second line therapies such as TNF-antagonists may interfere with anti-tumor effects as TNF was shown to be required for anti-tumor effects in hematological malignancies (Schmaltz et al., 2003) and TNF-blockade for autoimmunity was connected to a numerically increased risk for lymphoproliferative cancers (Askling et al., 2009; Setoguchi et al., 2006) and non-melanoma skin cancers (Chakravarty e al., 2005; Wolfe et al., 2007). A retrospective study that analyzed 790 patients with advanced melanoma being treated with ICI, reported a rate of serious infections of 13.5% in the subgroup of patients who received either glucocorticoids or infliximab (TNF antagonist; Del Castillo et al., 2016). A recent clinical study showed that TNF-blockade along with immunotherapy was connected to a non-response to ICI in 50% of the patients (Montfort at al., 2020).

In summary, patients developing severe irAEs (grade 3 and 4) after ICI are currently treated with a complete and durable interruption of the immunotherapy and the administration of glucocorticosteroids (Haanen et al., 2017). Both interventions may have negative effects on the anti-tumor immune response. Currently no prospective randomized trial supports the use of glucocorticosteroids or second line therapies such as the frequently used approaches with mycophenolate mofetile (MMF), TNF antagonists or cyclosporine A. Novel approaches that are active against irAE are lacking.

Neurological irAE symptoms are observed in 1-12 % of all patients receiving ICI (1, 3, 4), however, the actual incidence may be higher due to difficulties in distinguishing central nervous system immune-related adverse events (CNS-irAEs) versus other drug toxicity or infection of the CNS (5-7). The neuroinflammation caused by ICI has unpredictable onset and may present with heterogeneous manifestations, neuromuscular complications and even demyelinating disorders resembling multiple sclerosis. Current treatment options for CNS-irAEs include corticosteroids, mycophenolate, cyclosporine A and etanercept (12-14). The response rates are variable and therefore, a targeted therapy approach based on the biology of ICI-induced neuroinflammation is highly desirable, particularly with the expanding use of immunotherapies (9). Currently, the understanding of CNS-irAEs is based on morphological and immunohistochemical characterization of the CNS in post-mortem analyses on case series of encephalitis (4), while mechanistic studies on CNS-irAEs in preclinical models are missing.

Therefore, novel therapeutic approaches that interfere with irAEs, particularly CNS-irAEs, after ICI without diminishing anti-tumor effects and which induce little systemic side effects are an unmet medical need.

### SUMMARY OF THE INVENTION

In light of the prior art the technical problem underlying the present invention is to provide improved therapeutic strategies for the treatment of ICI-induced irAEs, particularly CNS-irAEs, which do not interfere with ICI mediated anti-tumor effects and cause less side effects than state of the art glucocorticoid treatment.

This problem is solved by the features of the independent claims. Preferred embodiments of the present invention are provided by the dependent claims.

In one aspect the present invention relates to an inhibitor of Spleen tyrosine kinase (Syk-inhibitor) for use in the treatment or prevention of immune checkpoint inhibition (ICI)-induced immune-related adverse events (irAEs) in a subject.

In embodiments irAEs comprise or consist of central nervous system immune-related adverse events (CNS-irAEs).

In embodiments the neuroinflammation caused by ICI ((CNS-)irAESs) may present with heterogeneous manifestations, comprising, without limitation thereto, encephalopathy, hypophysitis, meningitis, encephalitis associated with fever, headaches, tremors, altered mental status, cognitive impairment, and seizures (4, 8). Neuromuscular complications including Guillain-Barré syndrome, neuropathies, myasthenia gravis have also been reported (9). In addition, demyelinating disorders resembling multiple sclerosis have been reported in some ICI patients, where CD4 T cells isolated from the brains of patients demonstrated responses to myelin.

In embodiments the subject is suffering from / experiencing one or more of: cognitive deficits, neurotoxicity, pathological headaches, seizures, encephalopathy, hypophysitis, meningitis, encephalitis associated with fever, altered mental status, cognitive impairment, Guillain-Barré syndrome, neuropathies, peripheral neuropathies, myasthenia gravis and demyelinating disorders resembling multiple sclerosis, increased blood brain barrier (BBB) permeability, CNS infiltration of immune cells, partial or complete loss of eye sight, partial or complete loss of hearing, partial or complete loss of sensation, partial or complete paralysis, muscle weakness, memory loss, numbness in one or more limbs, tremor, paresis, confusion or disorientation, toxicities of cytokine release syndrome (CRS), neurotoxicity and/or neurocognitive defects.

The inventors analyzed the role of microglia in central nervous system immune-related adverse events (CNS-irAEs) using multiple mouse models and correlated the findings with post mortem analysis of the CNS of patients. The inventors surprisingly observed strong microglia activation, including morphological changes and MHC-II upregulation, in tumor-free and melanoma-bearing mice after immune checkpoint inhibition (ICI) therapy, exampled by anti-PD-1 treatment. Unbiased kinase profiling revealed activation of the Spleen Tyrosine Kinase (Syk) immune checkpoint inhibition (ICI) therapy, exampled by anti-PD-1 treatment. Depletion of microglia, but not T cells, improved neurocognitive function after immune checkpoint inhibition (ICI) therapy (anti-PD-1 treatment). Similarly, genetic deletion of microglia using Cx3cr1 creER:Csf1 rfl/fl mice resulted in improved neurocognitive activity. Unexpectedly pharmacological Syk inhibition reduced MHC-II expression on microglia and improved neurocognitive activity, without blocking the anti-melanoma effects of immune checkpoint inhibition (ICI) therapy (anti-PD-1 treatment). Clinical trials on pharmacological Syk inhibition for autoimmunity (22) and lymphoid malignancies (23, 24) support Syk inhibition as a viable strategy for treating CNS-irAEs. The inventors further confirmed their surprising finding that immune checkpoint inhibition (ICI) therapy (anti-PD-1 treatment) induces microglia activation in a patient cohort. The experimental data presented in the examples herein evidence the contribution of microglia to CNS-irAEs and identifies Syk inhibition as a therapeutic approach to interfere with this severe complication after immune checkpoint inhibition (ICI) immunotherapy (anti-PD-1-immunotherapy).

In embodiments the subject is receiving immune checkpoint blockade therapy.

In embodiments the checkpoint blockade therapy comprises administration of at least one antibody.

In embodiments the at least one antibody is specific for a protein selected from the group comprising cytotoxic T-lymphocyte antigen 4 (CTLA-4), programmed cell death-1 (PD-1) and/or programmed cell death ligand-1 (PD-L1) or Lymphocyte-activation gene 3 (LAG-3).

In embodiments the at least one antibody is specific for (targeting / directed against) programmed cell death -1 (PD-1).

Immunotherapy targeting PD-1 has the advantage that it restores immune function in the tumor microenvironment. In general, immune checkpoint inhibition (ICI) therapy may comprise a treatment with anti-programmed cell death receptor (PD-1) monoclonal antibodies, such as nivolumab (Opdivo - Bristol Myers Squibb), Cemiplimab (Libtayo Regeneron), Pembrolizumab (Keytruda, MK-3475, Merck), Pidilizumab (CT-011, Cure Tech), Dostarlimab (Jemperli, GlaxoSmithKline), Retifanlimab (Zynyz, Incyte) or BMS-936559 (Bristol Myers Squibb).

In embodiments an antibody that is specific for a certain protein is an antibody targeting said protein, namely an antibody that (specifically) binds to said protein, wherein said protein is a target antigen of said antibody.

In general, anti-PD-1 immunotherapy is one type of immune checkpoint inhibition (ICI) therapy that induces significant anti-tumor immune responses with clinical success. However, the success of this therapy is complicated by irAEs which include neurological symptoms in 1-12% of all treated patients (1, 3, 4, 8). The pathophysiology of irAEs affecting the CNS following anti-PD-1 treatment is not well understood. In particular, the role of microglia in this setting remains elusive. The inventors therefore aimed to unravel the underlying mechanisms of ICI therapy-induced irAEs, particularly CNS irAEs, to develop novel therapeutic interventions for these conditions.

In their research the inventors observed that mice treated with anti-PD-1 treatment showed morphological signs of microglia activation as well as upregulation of MHC-II and CSF-1R on microglia. Comparable morphological changes and marker expression have been previously reported for microglia in CNS autoimmune inflammation (17) and CNS alloimmune inflammation (18), indicating microglia activation. The inventors surprisingly observed that anti-PD-1 immunotherapy in a non-tumor setting can mediate neuroinflammation via direct drug interactions. This is in contrast to other immunotherapy induced neuroinflammatory settings, such as that observed with chimeric antigen receptor (CAR) T cells, which is dependent on the presence of tumor cells (30, 31). Microglia may be inflammatory or regulatory depending on the disease (29, 32). Single-cell profiling revealed that microglia subsets are distinctly affected in neuroinflammation (33). The inventors identified herein a disease-promoting role for microglia in anti-PD-1 induced CNS-irAEs. Depletion of microglia, in contrast to depletion of T-cells, B-cells or NK cells, reduced anti-PD-1-induced neurocognitive deficits. This finding is consistent with the inventor's observation that microglia activation by anti-PD-1 occurred independent of T cells in vitro. Further, the observation that anti-PD-1 directly activates microglia is consistent with a previous report showing that PD-1 deficiency in myeloid cells leads to their activation (34). Additionally, myeloid cell-specific PD-1 ablation induced increased T effector memory cell function, thereby mediating improved antitumor protection, despite preserved PD-1 expression in T cells (34). This further supports the role for PD-1 in myeloid cell activation, including microglia (as shown herein).

In embodiments the at least one antibody is specific for (targeting) cytotoxic T-lymphocyte antigen 4 (CTLA-4).

In embodiments the at least one antibody is specific for (targeting)programmed cell death ligand-1 (PD-L1).

In embodiments the at least one antibody is specific for (targeting) Lymphocyte-activation gene 3 (LAG-3).

In embodiments, the subject is or has been receiving immunosuppressive drugs, such as steroids, corticosteroid, cyclosporine and/or anti-TNF antibodies and/or is refractory to immunosuppressive drugs.

The inventor's in-depth analysis described in the examples herein enabled the identification of Syk inhibition, for example using Entospletinib or any other suitable Sky inhibitor, as a novel targeted approach for irAEs, particularly CNS-irAEs. Entospletinib is presently in clinical trials for acute myeloid leukemia (ClinicalTrials.gov Identifier: NCT05020665) and chronic lymphocytic leukemia (38, 39) which substantially facilitates necessary investigational steps required for clinical use. The surprising finding that Syk inhibition resulted in reduced microglia activation and improved neurocognitive activity, without blocking the anti-melanoma effects of anti-PD-1 antibody therapy, is highly clinically relevant, since SYK inhibitors are already approved and available in the clinics and are found herein to be a suitable therapeutic option for patients with anti-PD-1 induced neurocognitive dysfunction. The inventor's observation that Syk is a target for anti-PD-1 induced CNS-irAEs is consistent with a recent characterization of gain-of-function variants in SYK that cause immune dysregulation and systemic inflammation in humans and mice (40). In line with the present finding that CSF-1R inhibition reduced disease activity of CNS-irAEs, this treatment was also shown to attenuate experimental autoimmune encephalomyelitis (41). Additionally, depletion of microglia improves cognitive function following cranial irradiation (42). The depletion of microglia is not associated with major toxicity as microglia can self-renew from residual microglia after acute depletion (43).

The use of SYKi according to the invention is particularly supported by the present finding of increased microglial activation in anti-PD-1 treated patients as compared to control patients. Additionally, reduced TMEM119 and P2RY12 expression and a weak CD163 signal in microglia of anti-PD-1 treated patients further support microglia activation (44).

Kinase-inhibitor treatment was tested against neuroinflammation before in mouse models in the context of bacterial-induced inflammation using LPS, a bacterial protein inducing strong systemic inflammation (Kim et al., 2022). However, the use of kinase inhibition as a treatment option of ICI-induced irAEs or CNS-irAEs, which is a very different condition with a divergent mechanism than systemic, or pathogen-induced inflammation, has not been analyzed or considered before.

In summary, the present invention provides a novel and promising therapeutic approach considering the herein shown disease-promoting role for microglia in anti-PD-1 treatment-induced neurotoxicity and Syk as a mediator of CNS-irAEs. Mechanistically, Syk-inhibition interfered with PD-1, PD-L1 and MHC-II expression thereby reducing the severity of CNS-irAEs in mice. The present examples provide a strong rationale for a pharmacological Syk inhibitor-based approach to interfere with neurocognitive impairment as a severe complication after ICI therapy, particularly anti-PD-1 immunotherapy.

In embodiments the Syk-inhibitor is administered at least 1 hour before, during and/or at least one hour after administration of immune checkpoint blockade therapy.

In embodiments the administration of the Syk-inhibitor does not interfere with the anti-tumor response induced by the immune checkpoint blockade therapy, which is preferably an anti-PD1 treatment, due to the tissue specific adiponectin induction.

In embodiments of the SYK inhibitor for use according to the invention, the immunotherapy is an antibody-based cancer therapy. Non-limiting examples of antibody-based cancer therapy are monoclonal antibodies, (monoclonal) antibodies against programmed cell death-1 (PD-1), CTLA-4, ligand-1 (PD-L1) or Lymphocyte-activation gene 3 (LAG-3). or any other monoclonal antibody. In the context of the present invention any antibody-based cancer therapy is imagined.

The inventors found that mice treated with anti-PD-1 treatment showed morphological signs of microglia activation as well as upregulation of MHC-II and CSF-1R on microglia. As outlined in more detail below, the inventors thereupon could show that the depletion of microglia, in contrast to depletion of T-cells, B-cells or NK cells, reduced anti-PD-1-induced neurocognitive deficits. This finding is consistent with the inventor's observation that microglia activation by anti-PD-1 occurred independent of T cells in vitro. Further, the observation that anti-PD-1 directly activates microglia.

Hence, in embodiments the Syk-inhibitor reduces microglial activation in said subject. In other words, in embodiments the administration of a SYK inhibitor according to the invention to a subject (experiencing ICI-induced (CNS-) irAEs) reduces microglial activation in the subject.

In embodiments the microglial activation comprises microglia exhibiting, when compared to microglia cells of a healthy control subject, an elevated expression of MHC-II, F4/80, CD80 and/or CSF-1R, and/or morphological changes comprising reduced filament length, reduced number of dendrites, branching points, terminal dendrite points and/or Syk activation (phosphorylation).

The activation of Syk kinase may be induced through phosphorylation of one or more tyrosine, threonine and/or serine residues of the kinase.

In embodiments the Syk-inhibitor has a direct effect on the microglia, independent of CNS infiltration by T cells, B cells, macrophages, oligodendrocytes and/or NK cells, in said subject.

In embodiments of the method according to the invention an effective amount of an inhibitor of SYK is used to inhibit or reduce microglial activation compared to that which would occur in the absence of the inhibitor.

In embodiments inhibiting the activation of microglia by inhibiting SYK (by administering a SYK inhibitor as described herein) reduces the severity of ICI-induced (CNS-) irAEs and /or one or more of the symptoms/conditions described in the following.

In embodiments the inhibitor is administered to a subject upon occurrence of one or more of cognitive deficits, neurotoxicity, seizures, pathological headaches, increased blood brain barrier (BBB) permeability, CNS infiltration of immune cells, partial or complete loss of eye sight, partial or complete loss of hearing, partial or complete loss of sensation, partial or complete paralysis, muscle weakness, memory loss, numbness in one or more limbs, tremor, paresis, confusion or disorientation, toxicities of cytokine release syndrome (CRS), neurotoxicity and/or neurocognitive defects in said subject.

In embodiments the inhibitor is administered to a subject upon occurrence of one or more of encephalopathy, hypophysitis, meningitis, encephalitis associated with fever, tremors, altered mental status, cognitive impairment, Guillain-Barré syndrome, neuropathies, peripheral neuropathies, myasthenia gravis, demyelinating disorders resembling multiple sclerosis, cognitive deficits, neurotoxicity, seizures, pathological headaches, increased blood brain barrier (BBB) permeability, CNS infiltration of immune cells, partial or complete loss of eye sight, partial or complete loss of hearing, partial or complete loss of sensation, partial or complete paralysis, muscle weakness, memory loss, numbness in one or more limbs, tremor, paresis, confusion or disorientation, toxicities of cytokine release syndrome (CRS), neurotoxicity and/or neurocognitive defects in said subject.

In embodiments the administration of the SYK inhibitor reduces (microglia-mediated) neuroinflammation, preferably of the central nervous system (CNS).

In embodiments the inhibitor is selected from fostamatinib (R788), entospletinib (GS-9973), lanraplenib (GS-9876), cerdulatinib, dasatinib, entrectinib, erlotinib, paclitaxel, imatinib, nilvadipine, fostamatinib, TAK-659, R211, R406, R343, R112, BAY-61-3606, GSK143, SKI-G-618, SKI-O-85, ER-27319, YM193306, RO9021 and P505-15 (PRT062607).

In embodiments the inhibitor is selected from fostamatinib (R788), entospletinib (GS-9973), lanraplenib (GS-9876), cerdulatinib, TAK-659, R211, R406, R343, R112, BAY-61-3606, GSK143, SKI-G-618, SKI-O-85, ER-27319, YM193306, RO9021 and P505-15 (PRT062607).

The Syk-inhibitor for use according to any one of the preceding claims, wherein the inhibitor is selected from fostamatinib (R788), entospletinib (GS-9973), cerdulatinib (PRT062070), lanraplenib (GS-9876) and TAK-659.

In embodiments the Syk-inhibitor for use according to the invention is selected from fostamatinib, entospletinib, lanraplenib, dasatinib, entrectinib, erlotinib, paclitaxel, imatinib, nilvadipine, cerdulatinib, and fostamatinib.

In embodiments of a SYK inhibitor for use according to the invention, the SYK inhibitor is administered at least 1 minute, 5, 10, 15, 20, 30, 45 or 60 minutes, or at least 1 hour, 2, 3, 4, 5, 6, 7, 8, 9, 10, 11, 12, 24, 36, or 48 hours, or at least 1, 2, 3, 4, 5, 6, 7, 14, 21, 28, 30 days or at least 1, 2, 3, 4, 5, 6, 7, 8, 9, 10, 11, 12, 13, 14, 15, 16, 20, 24, or 52 weeks after administration of ICI (immuno)therapy, e.g., anti-PD-1 immunotherapy.

In embodiments of a SYK inhibitor for use according to the invention, the SYK inhibitor is administered at least 1 minute, 5, 10, 15, 20, 30, 45 or 60 minutes, or at least 1 hour, 2, 3, 4, 5, 6, 7, 8, 9, 10, 11, 12, 24, 36, or 48 hours, or at least 1, 2, 3, 4, 5, 6, 7, 14, 21, 28, 30 days after occurrence of one or more symptoms associated with (CNS-)irAEs, e.g., as disclosed herein.

Preferably an inhibitor of SYK is administered at least once daily for at least 1, 2, 3, 4, 5, 6, 7, 14, 21, 28, 30 days upon occurrence of one or more symptoms associated with (CNS-)irAEs, e.g., as disclosed herein.

In embodiments the inhibitor of SYK, is administered at least once to the subject, preferably at once per day. Accordingly, the subject may receive more than one dose of the inhibitor of SYK, wherein preferably not more than two doses per day are administered. In further embodiments, the subject receives at least 2, 3, 4 or 5 dosages of the inhibitor of SYK. In embodiments, the inhibitor of SYK is administered to the subject at least every 8 weeks, preferably every 2-4 weeks.

In embodiments of an inhibitor of SYK for use according to the invention, the subject suffers from cancer. In embodiments of an inhibitor of SYK for use according to the invention, the subject has been receiving, will be receiving (within minutes, hours or days) or is currently receiving a treatment against cancer. In embodiments said treatment is an immune checkpoint inhibition (ICI) therapy, preferably an immunotherapy, for example, anti-PD-1 immunotherapy.

In embodiments of an inhibitor of SYK for use according to the invention, the cancer is selected from the group comprising haematological malignancies, leukemia, lymphoma, myeloma, sarcoma, neuroblastoma, breast cancer, ovarian cancer, lung cancer, melanoma, carcinoma or another cancer, e.g., as disclosed herein, that is treatable by immune checkpoint inhibition therapy (ICI), preferably immunotherapy, for example, anti-PD-1 immunotherapy, e.g., such as any cancer disclosed herein.

In another aspect the present invention relates to a method of treatment of a mammalian subject suffering from immune checkpoint inhibition (ICI)-induced immune-related adverse events (irAEs), preferably ICI-induced CNS-irAEs, comprising administering an therapeutically effective amount of a SYK inhibitor to said subject.

In embodiments the present invention relates to a method of treatment of a mammalian subject suffering a neuroinflammatory disorder associated with a ICI therapy the subject has received or is receiving, wherein the method comprises administering to said subject a SYK inhibitor, preferably administering a therapeutically effective amount of a SYK inhibitor, to said subject.

In further aspect the present invention relates to a method of inhibiting SYK activity present in microglia in a mammalian subject, the method comprising using an effective, preferably a therapeutically effective, amount of an inhibitor of SYK to reduce immune checkpoint inhibition (ICI) therapy-induced (CNS-)irAEs without impairing the anti-cancer effects of the ICI therapy, which is preferably an anti-PD-1 immunotherapy.

In another aspect the present invention relates to the use of a SYK inhibitor for inhibiting microglia activation against ICI-induced (CNS-)irAEs, while preserving anti-tumor effects of the ICI-therapy. In embodiments of the present invention a SYK inhibitor is used for inhibiting microglia activation which is associated with ICI-therapy.

Each optional or preferred feature of the invention that is disclosed or described in the context of one aspect of the invention is herewith also disclosed in the context of the other aspects of the invention described herein.

All features disclosed in the context of the inhibitor of Spleen tyrosine kinase for use in the treatment and/or prevention of immune-related adverse events (irAE) in a subject that has received a checkpoint-inhibitor therapy of the invention also relate to and are herewith disclosed also in the context of the *in vitro* method of the invention, the method of treatment of the invention, and vice versa.

### DETAILED DESCRIPTION OF THE INVENTION

All cited documents of the patent and non-patent literature are hereby incorporated by reference in their entirety.

The present invention is directed to an inhibitor of Spleen tyrosine kinase (Syk-inhibitor) for use in the treatment or prevention of immune checkpoint inhibition (ICI)-induced immune-related adverse events (irAEs) in a subject, wherein irAEs preferably comprise or consist of central nervous system immune-related adverse events (CNS-irAEs), and preferably wherein the checkpoint blockade therapy comprises administration of at least one antibody, such as an anti-programmed cell death-1 (PD-1) antibody.

As used herein, the term "subject' means a human or non-human animal selected for treatment or therapy. The subject or patient, such as the subject in need of treatment or prevention, may be an animal, a vertebrate animal, a mammal, a rodent (e.g. a guinea pig, a hamster, a rat, a mouse), a murine (e.g. a mouse), a canine (e.g. a dog), a feline (e.g. a cat), an equine (e.g. a horse), a primate, a simian (e.g. a monkey or ape), a monkey (e.g. a marmoset, a baboon), an ape (e. g. gorilla, chimpanzee, orangutan, gibbon), or a human. The meaning of the terms "animal", "mammal", etc. is well known in the art and can, for example, be deduced from Wehner und Gehring (1995; Thieme Verlag). The term subject may also refer to a "patient" and may be used herein interchangeably therewith. In the context of this invention, it is particularly envisaged that animals are to be treated which are economically, agronomically or scientifically important. Preferably, the subject/patient is a mammal. More preferably, the subject/patient is a human.

In embodiments of the present invention, the subject that has received a checkpoint-inhibitor therapy suffers from cancer, such as malignant melanoma or another cancer treatable by checkpoint-inhibitor therapy.

### Tyrosine Kinase inhibition

The Tyrosine-protein kinase Spleen Associated Tyrosine Kinase (SYK) is a non-receptor tyrosine kinase that mediates signal transduction downstream of transmembrane receptors including the B-cell receptor (BCR), as it is widely expressed in hematopoietic cells. SYK comprises a C-terminal tyrosine kinase domain and two N-terminal SH2 domains that mediate the interaction with so called immunoreceptor tyrosine-based activation motifs resulting in the activation of SYK. SYK is also capable of autophosphorylation at Tyrosine-518. After upstream (e.g., BCR) receptor engagement SYK is tyrosine-phosphorylated by other Tyrosine kinases, such as SRC subfamily kinases, which creates binding sites for downstream targets of SYK. Direct downstream phosphorylation targets of SYK comprise VAV1, PLCG1, PI-3-kinase, LCP2 and BLNK. Activated SYK mediates BCR-downstream signaling through PI3K and BTK. SYK was initially to play a role in B-cell receptor (BCR) signaling, it has also been shown to mediate the maturation of B-cells, likely during pro-B to pre-B transition. SYK is also capable of autophosphorylation and is tyrosine-phosphorylated by the LYN kinase upon receptor engagement. Phosphorylation on Ser-297 is very common, it peaks 5 minutes after BCR stimulation. SYK phosphorylation at tyrosine Tyr-323, 348, 352, 630 or Ser-297 activates SYK and/or facilitates binding to different interaction partners.

Exemplary SYK inhibitors comprise, without limitation thereto, fostamatinib (R788), entospletinib (GS-9973), cerdulatinib (PRT062070), TAK-659, Dasatinib, Entrectinib, Erlotinib, Paclitaxel, Imatinib, Nilvadipine, Cerdulatinib, Fostamatinib (R788), ER-27319, BAY 61-3606, GSK143, P505-15 (BIIB057), GS-9973, Syk inhibitor (K00564a) and Syk inhibitor II (K00592a).

Fostamatinib was the first clinically available Syk inhibitor (formerly R788), which can selectively inhibit the BCR signalling pathway. Fostamatinib was developed for refractory B-cell lymphoma and DLBCL treatment (45). Fostamatinib is sold under the brand name Tavalisse (Rigel) as orally formulated tablets comprising fostamatinib disodium hexahydrate (salt), which is a prodrug of the active compound tamatinib (R-406). In some embodiments herein when referring to a SYK kinase inhibitor fostamatinib or a salt thereof, such as fostamatinib disodium hexahydrate, is meant.

Entospletinib (GS-9973) is a second generation compound kinase-selective oral SYK inhibitor that shows favorable selectivity with fewer dose-limiting adverse effects. Compared to fostamatinib, entospletinib possesses high selectivity for Janus kinase 2 (JAK-2), c-KIT, FMS-like tyrosine kinase 3 (FLT 3), RET, and VEGFR2 (45). Entospletinib was developed for oral administration by Kronos Bio Inc. USA.

Lanraplenib (GS-9876) is a highly selective SYK inhibitor that was developed for oral administration.

Cerdulatinib is a dual oral tyrosine kinase inhibitor of SYK and JAK 1/3, showing in vitro activity against B-cell lymphomas.

TAK-659 is a dual heteroaromatic SYK and FLT3 inhibitor. In lymphoma cells TAK-659 was shown to impair the pro-survival, proliferative, chemoresistant, and activation effects promoted by the microenvironment and has been tested in clinical studies against different types of lymphoma and AML (45).

The drug class of SYK inhibitors (SYKi; SYK-i) is an established drug class commonly intended for the treatment of various malignancies, such as cancer and particularly lymphoma and leukemia, as described in Mullard, Nat Rev Drug Discov. 2018 (47), Liu et al., J Hematol Oncol, 2017 (45) and Luis et al., 2022 (46).

The skilled person is familiar with suitable assays for testing the SYK kinase inhibitor activity of a compound. Exemplary assays for testing SYK activity and inhibition may also be purchased from, e.g., Thermo Fisher (Omnia Kinase Assay), BPS Bioscienece (SYK Assay Kit) or Promega (SYK Kinase Enzyme System). For example, in a standard laboratory kinase assay SYK kinase may be incubated with a kinase or stimulant commonly activating SYK or already activated SYK, and at least one SYK kinase downstream target. Alternatively, the assay may also be performed using hematopoietic cells or B-cells, which express SYK, wherein receptor, e.g., BCR, stimulation is performed to activate SYK in said cells. A SYKi may be added to said reaction (or said cells), thereby blocking the kinase activity of SYK and the phosphorylation of downstream targets of SYK. The relative levels of activity (e.g., target phosphorylation capability) and/or site-specific phosphorylation of SYK downstream targets may subsequently be measured via immunoblotting or mass spectrometry.

### ICI treatment

"Immune checkpoints" are an important part of the immune system, as they prevent an excessive immune response, which can lead to autoimmune reactions or generally damage healthy cells of the body. Immune checkpoints are effective when proteins on the surface of T cells (e.g., PD-1) recognize and bind to checkpoint proteins (e.g., PD-L1), on other cells, e.g., tumor cells. The binding of the checkpoint proteins to the partner proteins on T cells can result in T cell-inhibition and prevention of T cells killing the other cells. This mechanism can prevent auto-immune actions of T cells, but also the killing of tumor cells.

"Immune checkpoint inhibition" (ICI)-therapy, "checkpoint-inhibitor therapy" or "immune checkpoint blockade (ICB)", block/inhibit (immune) checkpoint proteins from binding to their partner proteins on T cells, thereby preventing the inhibition of T cell-mediated killing of, e.g., cancer cells. Immune checkpoint blockade therapy can comprise a treatment with anticytotoxic T lymphocyte-associated antigen 4 (CTLA-4) (ipilimumab and tremelimumab), anti-programmed cell death receptor (PD-1) (nivolumab and pembrolizumab), or anti-PD-ligand (PD-L1) (durvalumab, atezolizumab, and avelumab) monoclonal antibodies. ICI uses the infiltration of immune cells into a tumor or tumor environment to initiate and/or revive an effective anti-tumor immune response. The PD-1/PD-L1 signaling pathway is considered crucial for the interactions between immune, stromal and tumor cells. Herein the terms "Immune checkpoint inhibition" (ICI) therapy, "Immune checkpoint inhibitor" (ICI)-therapy, "Immune checkpoint blockade" (ICB) therapy, "checkpoint-inhibitor therapy" or "immune checkpoint inhibitor" therapy may be used interchangeably.

"Immune checkpoint inhibitors" or "ICI" therapy can cause various side effects, which can depend on the patient health at treatment initiation, the type of cancer that is treated, its stage, the drug(s) administered and its/their dose. A group of side effects can be summarized as immune-related adverse events (irAEs).

### Immune-related adverse events (irAE)

As used herein, the term "Immune-related adverse events" (irAE) relates any side effect specific that specifically occur in the context of an immune checkpoint inhibitor treatment, for example in the context of a cancer therapy. IrAEs are unique and are different to adverse events occurring in the context of traditional cancer therapies, and typically have a delayed onset and prolonged duration. IrAEs can involve any organ or system. These effects are frequently low grade and are treatable and reversible; however, some adverse effects can be severe and lead to permanent disorders. Management is primarily based on corticosteroids and other immunomodulatory agents, which should be prescribed carefully to reduce the potential of short-term and long-term complications.

Frequent immune-related adverse events (irAEs) comprise gastrointestinal, endocrine, and dermatologic toxicities. Rare but more severe and potential fatal irAEs can comprise neurotoxicity, cardiotoxicity, and pulmonary toxicity. The observed cytotoxicity is commonly graded according to 4 grades of severity (presently according to the Common Terminology Criteria for Adverse Events, European Society for Medical Oncology guideline and American Society of Clinical Oncology guideline; see e.g., Brahmer et al., 2018, Haanen et al., 2018). Higher grades of irAE cytotoxicity are commonly treated with discontinuation of ICI treatment and/or initiation of glucocorticoid-treatment, e.g., comprising administration of prednisone.

In particular comprised by the term irAEs are symptoms of an autoimmune disease and autoimmune disease, such as (autoimmune) colitis, (autoimmune)hepatitis, (autoimmune)thyroiditis and (autoimmune)dermatitis.

The irAE due to administration of a checkpoint-inhibitor therapy in the present invention is not particularly limited. An irAE is to be understood as an adverse event that is presumed to be immune-related: irAE (see, for example, Drug Interview Form of OPDIVO^{®} Intravenous Infusion 20 mg-100 mg, revised in April 2016 (version 9); Properties and Handling of Adverse Events of an Anti-CTLA-4 Antibody, Ipilimumab (YERVOY^{®}), dated Aug. 24, 2015, issued by the Committee on Safety of New Drugs for Malignant Melanoma of the Japanese Dermatological Association).

Specific embodiments of the irAE of the present invention include interstitial lung disease, myasthenia gravis, myositis, colitis, type 1 diabetes mellitus, hepatic dysfunction (hepatic disorder), pulmonary disorder such as hepatitis (e.g., autoimmune pneumonia), pituitarism such as hypopituitarism or hypophysitis, thyroid dysfunction such as hypothyroidism, neuropathy, nephropathy, encephalitis, adrenal disorder such as adrenal insufficiency, severe skin disorder, venous thromboembolism, infusion reaction, psoriasis, psoriasiform rash, diarrhea (e.g., severe diarrhea), rheumatoid arthritis, uveitis, episcleritis, bursitis, exacerbation of radiodermatitis, chronic inflammatory demyelinating polyneuropathy (hereinafter also referred to as demyelinating polyneuropathy), biliary tract disorder, or nephritis, and pituitarism is preferable.

Central nervous system immune-related adverse events (CNS-irAEs) are neurological irAE symptoms, which occur majorly in the CNS. The neuroinflammation caused by ICI has unpredictable onset and may present with heterogeneous manifestations (symptoms), neuromuscular complications and even demyelinating disorders resembling multiple sclerosis. Current treatment options for CNS-irAEs include corticosteroids, mycophenolate, cyclosporine A and etanercept, but response rates are highly variable. At present, the understanding of CNS-irAEs is based on morphological and immunohistochemical characterization of the CNS in post-mortem analyses on case series of encephalitis, while mechanistic studies on CNS-irAEs in preclinical models are still missing. ICI-related neuroinflammation may present with heterogeneous manifestations (symptoms), including, without being limited thereto, encephalopathy, hypophysitis, meningitis, encephalitis associated with fever, headaches, tremors, altered mental status, cognitive impairment, seizures, Guillain-Barré syndrome, neuropathies, peripheral neuropathies, myasthenia gravis and demyelinating disorders resembling multiple sclerosis.

The immune-related adverse event (IrAE) has been commonly known to occur, for example, after 8 to 12 weeks after administration of ICI. The immune-related adverse event can be evaluated by "grade" or "irAE evaluation". Here, "irAE evaluation" is an index representing the seriousness of a disease, and is represented by 1 to 3. In the irAE evaluation, 1 represents a condition that "does not require additional therapeutic intervention due to irAE", 2 represents a condition that "requires drug intervention, etc., due to irAE, but does not require hospitalization treatment or does not require interruption of treatment", and 3 represents a condition that "requires drug intervention, etc., accompanied by hospitalization due to irAE and requires interruption of treatment". The correspondence between "irAE evaluation" and "grade" varies depending on each disease.

Microglia are a type of neuroglia (glial cells) found throughout the brain and spinal cord where they majorly act as resident macrophage. Microglia act as the first line of active immune defense in the central nervous system (CNS). Microglia are the cells taking care of the overall maintenance of the brain, as they patrol the CNS for damaged or redundant neurons and synapses, and pathogens. In their function as CNS macrophages microglia phagocytize foreign bodies and present respective antigens to T-cells. The resting form of microglia is termed ramified microglia and comprises a small cellular body and elongated branching processes. Opposite to amoeboid (phagocytose) forms of microglia, the cell body of the ramified microglia remains in place while its branches are constantly moving and surveying the surrounding area. Non-phagocytotic activated microglia are considered an in between stadium between ramified microglia and fully active phagocytic microglia. (Re)active microglia may be recognized by expression of Iba1. The most active microglia are activated phagocytic microglia, which are characterized by a large, ameboid shape. Activated phagocytic microglia may interact with neural cells and astrocytes. Activated microglia may also be characterized by an elevated expression of MHC-II, F4/80, CD80 and/or CSF-1R, and/or morphological changes comprising reduced filament length, reduced number of dendrites, branching points, terminal dendrite points and/or Syk activation (phosphorylation).

### Immune checkpoint inhibition

In the context of the present invention, an immune checkpoint inhibitor is a drug that activates immune cells by inhibiting immune checkpoint molecules.

Immune checkpoint molecules are molecules in the immune system that either turn up a signal (co-stimulatory molecules) or turn down a signal provided to immune effector cells. Thus, immune checkpoint molecules can be subdivided into co-stimulatory checkpoint molecules or co-inhibitory checkpoint molecules. Co-stimulatory checkpoint molecules include co-stimulatory lymphocyte receptors, which are lymphocyte surface-receptors that can lead to an activation or stimulation of lymphocyte effector functions. Co-inhibitory checkpoint molecules include co-inhibitory lymphocyte receptors, which are lymphocyte surface-receptors that can lead to an inhibition of lymphocyte effector functions.

The major histocompatibility complex (MHC) is a large locus on vertebrate DNA containing a set of closely linked polymorphic genes that code for cell surface proteins essential for the adaptive immune system. This locus got its name because it was discovered in the study of tissue compatibility upon transplantation. Later studies revealed that tissue rejection due to incompatibility is an experimental artifact masking the real function of MHC molecules - binding an antigen derived from self-proteins or from pathogen and the antigen presentation on the cell surface for recognition by the appropriate T-cells. MHC molecules mediate interactions of leukocytes, with other leukocytes or with body cells. The MHC determines compatibility of donors for organ transplant, as well as one's susceptibility to an autoimmune disease via cross-reacting immunization.

MHC class I molecules are expressed in all nucleated cells and also in platelets-in essence all cells but red blood cells. It presents epitopes to killer T cells, also called cytotoxic T lymphocytes (CTLs). A CTL expresses CD8 receptors, in addition to T-cell receptors (TCR)s. When a CTL's CD8 receptor docks to a MHC class I molecule, if the CTL's TCR fits the epitope within the MHC class I molecule, the CTL triggers the cell to undergo programmed cell death by apoptosis. Thus, MHC class I helps mediate cellular immunity, a primary means to address intracellular pathogens, such as viruses and some bacteria, including bacterial L forms, bacterial genus Mycoplasma, and bacterial genus Rickettsia. In humans, MHC class I comprises HLA-A, HLA-B, and HLA-C molecules.

MHC class II can be conditionally expressed by all cell types, but normally occurs only on "professional" antigen-presenting cells (APCs): macrophages, B cells, and especially dendritic cells (DCs). An APC takes up an antigenic protein, performs antigen processing, and returns a molecular fraction of it-a fraction termed the epitope-and displays it on the APC's surface coupled within an MHC class II molecule (antigen presentation). On the cell's surface, the epitope can be recognized by immunologic structures like T cell receptors (TCRs). The molecular region which binds to the epitope is the paratope. On surfaces of helper T cells are CD4 receptors, as well as TCRs. When a naive helper T cell's CD4 molecule docks to an APC's MHC class II molecule, its TCR can meet and bind the epitope coupled within the MHC class II. This event primes the naive T cell. According to the local milieu, that is, the balance of cytokines secreted by APCs in the microenvironment, the naive helper T cell (Th0) polarizes into either a memory Th cell or an effector Th cell of phenotype either type 1 (Th1), type 2 (Th2), type 17 (Th17), or regulatory/suppressor (Treg), as so far identified, the Th cell's terminal differentiation. MHC class II thus mediates immunization to-or, if APCs polarize Th0 cells principally to Treg cells, immune tolerance of-an antigen. The polarization during primary exposure to an antigen is key in determining a number of chronic diseases, such as inflammatory bowel diseases and asthma, by skewing the immune response that memory Th cells coordinate when their memory recall is triggered upon secondary exposure to similar antigens. B cells express MHC class II to present antigens to Th0, but when their B cell receptors bind matching epitopes, interactions which are not mediated by MHC, these activated B cells secrete soluble immunoglobulins: antibody molecules mediating humoral immunity. Class II MHC molecules are also heterodimers, genes for both α and β subunits are polymorphic and located within MHC class II subregion. Peptide-binding groove of MHC-II molecules is forms by N-terminal domains of both subunits of the heterodimer, α1 and β1, unlike MHC-I molecules, where two domains of the same chain are involved. In addition, both subunits of MHC-II contain transmembrane helix and immunoglobulin domains α2 or β2 that can be recognized by CD4 co-receptors. In this way MHC molecules chaperone which type of lymphocytes may bind to the given antigen with high affinity, since different lymphocytes express different T-Cell Receptor (TCR) co-receptors.

F4/80 is a membrane protein and a mature mouse macrophage and microglial marker. The F4/80 gene is located on mouse chromosome 17 and encodes a polypeptide of 931 amino acids that is processed into a mature protein of 904 amino acids. The N-terminus of the F4/80 polypeptide contains seven tandem EGF-like domains and shows a high degree of homology to proteins such as fibrillin-1 and fibulin-2. The C-terminal sequence demonstrates homology to members of the TM7 superfamily, such as the G protein-coupled receptors for peptide hormones, e.g. parathyroid hormone, calcitonin, and glucagon.

F4/80 is highly and constitutively expressed on most resident tissue macrophages, including the red pulp macrophages in the spleen, microglia in the brain, Kupffer's cells in the liver, and Langerhans' cells in the skin. Furthermore, the expression of F4/80 is tightly regulated according to the physiological status of cells. Thus, the precursor of tissue macrophages, the blood monocyte, is known to express less F4/80 than its mature counterparts.

CD80 a type-1 transmembrane glycoprotein and a member of the B7 family and the immunoglobulin superfamily, which is composed of molecules present in antigen-presenting cells (APCs) and their receptors present on the T cells. Surface CD80 is expressed transiently on activated B cells, macrophages, and dendritic cells. Surprisingly, CD80 is downregulated on most of the cancer cells, and the loss of CD80 alone is sufficient to allow them to escape the attack of the immune system and to impart anergy and apoptosis in tumor-infiltrating T cells. Previous studies have showed that CD80 is the ligand for the proteins CD28 involved in autoregulation and intercellular association and CTLA-4 (expressed on the surface of T cells) Interactions of CD80, CD28, and CTLA-4 play a role in the immunological synapse in T- and B-cell activation, proliferation, and differentiation. The interaction between CD80 and CD28, together with TCR and MHC interaction, induces the activation of nuclear factor-κB (NF-κB), mitogen-activated protein kinase (MAPK), and the calcium-calcineurin pathway, thereby playing a diverse role in manipulating both the innate and the adaptive immune system. CD80 interactions are involved in various diseases including multiple autoimmune diseases and various cancers.

The colony-stimulating factor-1 receptor (CSF-1R), also known as macrophage colony-stimulating factor (M-CSF) receptor, is a transmembrane tyrosine kinase receptor found at the cell surface of microglial cells, bone-marrow-derived macrophages, monocytes, and other cell types (osteoclasts, dendritic cells). The CSF-1/CSF-1R axis regulates cell survival, proliferation, differentiation, and functions of the mononuclear phagocytes. CSF-1R exists as an autoinhibited form and activates through dimerization and auto-phosphorylation of several tyrosine residues initiating a signalling cascade and the internalization of the receptor. The cascade is activated upon binding the endogenous CSF-1 or interleukin-34 (IL-34) and includes PI3K-AKT and AMPK pathways implicated in macrophages differentiation

"Programmed cell death protein 1" (PD-1, or CD279 -cluster of differentiation 279), is a cell surface protein/receptor of B and T cells, which plays a role in the regulation of auto-immune responses by promoting self-tolerance through suppression of T cell inflammatory activity. PD-1 has two ligands, PD-L1 and PD-L2. PD-1 is an immune checkpoint, which guards against autoimmunity through two mechanisms: by promoting apoptosis of antigen-specific T cells in lymph nodes, and by reducing apoptosis in regulatory T cells. An advantage of targeting PD-1 is that it can restore immune function in the tumor microenvironment. Immune checkpoint inhibition (ICI) therapy can comprise a treatment with anti-programmed cell death receptor (PD-1) monoclonal antibodies, such as nivolumab (Opdivo - Bristol Myers Squibb), Pembrolizumab (Keytruda, MK-3475, Merck), Pidilizumab (CT-011, Cure Tech) and BMS-936559 (Bristol Myers Squibb). Both Atezolizumab (MPDL3280A, Roche) and Avelumab (Merck KGaA, Darmstadt, Germany & Pfizer) are monoclonal antibodies directed against PD-L1, the ligand of PD-1.

The transmembrane protein "programmed death-ligand 1" (PD-L1 or, CD274 - cluster of differentiation 274, or B7-H1 - B7 homolog 1) is encoded in human by the CD274 gene. IFN-y stimulation induces PD-L1 expression on T cells, NK cells, macrophages, myeloid dendritic cells, B cells, epithelial cells, and vascular endothelial cells. Binding of PD-L1 to its receptor PD-1, which is present on activated T cells, B cells and myeloid cells, modulates activation or inhibition of the immune cells. The binding of PD-L1 to its receptor PD-1 on T cells initiates a signal that inhibits TCR-mediated activation of T cell proliferation und IL-2 production. Interaction of PD-1 with its ligand PD-L1 can prevent autoimmunity. PD-L1 can be highly expressed in a variety of cancers, particularly lung cancer. Immune checkpoint inhibitor (ICI) therapy can comprise a treatment with anti-PD-ligand (PD-L1) (durvalumab, atezolizumab, and avelumab) monoclonal antibodies.

"Cytotoxic T-lymphocyte-associated protein 4" (CTLA-4, or CTLA4, or CD152 - cluster of differentiation 152), is a protein receptor constitutively expressed in regulatory T cells, which functions as an immune checkpoint that can downregulate a T cell-mediated immune response. CTLA-4 can inactivate T cell effector function when bound to CD80 or CD86 on the surface of antigen-presenting cells. CTLA-4 is a regulatory molecule that represses T cell effector function after initial activation by costimulatory signals. Human monoclonal antibodies targeting CTLA-4 are used in ICI treatment and can increase T-cell function and anti-tumor immune response. Immune checkpoint inhibition (ICI) therapy can comprise a treatment with anticytotoxic T lymphocyte-associated antigen 4 (CTLA-4) (ipilimumab and tremelimumab) monoclonal antibodies.

TIM-3 (T-cell Immunoglobulin domain and Mucin domain 3) expresses on activated human CD4+ T cells and regulates Th1 and Th17 cytokines. TIM-3 acts as a negative regulator of Th1/Th17 function by triggering cell death upon interaction with its ligand, galectin-9.

VISTA (V-domain Ig suppressor of T cell activation) is a protein that is primarily expressed on hematopoietic cells so that consistent expression of VISTA on leukocytes within tumors may allow VISTA blockade to be effective across a broad range of solid tumors.

TIGIT (T cell immunoreceptor with Ig and ITIM domains, also called WUCAM and Vstm3) is an immune receptor present on some T cells and Natural Killer Cells and regulates T cell mediated immunity. TIGIT could bind to CD155 on DCs and macrophages with high affinity and to CD112 with lower affinity.

Co-inhibitory lymphocyte receptors of the present invention comprise PD-1, CTLA-4, TIM-3, LAG-3, TIGIT, BTLA or VISTA. Co-stimulatory lymphocyte receptors of the present invention comprise OX40, 4-1BB, GITR, CD27, HVEM, CD28 or CD40.

An inhibitor of a receptor prevents the generation of a signal by the respective receptor. Accordingly, an inhibitor of a co-inhibitory lymphocyte receptor is a molecule that prevents the activation of the respective receptor and thereby prevents the generation of an inhibitory signal. Conversely, an activator of a receptor induces the generation of a signal by the respective receptor and an activator of a co-stimulatory lymphocyte receptor leads to the generation of a stimulatory signal.

Checkpoint modulators are molecules that interfere with the activity of immune checkpoint molecules, either by stimulating or inhibiting the activity of immune checkpoint molecules.

Soluble checkpoint modulators are molecules that may be able to freely diffuse and, for example, are not bound to a cell membrane or do not remain intracellular.

Checkpoint inhibitors in the sense of the invention comprise lymphocyte-stimulating checkpoint modulators, which are molecules that lead to an activation of lymphocytes, preferably effector T cells, either through activation of a co-stimulatory checkpoint molecule, or through inhibition of a co-inhibitory checkpoint molecules. Furthermore, soluble lymphocyte-stimulating checkpoint modulators include molecules that interfere with the activation of membrane bound immune checkpoint molecules, such as a soluble form of the respective immune checkpoint molecule.

Checkpoint modulators can be naturally occurring molecules or engineered molecules with the respective function interfering with or modulating the activity of an immune checkpoint molecule. Checkpoint modulators include, for example, antibodies or antibody-fragments activity directed against immune checkpoint molecule with agonistic or antagonistic, and ligands or modified ligands of immune checkpoint molecules.

The present invention encompasses both treatment and prophylactic treatment of a subject. A "prophylactic" treatment is a treatment administered to a subject who does not exhibit signs of a disease or exhibits only early signs for the purpose of decreasing the risk of developing pathology. Prophylactic treatment is administered to prevent the progression, acceleration, escalation and/or occurrence of a disease.

"Glucocorticosteroids", which are further called "glucocorticoids", "corticosteroids" or simply "steroids", are the presently most effective anti-inflammatory substances used in the treatment of chronic inflammatory, auto-immune and immune diseases. Glucocorticoids induce immunosuppression, which majorly comprises the decreases in the function and numbers of lymphocytes, of both B cells and T cells. Glucocorticoids are lipophilic hormones, which can be subclassified into glucocorticoids produced in the zona fasciculata of the adrenal cortex, mineralocorticoids produced in the zona glomerulosa, and sex hormones produced in the zona reticularis and to a great extent in the gonads. A variety of synthetic glucocorticoids are available for therapeutic use. Examples of glucocorticoids are cortisol (hydrocortisone), cortisone, prednisone, prednisolone, methylprednisolone and dexamethasone.

### Cancer

In the context of the present invention, the term "cancer" relates to the treatment of all kinds of cancer, independent of whether the cancer is associated with the formation of a solid tumor or whether the cancer cells do not form a solid tumor, as it is the case for certain leukemias.

Cancer comprises a group of diseases that can affect any part of the body and is caused by abnormal cell growth and proliferation. These proliferating cells have the potential to invade the surrounding tissue and/or to spread to other parts of the body where they form metastasis. Worldwide, there were 14 million new cases of cancer and 8.2 million cancer related deaths in 2012 (World Cancer Report 2014). The majority of cancers is caused by environmental signals involving tobacco use, obesity and infections among others, while around 5-10% are genetic cases. Cancers can be classified into subcategories based on the cell of origin. The most common subcategories are carcinomas from epithelial cells, sarcomas from connective tissue and lymphomas and leukemias from hematopoietic cells. Cancer is associated with a high variety of local and systemic symptoms and cannot be cured in many cases. In light of the high number of new cancer patients and cancer related deaths novel treatment strategies are required.

Cancer according to the present invention refers to all types of cancer or neoplasm or malignant tumors found in mammals, including leukemias, sarcomas, melanomas and carcinomas. Either solid tumors and/or liquid tumors (such as leukemia or lymphoma) may be treated.

Melanomas include, but are not limited to include, for example, acral-lentiginous melanoma, amelanotic melanoma, benign juvenile melanoma, Cloudman's melanoma, S91 melanoma, Harding-Passey melanoma, juvenile melanoma, lentigo maligna melanoma, malignant melanoma, nodular melanoma, subungal melanoma, and superficial spreading melanoma.

Leukemias include, but are not limited to acute nonlymphocytic leukemia, chronic lymphocytic leukemia, acute granulocytic leukemia, chronic granulocytic leukemia, acute promyelocytic leukemia, adult T-cell leukemia, aleukemic leukemia, a leukocythemic leukemia, basophylic leukemia, blast cell leukemia, bovine leukemia, chronic myelocytic leukemia, leukemia cutis, embryonal leukemia, eosinophilic leukemia, Gross' leukemia, hairy-cell leukemia, hemoblastic leukemia, hemocytoblastic leukemia, histiocytic leukemia, stem cell leukemia, acute monocytic leukemia, leukopenic leukemia, lymphatic leukemia, lymphoblastic leukemia, lymphocytic leukemia, lymphogenous leukemia, lymphoid leukemia, lymphosarcoma cell leukemia, mast cell leukemia, megakaryocytic leukemia, micromyeloblastic leukemia, monocytic leukemia, myeloblastic leukemia, myelocytic leukemia, myeloid granulocytic leukemia, myelomonocytic leukemia, Naegeli leukemia, plasma cell leukemia, plasmacytic leukemia, promyelocytic leukemia, Rieder cell leukemia, Schilling's leukemia, stem cell leukemia, subleukemic leukemia, and undifferentiated cell leukemia.

Sarcomas include, but are not limited to a chondrosarcoma, fibrosarcoma, lymphosarcoma, melanosarcoma, myxosarcoma, osteosarcoma, Abernethy's sarcoma, adipose sarcoma, liposarcoma, alveolar soft part sarcoma, ameloblastic sarcoma, botryoid sarcoma, chloroma sarcoma, chorio carcinoma, embryonal sarcoma, Wilms' tumor sarcoma, endometrial sarcoma, stromal sarcoma, Ewing's sarcoma, fascial sarcoma, fibroblastic sarcoma, giant cell sarcoma, granulocytic sarcoma, Hodgkin's sarcoma, idiopathic multiple pigmented hemorrhagic sarcoma, immunoblastic sarcoma of B cells, lymphoma, immunoblastic sarcoma of T-cells, Jensen's sarcoma, Kaposi's sarcoma, Kupffer cell sarcoma, angiosarcoma, leukosarcoma, malignant mesenchymoma sarcoma, parosteal sarcoma, reticulocytic sarcoma, Rous sarcoma, serocystic sarcoma, synovial sarcoma, and telangiectaltic sarcoma.

Carcinomas include, but are not limited to acinar carcinoma, acinous carcinoma, adenocystic carcinoma, adenoid cystic carcinoma, carcinoma adenomatosum, carcinoma of adrenal cortex, alveolar carcinoma, alveolar cell carcinoma, basal cell carcinoma, carcinoma basocellulare, basaloid carcinoma, basosquamous cell carcinoma, bronchioalveolar carcinoma, bronchiolar carcinoma, bronchogenic carcinoma, cerebriform carcinoma, cholangiocellular carcinoma, chorionic carcinoma, colloid carcinoma, comedo carcinoma, corpus carcinoma, cribriform carcinoma, carcinoma en cuirasse, carcinoma cutaneum, cylindrical carcinoma, cylindrical cell carcinoma, duct carcinoma, carcinoma durum, embryonal carcinoma, encephaloid carcinoma, epiermoid carcinoma, carcinoma epitheliale adenoides, exophytic carcinoma, carcinoma exulcere, carcinoma fibrosum, gelatiniform carcinoma, gelatinous carcinoma, giant cell carcinoma, carcinoma gigantocellulare, glandular carcinoma, granulosa cell carcinoma, hair-matrix carcinoma, hematoid carcinoma, hepatocellular carcinoma, Hurthle cell carcinoma, hyaline carcinoma, hypernephroid carcinoma, infantile embryonal carcinoma, carcinoma in situ, intraepidermal carcinoma, intraepithelial carcinoma, Krompecher's carcinoma, Kulchitzky-cell carcinoma, large-cell carcinoma, lenticular carcinoma, carcinoma lenticulare, lipomatous carcinoma, lymphoepithelial carcinoma, carcinoma medullare, medullary carcinoma, melanotic carcinoma, carcinoma molle, mucinous carcinoma, carcinoma muciparum, carcinoma mucocellulare, mucoepidermoid carcinoma, carcinoma mucosum, mucous carcinoma, carcinoma myxomatodes, nasopharyngeal carcinoma, oat cell carcinoma, carcinoma ossificans, osteoid carcinoma, papillary carcinoma, periportal carcinoma, preinvasive carcinoma, prickle cell carcinoma, pultaceous carcinoma, renal cell carcinoma of kidney, reserve cell carcinoma, carcinoma sarcomatodes, schneiderian carcinoma, scirrhous carcinoma, carcinoma scroti, signet-ring cell carcinoma, carcinoma simplex, small-cell carcinoma, solanoid carcinoma, spheroidal cell carcinoma, spindle cell carcinoma, carcinoma spongiosum, squamous carcinoma, squamous cell carcinoma, string carcinoma, carcinoma telangiectaticurn, carcinoma telangiectodes, transitional cell carcinoma, carcinoma tuberosum, tuberous carcinoma, verrucous carcinoma, and carcinoma villosum.

Additional cancers include, but are not limited to Hodgkin's Disease, Non-Hodgkin's Lymphoma, multiple myeloma, neuroblastoma, breast cancer, ovarian cancer, lung cancer, rhabdomyosarcoma, primary thrombocytosis, primary macroglobulinemia, small-cell lung tumors, primary brain tumors, stomach cancer, colon cancer, malignant pancreatic insulanoma, malignant carcinoid, urinary bladder cancer, premalignant skin lesions, testicular cancer, lymphomas, thyroid cancer, esophageal cancer, genitourinary tract cancer, malignant hypercalcemia, cervical cancer, endometrial cancer, adrenal cortical cancer, and prostate cancer.

In some embodiments, "tumor" shall include, without limitation, a prostate tumor, a pancreatic tumor, a squamous cell carcinoma, a breast tumor, a melanoma, a basal cell carcinoma, a hepatocellular carcinoma, a choloangiocellular carcinoma, testicular cancer, a neuroblastoma, a glioma or a malignant astrocytic tumor such as glioblastma multiforme, a colorectal tumor, an endometrial carcinoma, a lung carcinoma, an ovarian tumor, a cervical tumor, an osteosarcoma, a rhabdo/leiomyosarcoma, a synovial sarcoma, an angiosarcoma, an Ewing sarcoma/PNET and a malignant lymphoma. These include primary tumors as well as metastatic tumors (both vascularized and non-vascularized).

### Therapeutic application of the invention

### Pharmaceutical combination or combination medicine

According to the present invention, a "pharmaceutical combination" or "combination medicine" is the presence of SYK inhibitor according to the invention, in proximity to one another. In one embodiment, the combination is suitable for combined administration.

In one embodiment, the pharmaceutical combination or combination medication as described herein is characterized in that SYK inhibitor is in a pharmaceutical composition in admixture with a pharmaceutically acceptable carrier, and the photosensitizing agent is in a separate pharmaceutical composition in admixture with a pharmaceutically acceptable carrier, and the blood sample or lymphoid cells are in a separate pharmaceutical composition in admixture with a pharmaceutically acceptable carrier. The combination medication or pharmaceutical combination of the present invention can therefore in some embodiments relate to the presence of two or three or even more separate compositions or dosage forms in proximity to each other. The agents in combination are not required to be present in a single composition.

### Combined administration

According to the present invention, the term "combined administration", otherwise known as coadministration or joint treatment, encompasses in some embodiments the administration of separate formulations of the compounds described herein, whereby treatment may occur within minutes of each other, in the same hour, on the same day, in the same week or in the same month as one another. Alternating administration of two agents is considered as one embodiment of combined administration. Staggered administration is encompassed by the term combined administration, whereby one agent may be administered, followed by the later administration of a second agent, optionally followed by administration of the first agent, again, and so forth. Simultaneous administration of multiple agents is considered as one embodiment of combined administration. Simultaneous administration encompasses in some embodiments, for example the taking of multiple compositions comprising the multiple agents at the same time, e.g., orally by ingesting separate tablets simultaneously or by a combination of oral and intravenous administration. A combination medicament, such as a single formulation comprising multiple agents disclosed herein, and optionally additional anti-cancer and/or immuno-suppressing (e.g., steroids) or immuno-modulating medicaments, may also be used in order to co-administer the various components in a single administration or dosage.

In the context of the present invention combined administration or combined use may comprise in preferred embodiments the administration a SYK inhibitor according to the invention and one or more immuno-suppressing or immuno-modulating medicaments, such as steroids or glucocorticoids, to the subject.

A combined therapy or combined administration of one agent may precede or follow treatment with the other agent to be combined, by intervals ranging from minutes to weeks. In embodiments where the second agent and the first agent are administered separately, one would generally ensure that a significant period of time did not expire between the time of each delivery, such that the first and second agents would still be able to exert an advantageously combined synergistic effect on a treatment site. In such instances, it is contemplated that one would contact the subject with both modalities within about 12-96 hours of each other and, in embodiments preferably, within about 6-48 hours of each other. In some situations, it may be desirable to extend the time period for treatment significantly, however, where several days (2, 3, 4, 5, 6 or 7) to several weeks (1, 2, 3, 4, 5, 6, 7, 8, 9, 10, 11 or 12) lapse between the respective administrations.

In embodiments, the therapeutic agent or combination medication is administered from day 1, 2, 3, 4, 5, 6 or day 7 of the occurrence of (CNS-)irAE symptoms, or for 1, 2, 3, 4, 5, 6, 7, 8, 9, 10, 12, 14, 16, 18, 20, 22, 24, 26, 28, 30, 35, 40, 45 or 50 weeks, or from day 1 to at least 3 weeks, after occurrence of (CNS-)irAE symptoms. In embodiments, the therapeutic agent or combination medication is administered between 1 - 5 days before the administration of the ICI therapy, or for 1, 2, 3, 4, 5, 6 or 7 days or 1, 2, 3, 4, 5, 6, 7, 8, 9, 10, 12, 14, 16, 18, 20, 22, 24, 26, 28, 30, 35, 40, 45 or 50 weeks before the administration of the ICI therapy. In preferred embodiments, the therapeutic agent or combination medication is administered between 1-14 days, more preferable for 1-5 days before the administration of the ICI therapy.

In the meaning of the invention, any form of administration of the multiple agents described herein is encompassed by combined administration, such that a beneficial additional therapeutic effect, preferably a synergistic effect, is achieved through the combined administration of the two medications.

It is understood that substituents and substitution patterns of the compounds described herein can be selected by one of ordinary skill in the art to provide compounds that are chemically stable and that can be readily synthesized by techniques known in the art and further by the methods set forth in this disclosure.

### Pharmaceutical compositions

Another aspect of the disclosure includes "pharmaceutical compositions" prepared for administration to a subject and which include a "therapeutically effective amount" of one or more of the compounds disclosed herein. In certain embodiments, the pharmaceutical compositions are useful for treating immune checkpoint inhibition (ICI)-induced (CNS-)immune related adverse events ((CNS-)irAEs) in a subject. The therapeutically effective amount of a disclosed compound (e.g., agent, substance, blood sample or cells) will depend on the route of administration, the species of subject and the physical characteristics of the subject being treated. Specific factors that can be taken into account include disease severity and stage, weight, diet and concurrent medications. The relationship of these factors to determining a therapeutically effective amount of the disclosed compounds is understood by those of skill in the art.

Pharmaceutical compositions for administration to a subject can include at least one further pharmaceutically acceptable additive such as carriers, thickeners, diluents, buffers, preservatives, surface active agents and the like in addition to the molecule of choice. Pharmaceutical compositions can also include one or more additional active ingredients such as antimicrobial agents, anti-inflammatory agents, anesthetics, and the like. The pharmaceutically acceptable carriers useful for these formulations are conventional. Remington's Pharmaceutical Sciences, by E. W. Martin, Mack Publishing Co., Easton, PA, 19th Edition (1995), describes compositions and formulations suitable for pharmaceutical delivery of the compounds herein disclosed.

In general, the nature of the carrier will depend on the particular mode of administration being employed. For instance, parenteral formulations usually contain injectable fluids that include pharmaceutically and physiologically acceptable fluids such as water, physiological saline, balanced salt solutions, aqueous dextrose, glycerol or the like as a vehicle. For solid compositions (for example, powder, pill, tablet, or capsule forms), conventional non-toxic solid carriers can include, for example, pharmaceutical grades of mannitol, lactose, starch, or magnesium stearate. In addition to biologically-neutral carriers, pharmaceutical compositions to be administered can contain minor amounts of non-toxic auxiliary substances, such as wetting or emulsifying agents, preservatives, and pH buffering agents and the like, for example sodium acetate or sorbitan monolaurate.

The pharmaceutical compositions can be administered by intramuscular, intraocular, subcutaneous, intravenous, intra-arterial, intra-articular, intraperitoneal, intrathecal, intracerebroventricular, or parenteral routes. Optionally, compositions can be administered to subjects by a variety of mucosal administration modes, including by oral, rectal, intraocular, intranasal, intrapulmonary, or transdermal delivery, or by topical delivery to other surfaces.

The compositions according to the invention can alternatively contain as pharmaceutically acceptable carrier substances as required to approximate physiological conditions, such as pH adjusting and buffering agents, tonicity adjusting agents, wetting agents and the like, for example, sodium acetate, sodium lactate, sodium chloride, potassium chloride, calcium chloride, sorbitan monolaurate, and triethanolamine oleate. For solid compositions, conventional nontoxic pharmaceutically acceptable vehicles can be used which include, for example, pharmaceutical grades of mannitol, lactose, starch, magnesium stearate, sodium saccharin, talcum, cellulose, glucose, sucrose, magnesium carbonate, and the like.

In accordance with the treatment methods according to the invention, the compound can be delivered to a subject in a manner consistent with conventional methodologies associated with management of the disorder for which treatment or prevention is sought. In accordance with the disclosure herein, a prophylactically or therapeutically effective amount of the compound and/or other biologically active agent is administered to a subject in need of such treatment for a time and under conditions sufficient to prevent, inhibit, and/or ameliorate a selected disease or condition or one or more symptom(s) thereof.

"Administration of and "administering a" compound should be understood to mean providing a compound, a prodrug of a compound, a combination of at least two compounds or a pharmaceutical composition as described herein. The compound(s) or composition(s) can be administered by another person to the subject (e.g., intravenously) or it can be self-administered by the subject (e.g., tablets).

Dosage can be varied by the attending clinician to maintain a desired concentration at a target site (for example, the lungs or systemic circulation). Higher or lower concentrations can be selected based on the mode of delivery, for example, intravenous or subcutaneous delivery, trans-epidermal, rectal, oral, pulmonary, or intranasal delivery. Dosage can also be adjusted based on the release rate of the administered formulation, for example, of an intrapulmonary spray versus powder, sustained release oral versus injected particulate or transdermal delivery formulations, and so forth.

The present invention also relates to a method of treatment of subjects suffering from the medical conditions disclosed herein. The method of treatment comprises preferably the administration of a therapeutically effective amount of a compound disclosed herein to a subject in need thereof.

A "therapeutically effective amount" refers to a quantity of a specified compound sufficient to achieve a desired effect in a subject being treated with said compound. For example, this may be the amount of a compound disclosed herein useful in treating a condition of immune checkpoint inhibition (ICI)-induced (CNS-)immune related adverse events ((CNS-)irAEs) in a subject. The therapeutically effective amount or diagnostically effective amount of a compound will be dependent on the subject being treated, the severity of the affliction, and the manner of administration of the therapeutic composition. Dosage regimens can be adjusted to provide an optimum prophylactic or therapeutic response. A therapeutically effective amount is also one in which any toxic or detrimental side effects of the compound and/or other biologically active agent is outweighed in clinical terms by therapeutically beneficial effects.

A non-limiting range for a therapeutically effective amount of a SYK inhibitor and optionally glucocorticoids, and/or other biologically active agent within the methods and formulations of the disclosure is about 0.001 mg/kg body weight to 50 mg/kg body weight, 0.01 mg/kg body weight to about 20 mg/kg body weight, such as about 0.05 mg kg to about 5 mg/kg body weight, or about 0.2 mg/kg to about 2 mg/kg body weight.

As used herein, the term "approximately" or "about" is used to describe and account for small variations. For example, the term may refer to less than or equal to 10, such as less than or equal down to 1, when appropriate, also the term may refer to more than or equal to 10, such as more than or equal up to 100 or more, when appropriate. It is to be understood that range format is used for the sake of simplicity and brevity and is to be flexibly understood to include numeric values expressly stated as boundaries of a range, encompassing each numeric value and subranges.

The instant disclosure also includes kits, packages and multi-container units containing the herein described pharmaceutical compositions, active ingredients, and/or means for administering the same for use in the prevention and treatment of diseases and other conditions in mammalian subjects.

### FIGURES

The invention is further described by the following figures. These are not intended to limit the scope of the invention but represent preferred embodiments of aspects of the invention provided for greater illustration of the invention described herein.

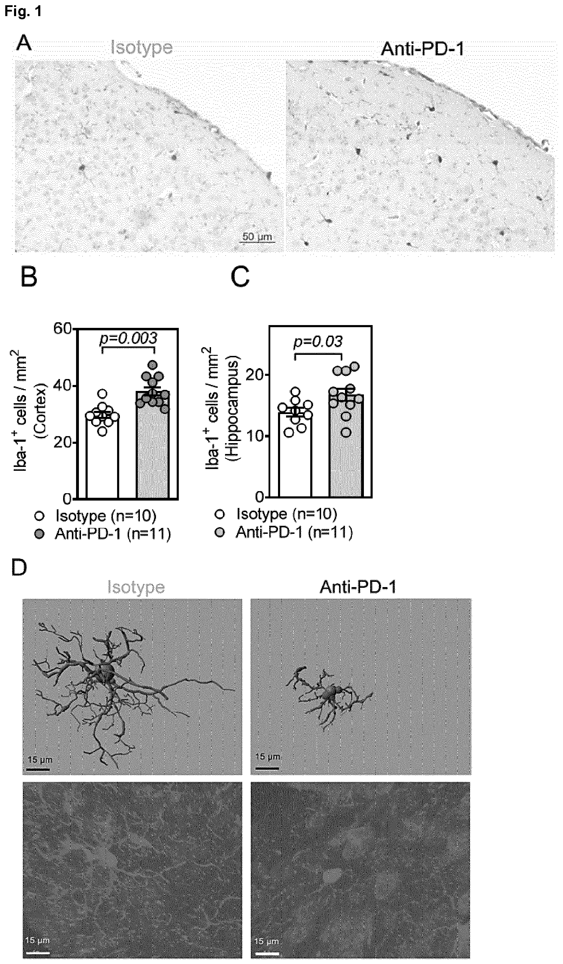

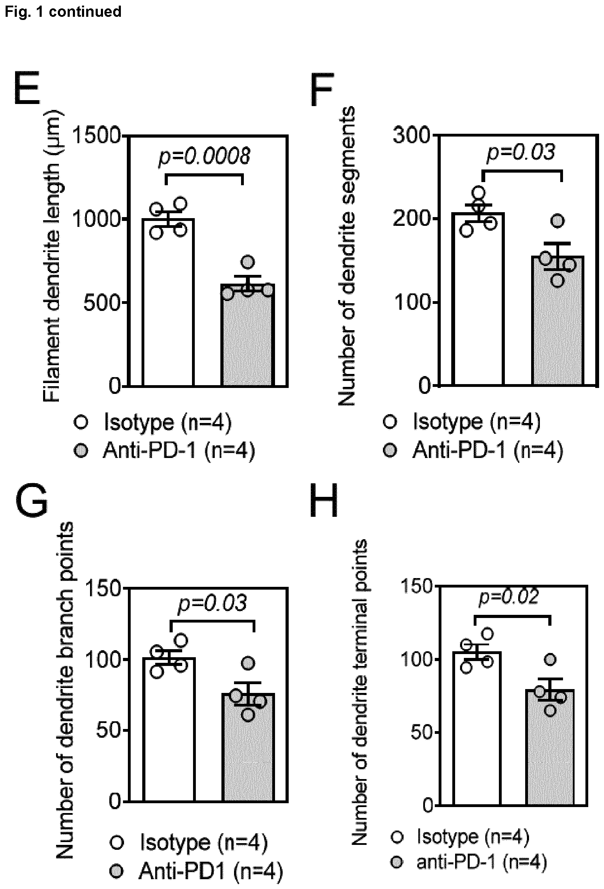

**Figure 1****:** Microglia show signs of activation upon anti-PD-1 treatment in the absence of tumor. (A-C) Histology of brain samples immunostained for Iba-1⁺ cells from the cortex of mice treated with anti-PD-1 or isotype control on day 22 after treatment. **(A)** Representative images from each group are shown. Scale bars, 50µm. The scatter plots show the number of Iba-1⁺ cells (per mm²) in cortex **(B)** and hippocampus **(C).** The results (mean ± s.e.m.) were combined. The experiment was performed three times. The *P*-values were calculated using the unpaired student's t-test. **(D)** Representative images showing Imaris (Bitplane)-based 3D-reconstruction of Iba-1⁺ microglia from the cortex of mice treated with anti-PD-1 or isotype control as indicated. Scale bar: 10µm. **(E-H)** Scatter plots showing Imaris-based automated quantification of microglial morphology from microglia the cortex of mice treated with anti-PD-1 or isotype control on day 22 after treatment. The experiment was performed two times and the results (mean ± s.e.m.) were combined. The *P-*values were calculated using the unpaired student's t-test.

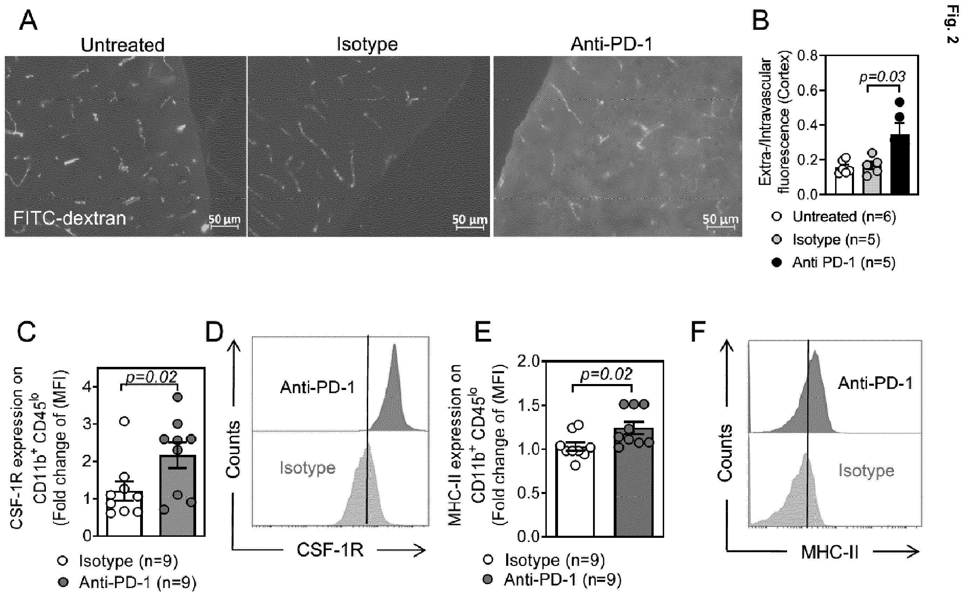

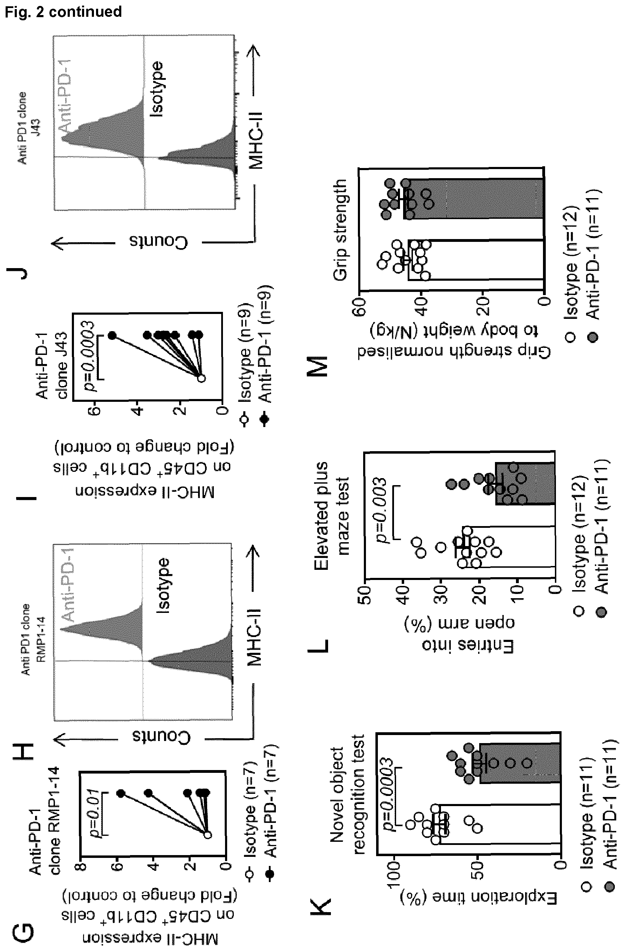

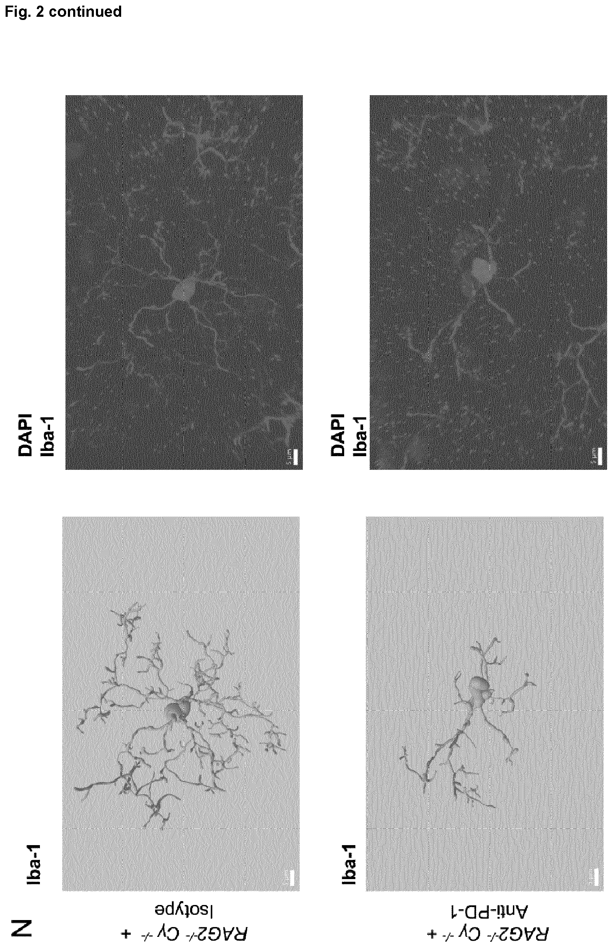

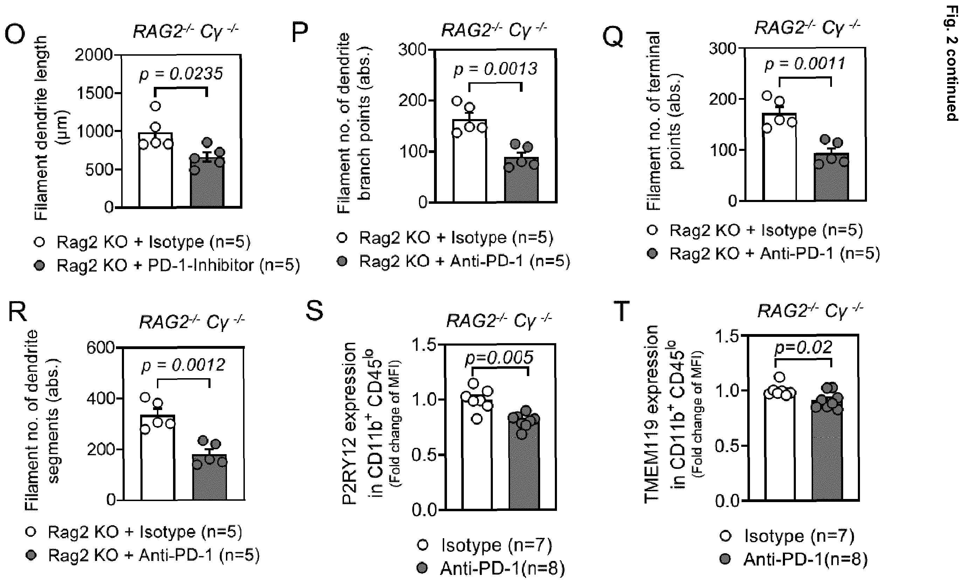

**Figure 2****:** Anti-PD-1 treatment causes neurocognitive deficits and increased CSF-1R and MHC-II expression on microglia. **(A-B)** Representative immunofluorescence images and scatter plot depicting the quantification of the ratio of extravascular versus intravascular FITC-dextran indicative of BBB leakage via extravasation of FITC-dextran in the cortex of either untreated, isotype-treated, or anti-PD-1-treated mice. Scale bar: 50µm. **(C-D)** The scatter plot **(C)** and representative histogram **(D)** show the quantification (fold change of MFI) of CSF-1R (CD115) on microglia (CD45^{lo} CD11b⁺) isolated from the CNS of mice treated with anti-PD-1 or isotype control on day 22. **(E-F)** The scatter plot **(E)** and representative histogram **(F)** show the quantification (fold change of MFI) for MHC-II on microglia (CD45^{lo} CD11b⁺) isolated from the CNS of mice treated with anti-PD-1 or isotype control on day 22. (G, **H)** The scatter plot **(G)** and representative histogram **(H)** show the fold change of MFI for MHC-II on cultured primary microglia (CD45⁺ CD11b⁺) treated with either isotype control (blue) or anti-PD-1 (red, anti-PD-1 clone RMP1-14) for 48 hours. (I,**J)** The scatter plot (I) and representative histogram **(J)** show the fold change of MFI for MHC-II on primary microglia (CD45⁺ CD11b⁺) cultured in the presence of isotype control (blue) or anti-PD-1 (red, Anti PD-1 clone J43) for 48 hours. Each sample pair is derived from an individual donor mouse. The experiments were performed with 7 **(G)** and 9 **(I)** biological replicates. **(K)** The scatter plot shows the time spend in exploring a novel object with respect to the total time by mice treated with anti-PD-1 or isotype control on day 22. **(L)** The scatter plot shows the percentage of open arm entries by mice treated with anti-PD-1 or isotype control in an elevated plus maze test on day 22. **(M)** The scatter plot shows the grip strength normalized to body weight (N/kg) of mice treated with anti-PD-1 or isotype control in a grip strength test on day 22. The experiment was repeated two times **(A-B, K-M)** and three times **(C, E).** The results (mean ± s.e.m.) were pooled. The *P*-values were calculated using the student's t-test **(B, C, E, K-M)** and paired samples Wilcoxon test **(G, I). (N)** Representative images showing Imaris (Bitplane)-based 3D-reconstruction of Iba-1⁺ microglia from the cortex of RAG2^{-/-}Cγ^{-/-} mice treated with anti-PD-1 or isotype control antibody as indicated. Scale bar: 5 µm. **(O-R)** Scatter plots showing Imaris-based automated quantification of microglial morphology from microglia the cortex of RAG2^{-/-}Cγ^{-/-} mice treated with anti-PD-1 or isotype control on day 22 after treatment. The experiment was performed two times and the results (mean ± s.e.m.) were combined. The *P-*values were calculated using the unpaired student's t-test. **(S)** The scatter plot shows the quantification (fold change of MFI) of P2YR12 on microglia (CD45^{lo} CD11b⁺) isolated on day 22 from the CNS of RAG2^{-/-}Cγ^{-/-} mice treated with anti-PD-1 or isotype control. **(T)** The scatter plot shows the quantification (fold change of MFI) of TMEM119 on microglia (CD45^{lo} CD11b⁺) isolated on day 22 from the CNS of RAG2^{-/-}Cγ^{-/-} mice treated with anti-PD-1 or isotype control.

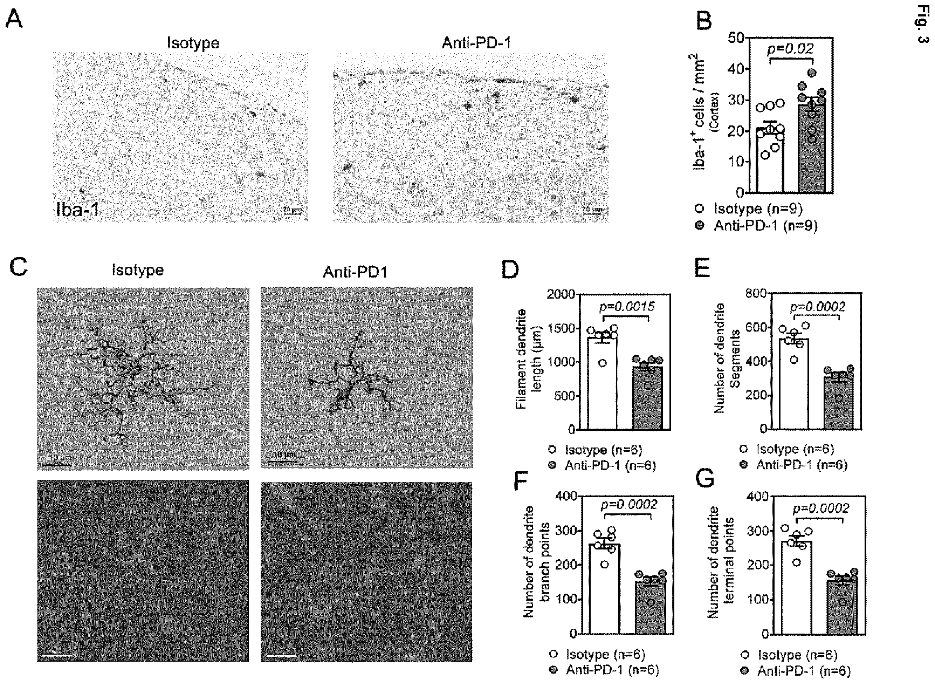

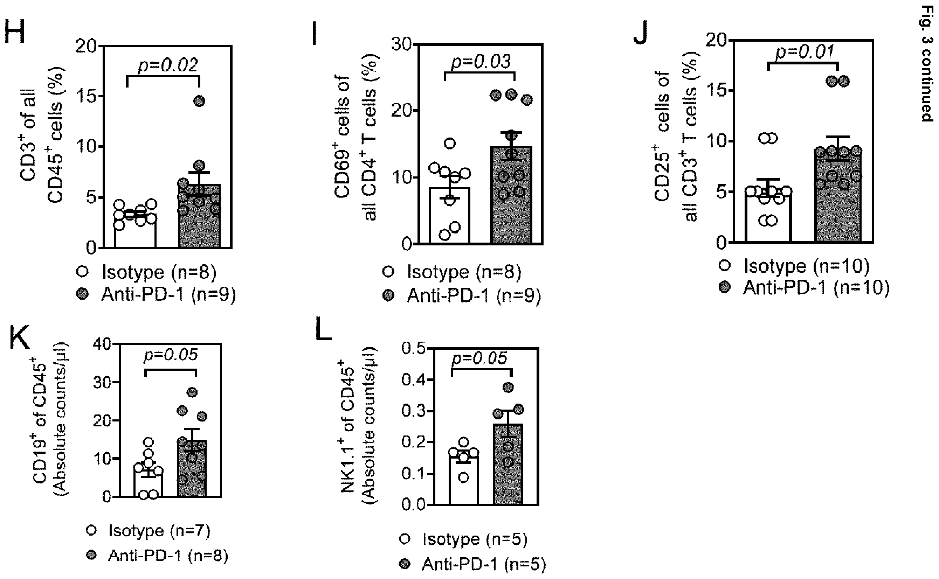

**Figure 3****:** Anti-PD-1 activates microglia in melanoma bearing mice. **(A, B)** Histology of CNS samples immunostained for Iba-1⁺ cells (brown stain) from the cortex of melanoma bearing mice treated with anti-PD-1 or isotype control on day 23 post tumor injection. **(A)** Representative images from each group are shown. Scale bars, 20µm. **(B)** The scatter plot shows the number of Iba-1⁺ cells (per mm²) in the cortex. The experiment was performed three times **(B). (C)** Representative images showing Imaris (Bitplane)-based 3D-reconstruction of Iba-1⁺ microglia from the cortex of melanoma bearing mice treated with anti-PD-1 or isotype control as indicated. Scale bar, 10µm. **(D-G)** Scatter plots showing Imaris-based automated quantification of microglial morphology from microglia from the cortex of melanoma bearing mice treated with anti-PD-1 or isotype control on day 23. **(H)** Flow cytometry based scatter plot shows quantification of CD3⁺ T cells among all viable cells isolated from the CNS of melanoma bearing mice treated with either isotype antibody or anti-PD-1 on day 23 after tumor injection. **(I)** Flow cytometry based scatter plot shows quantification of CD69⁺ T cells among all CD4+ T cells isolated from the CNS of melanoma bearing mice treated with either isotype antibody or anti-PD-1 on day 23 after tumor injection. **(J)** Flow cytometry based scatter plot shows quantification of CD25⁺ T cells among all CD3⁺ T cells isolated from the CNS of melanoma bearing mice treated with either isotype antibody or anti-PD-1 on day 23 after tumor injection. **(K)** Flow cytometry based scatter plot shows quantification of CD19⁺ B cells among all CD45⁺ T cells isolated from the CNS of melanoma bearing mice treated with either isotype antibody or anti-PD-1 on day 23 after tumor injection. **(L)** Flow cytometry based scatter plot shows quantification of NK1.1⁺ NK cells among all CD45⁺ T cells isolated from the CNS of melanoma bearing mice treated with either isotype antibody or anti-PD-1 on day 23 after tumor injection. The experiments were performed two times (**C-L**).The results (mean ± s.e.m.) were pooled. The *P*-values were calculated using the unpaired student's t-test (**A-L**)**.**

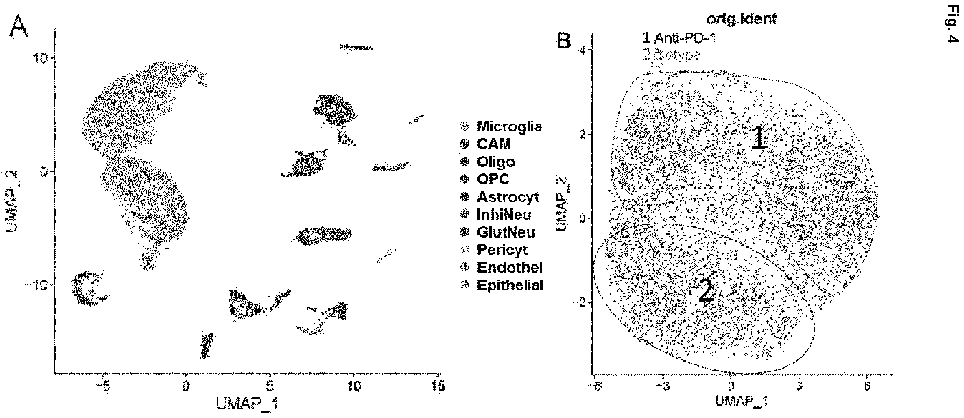

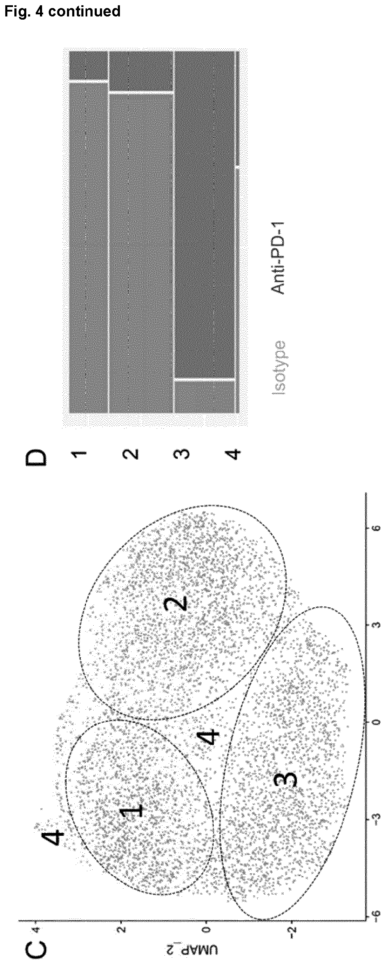

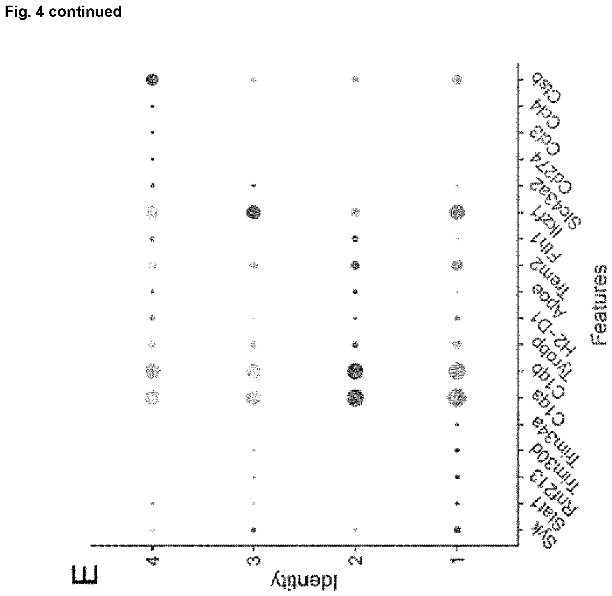

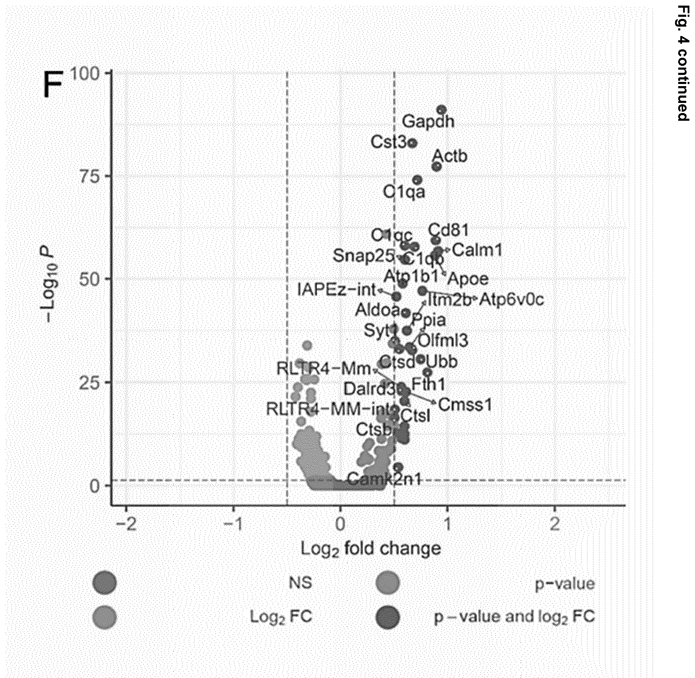

**Figure 4****:** snRNA sequencing reveals differential gene expression in microglia after anti-PD-1 treatment in melanoma bearing mice. **(A)** UMAP visualization of cells from the CNS of melanoma bearing mice treated with anti-PD-1 (1) or Isotype control (2). Greyscale-coding is based on cluster assignment by the Seurat v4 algorithm. The different clusters were determined by marker gene expression. Nuclei from the cerebral cortex of the CNS were sorted and analyzed using 10x Genomics single-nuclei 3' mRNA sequencing (snRNA Seq). The resulting dataset comprised several sub-clusters of different cell types, including microglia, CNS-associated macrophages (CAMs), oligodendrocytes (Oligo) and others, such as OPCs, astrocytes, inhibitory neurons (InhiNeu), glutamatergic neurons (GlutNeu), pericytes, endothelia and epithelial cells, according to their marker gene expression. **(B)** UMAP visualization of microglia isolated from the CNS of melanoma bearing mice. The greyscale coding is according to treatment with anti-PD-1 (cluster 1, top) or Isotype (cluster 2, bottom). Circles broadly indicate clusters 1 and 2. **(C)** UMAP visualization of microglia from the CNS of melanoma bearing mice treated with anti-PD-1 or Isotype. Clustering assignment revealed 4 clusters (1-4). Circles broadly indicate clusters. **(D)** The bar diagram shows the 4 clusters with grey-scale coding according to treatment with anti-PD-1 or Isotype control (1-4). **(E)** Visualization of the gene expression in microglia according to the 4 clusters. The grey scaling and size of the point are according to percent and average expression of the respective gene. **(F)** Enhanced volcano blot showing the major differentially regulated genes in microglia from the CNS of melanoma bearing mice. The grey scaling is according to the p-value.

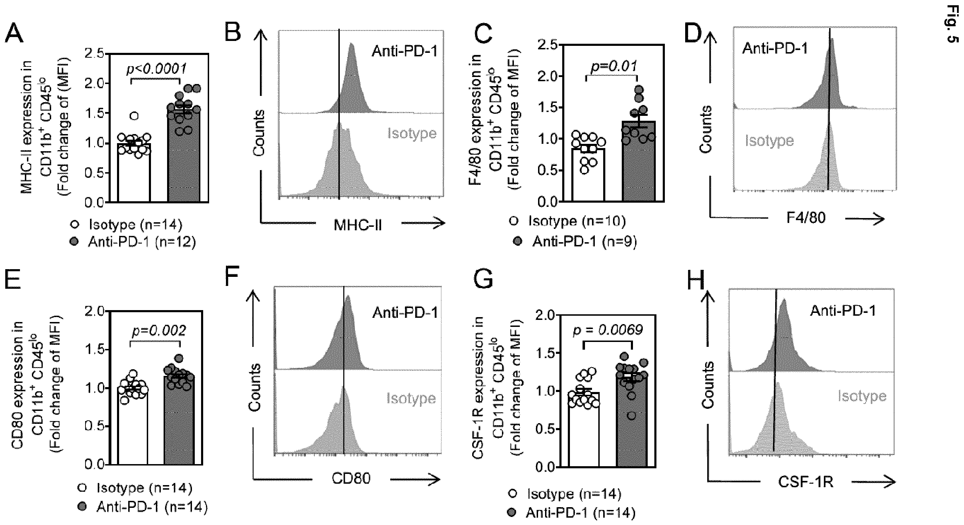

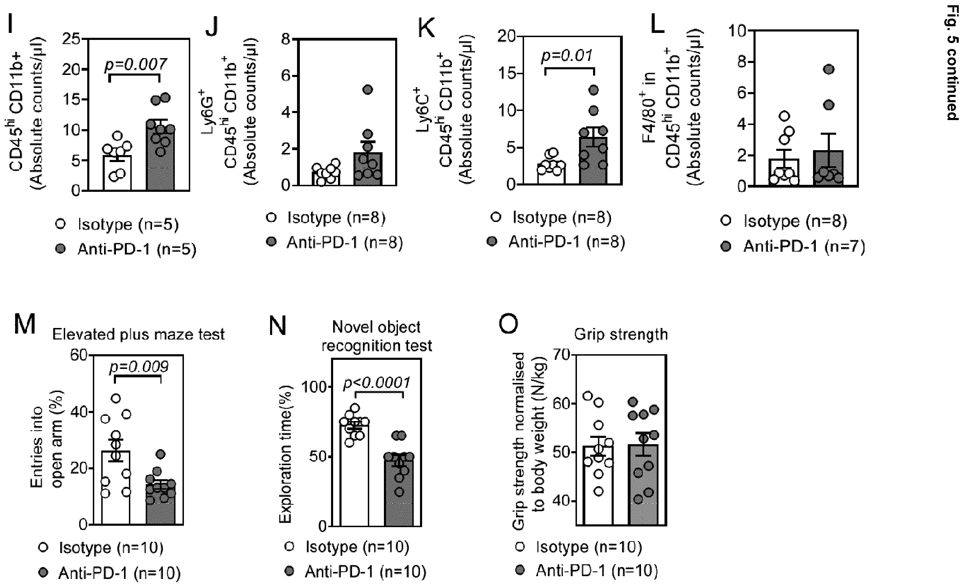

**Figure 5****:** MHC-II and CSF-1R expression increase on microglia in response to anti-PD-1 in melanoma bearing mice. **(A-B)** The scatter plot **(A)** and representative flow cytometry **(B)** show the quantification (fold change of MFI) of MHC-II on microglia (CD45^{lo} CD11b⁺) isolated from the CNS of melanoma bearing mice treated with anti-PD-1 or isotype on day 23 after melanoma cell injection. **(C-D)** The scatter plot **(C)** and representative flow cytometry **(D)** show the quantification (fold change of MFI) of F4/80 on microglia (CD45^{lo} CD11b⁺) isolated from the CNS of melanoma bearing mice treated with anti-PD-1 or isotype day 23 after tumor injection. **(E-F)** The scatter plot **(E)** and representative flow cytometry **(F)** show the quantification (fold change of MFI) of CD80 on microglia (CD45^{lo} CD11b⁺) isolated from the CNS of melanoma bearing mice treated with anti-PD-1 or isotype day 23 after tumor injection. **(G-H)** The scatter plot **(G)** and representative flow cytometry **(H)** show the quantification (fold change of MFI) of CD115 (CSF-1R) on microglia (CD45^{lo} CD11b⁺) isolated from the CNS of melanoma bearing mice treated with anti-PD-1 or isotype day 23 after tumor injection. **(I-L)** The scatter plots show the quantification (absolute counts/µl) of CD45^{hi} CD11b⁺ cells **(I),** Ly6G⁺ CD45^{hi} CD11b⁺ (**J**), Ly6C⁺ CD45^{hi} CD11b⁺ (**K**), F4/80⁺ CD45^{hi} CD11b⁺ **(L)** isolated from the CNS of melanoma bearing mice treated with anti-PD-1 or isotype day 23 after tumor injection. **(M)** The scatter plot shows the percentage of open arm entries by melanoma bearing mice treated with anti-PD-1 or isotype in an elevated plus maze test on day 23 after tumor injection. **(N)** The scatter plot shows the time spend in exploring a novel object with respect to the total time by melanoma bearing mice treated with anti-PD-1 or isotype on day 23 after tumor injection. **(O)** The scatter plot shows the grip strength normalized to body weight (N/kg) of melanoma bearing mice treated with anti-PD-1 or isotype in a grip strength test on day 23 after tumor injection. The experiment was performed three times **(A,C,E,G)** or two times **(I-O).** The results (mean ± s.e.m.) were pooled. The *P*-values were calculated using the student's t-test.

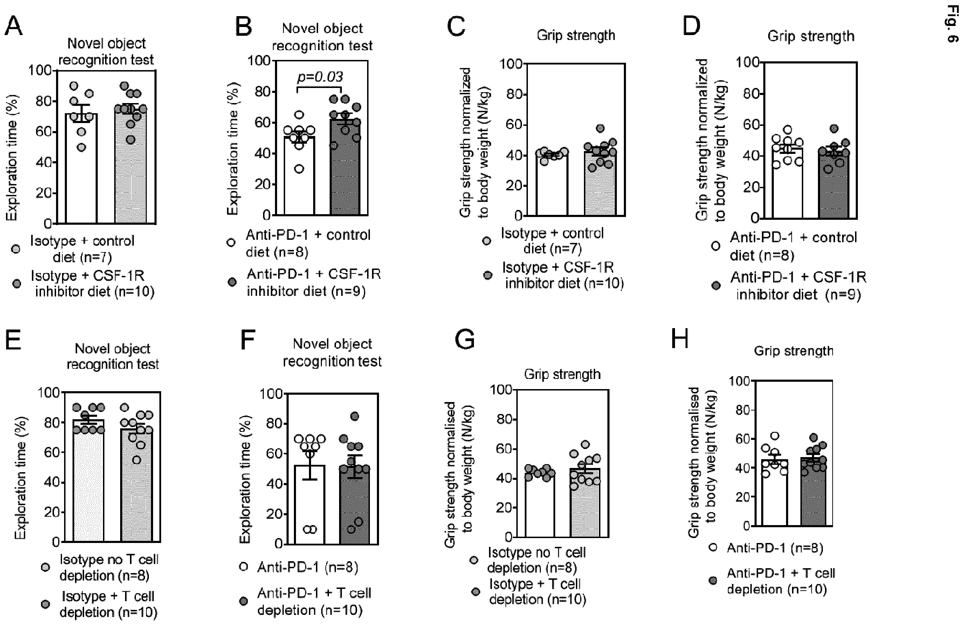

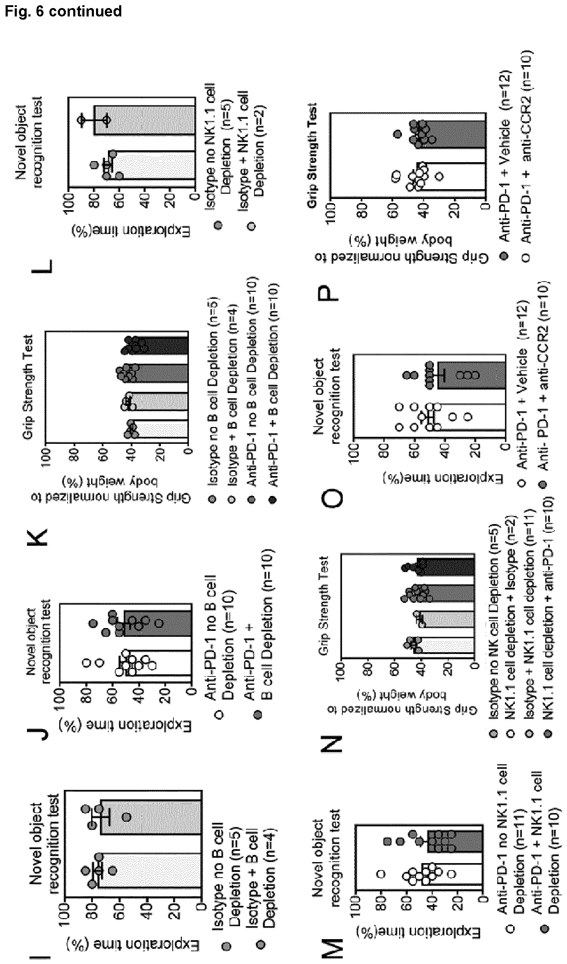

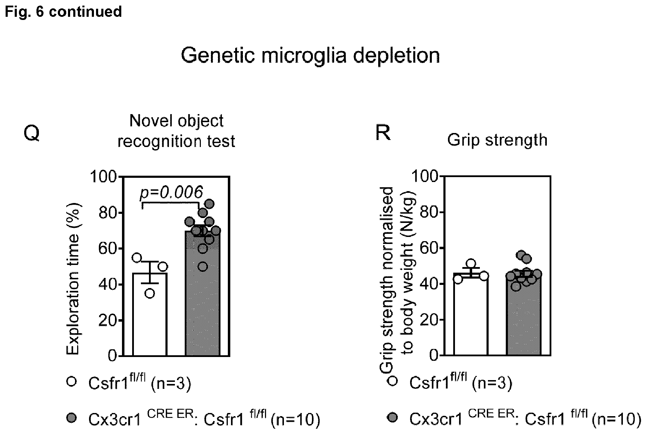

**Figure 6****:** Neurocognitive defects of anti-PD-1 treated mice are dependent on microglia but not T cells, B cells, NK cells or CCR2⁺ cells. **(A-D)** Neurocognitive tests were performed on day 23 on melanoma bearing mice that were treated with isotype or anti-PD-1 and received either control diet or CSF-1R inhibitor (PLX5622) diet leading to depletion of CSF-1R positive cells. **(A-B)** The scatter plot shows the time spent exploring a novel object with respect to total time by melanoma bearing mice treated with isotype control **(A)** or anti-PD-1 **(B)** in a novel object recognition test. **(C-D)** The scatter plot shows the grip strength normalized to body weight (N/kg) of melanoma bearing mice treated with isotype control **(C)** or anti-PD-1 **(D)** in a grip strength test. **(E-H)** Neurocognitive tests were performed on melanoma bearing mice that were treated with either isotype or anti-PD-1 and additionally received anti-CD4 and anti-CD8 T cell depleting antibodies or the isotype control for T cell depletion ("no T cell depletion group"). **(E-F)The** scatter plot shows the time spent exploring a novel object with respect to total time by melanoma bearing mice treated with either T cell depleting antibodies or isotype for T cell depletion and isotype control **(E)** or anti-PD-1 **(F). (G-H)** The scatter plot shows grip strength normalized to body weight (N/kg) of melanoma bearing mice receiving either T cell depleting antibodies or isotype for T cell depletion and isotype control **(G)** or anti-PD-1 **(H). (I-K)** Neurocognitive tests were performed on melanoma bearing mice that were treated with either isotype or anti-PD-1 and additionally received anti-CD19/CD45R B cell depleting antibodies or the isotype control for B cell depletion ("no B cell depletion group"). **(I, J)** The scatter plots show the time spent exploring a novel object with respect to total time by melanoma bearing mice treated with either B cell depleting antibodies or isotype for B cell depletion and isotype control **(I)** or anti-PD-1 **(J). (K)** The scatter plot shows grip strength normalized to body weight (N/kg) of melanoma bearing mice receiving either B cell depleting antibodies or isotype for B cell depletion and isotype control or anti-PD-1. **(L-N)** Neurocognitive tests were performed on melanoma bearing mice that were treated with either isotype or anti-PD-1 and additionally received NK cell depleting antibodies (anti-NK1.1) or the isotype control for NK cell depletion ("no B cell depletion group"). **(L, M)** The scatter plots show the time spent exploring a novel object with respect to total time by melanoma bearing mice treated with either NK cell depleting antibodies or isotype for NK cell depletion and isotype control **(L)** or anti-PD-1 **(M)**. **(N)** The scatter plot shows grip strength normalized to body weight (N/kg) of melanoma bearing mice receiving either NK cell depleting antibodies or isotype for NK cell depletion and isotype control or anti-PD-1. **(O)** Neurocognitive tests were performed on melanoma bearing mice that were treated with anti-PD-1 and additionally received anti-CCR2⁺ cell depleting antibodies or vehicle (no isotype for the anti-CCR2 (MC21) antibody available). The scatter plots show the time spent exploring a novel object with respect to total time by melanoma bearing mice treated with either CCR2 antibody plus anti-PD-1 or vehicle plus anti-PD-1. **(P)** The scatter plot shows grip strength normalized to body weight (N/kg) of melanoma bearing mice receiving either CCR2⁺ cell depleting antibodies plus anti-PD-1 or vehicle plus anti-PD-1. **(Q)** The scatter plot shows the time spent exploring a novel object with respect to total time by melanoma bearing *Csf-1r*^{*fl*/}*^{fl} or Cx3cr1^{CRE ER}: Csf1r*^{*fl*/*fl*} mice treated with anti-PD-1 in a novel object recognition test. **(R)** The scatter plot shows the grip strength normalized to body weight (N/kg) of melanoma bearing *Csf1r*^{*fl*/*fl*} or *Cx3cr1^{CRE ER} Csf-1r*^{*fl*/*fl*} mice treated with anti-PD-1 in a grip strength test. All experiments were performed twice and results (mean ± s.e.m.) were pooled. *P-*values were calculated using Student's t-test.

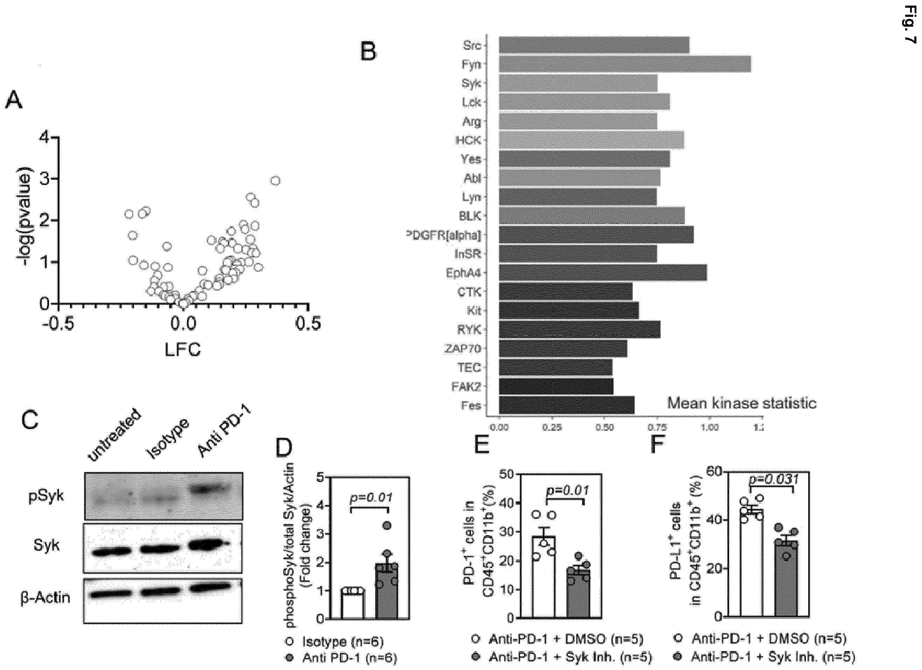

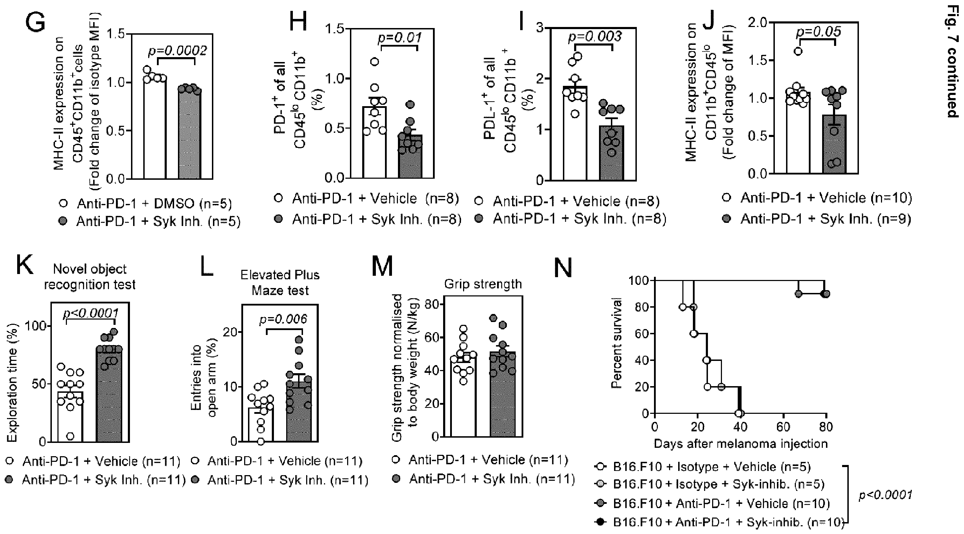

**Figure 7****:** Phospho-kinase analysis identifies SYK activation in microglia upon anti-PD-1 treatment. **(A)** The volcano plot visualizes the result of the kinase activity arrays by plotting - for each test the effect size (x-axis, LFC or delta) versus significance (y-axis, -Iog10 (p-value)) of the test. Dots indicate peptides that are significant different compared to control (p<0.05). **(B)** The bar plot shows kinases that are more frequently phosphorylated in the anti-PD-1 group compared to the isotype control group. **(C-D)** Representative Western blot image **(C)** and quantification **(D)** of phospho Syk/total Syk normalized to β-actin (fold change with respect to isotype (0µM) treated controls) using protein derived from primary murine microglia (n=6 biological replicates) treated with 20µg of anti-PD-1 for 5 min. Each data point represents an individual sample of one independent cell culture experiment. *P*-values were calculated using Student's unpaired *t*-test. **(EG)** Cultured primary microglia were treated with anti-PD-1 or isotype along with Syk inhibitor (Syk Inh.) or vehicle for 48h. The scatter plot shows the quantification (percentage) of PD-1⁺ **(E),** PD-L1⁺ **(F)**, and MHC-II⁺ **(G)** cells in primary microglia (CD45^{lo}CD11b⁺). The experiment was performed twice and results (mean ± s.e.m.) were pooled. *P*-values were calculated using Student's unpaired *t*-test. **(H-I)** The scatter plot shows the quantification (percentage) of PD-1 **(H)** and PD-L1 **(I)** on microglia (CD45^{lo}CD11 b⁺) isolated from the CNS of melanoma bearing mice on day 17 after tumor injection treated with anti-PD-1 and Syk inhibitor or vehicle as indicated. The experiment was performed twice and results (mean ± s.e.m.) were pooled. *P*-values were calculated using Student's unpaired t-test. **(J)** The scatter plot shows the quantification (fold change of MFI) of MHC-II on microglia (CD45^{lo} CD11b⁺) isolated from the CNS of melanoma bearing mice on day after tumor injection treated with anti-PD-1 and Syk inhibitor or vehicle as indicated. The experiment was performed twice and results (mean ± s.e.m.) were pooled. *P-*values were calculated using Student's unpaired *t*-test. **(K-M)** Neurocognitive tests were performed on melanoma bearing mice on day 17 post tumor injection that were treated with anti-PD-1 and Syk inhibitor or vehicle. The results (mean ± s.e.m.) were pooled. The experiment was performed twice. *P*-values were calculated using Student's *t*-test. **(K)** The scatter plot shows the time spent exploring a novel object with respect to the total time by melanoma bearing mice treated with anti-PD-1 and Syk inhibitor or vehicle. **(L)** The scatter plot shows the entries into open arms of melanoma bearing mice treated with anti-PD-1 and Syk inhibitor or vehicle. **(M)** The scatter plot shows the grip strength normalized to body weight (N/kg) of melanoma bearing mice treated with anti-PD-1 and Syk inhibitor/vehicle. **(N)** Survival rates of melanoma bearing mice that were treated with isotype or anti-PD-1 and Syk inhibitor or vehicle as indicated. The experiments were performed twice and results were pooled. *P*-values were calculated using a two-sided Mantel-Cox test.

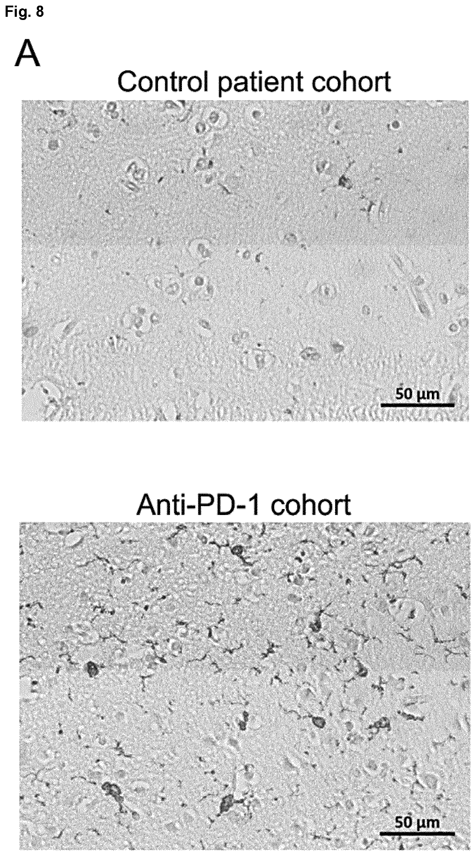

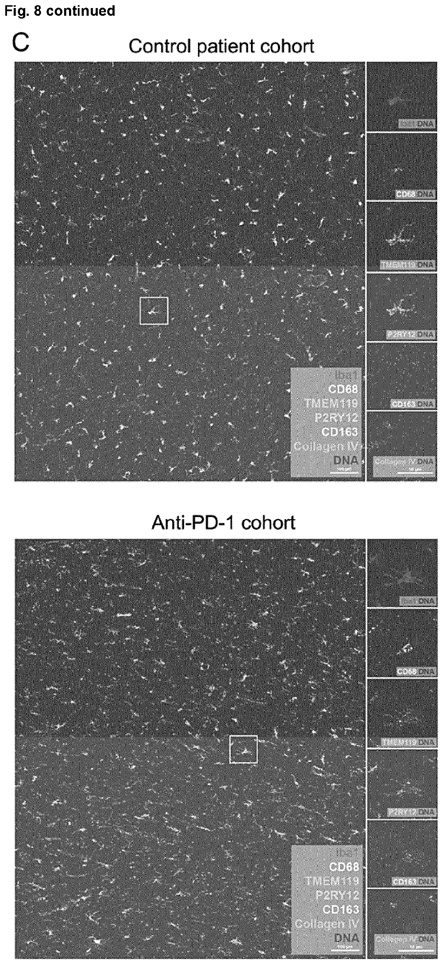

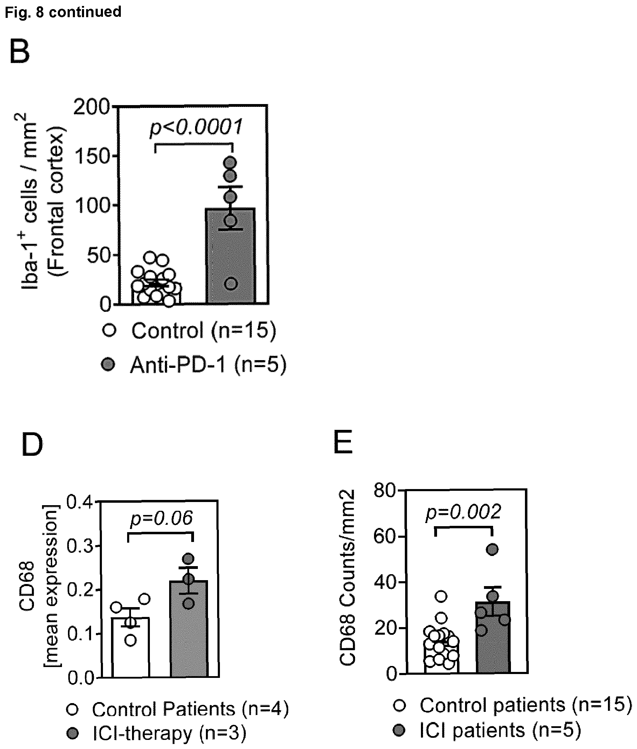

**Figure 8****:** Anti-PD-1 treated patients exhibit an activated microglia phenotype. **(A, B)** Immunohistochemistry for Iba-1⁺ cells of frontal cortex samples derived from control patients or patients treated with anti-PD-1. **(A)** Representative images from each group are shown. Scale bars, 50µm. **(B).** The scatter plot shows the number of Iba-1⁺ cells (per mm²) in grey matter of frontal lobe. The experiment was performed once. The *P*-values were calculated using the student's t-test. **(C)** Representative Imaging Mass Cytometry (IMC) visualization of human frontal cortex tissue of control patients and patients having undergone anti-PD-1 therapy as indicated. Iba1, CD68, TMEM119, P2RY12, CD163, Collagen IV and DNA are shown. The scale bars represent 100 µm in the main images and 50 µm in the inserts. **(D)** The scatter plot depicts IMC based quantification of CD68⁺ myeloid cells in post-mortem FFPE samples obtained from the frontal cortex of anti-PD-1 treated patients (n=3) and the age matched controls (n=4). **(E)** The stacked bar chart shows mean count per group of immune cell cluster composition of control patients (control, n=4) and patients treated with anti-PD-1 (n=3).

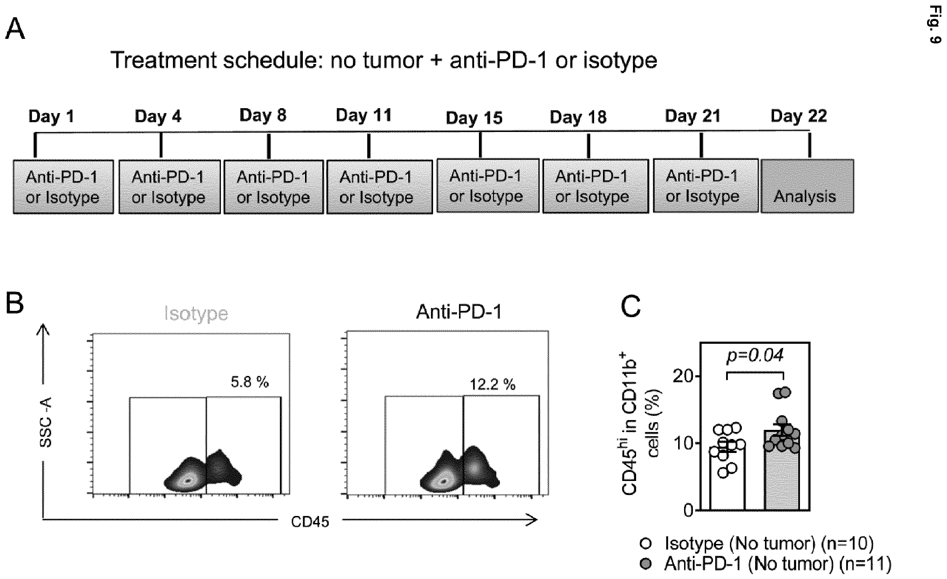

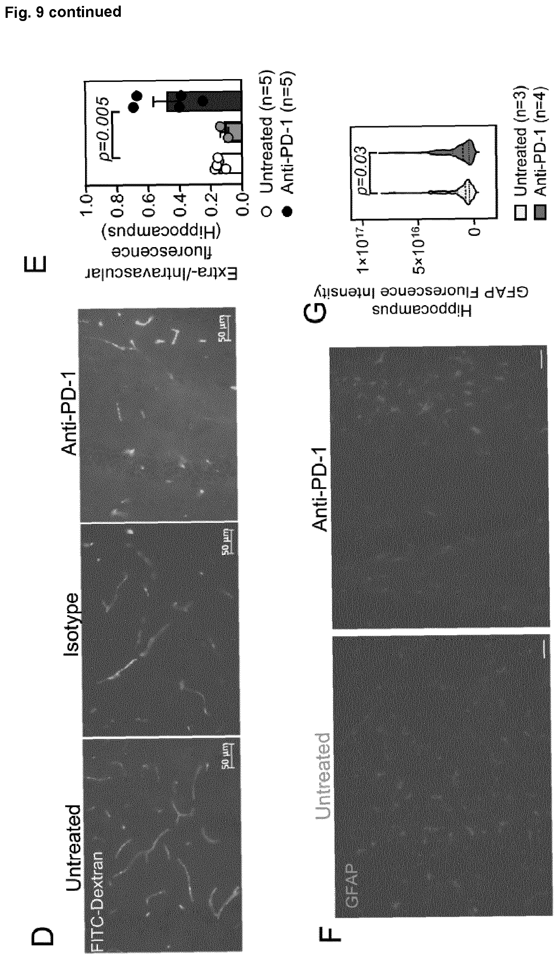

**Figure 9****:** Treatment sketch for CNS-irAE induction in the absence of tumor, BBB leakage and astrocyte activation. **(A)** Schematic for the treatment of healthy mice (no tumor) with 250µg of isotype or anti-PD-1 (per dose) at the time points indicated. Analysis was performed on day 22. **(B-C)** Flow cytometry for CD45^{hi} cells within all CD11b⁺ cells isolated from the CNS of C57BL/6 mice treated with either isotype or anti-PD-1 on day 22 as indicated. **(B)** Representative flow cytometry plot from each group. **(C)** Scatter plot showing the quantification of CD45^{hi} cells among all CD11b⁺ cells from the indicated groups. The experiments were performed three times. *P-*values were calculated using Student's unpaired t-test. **(D)** Representative images of the hippocampus of untreated, isotype-treated, or anti-PD-1-treated mice following FITC-Dextran injection. BBB leakage correlates with extravasation of FITC-Dextran into the extravascular space. Scale bar: 50µm. **(E)** Scatter plot showing the ratio of extravascular versus intravascular FITC-dextran fluorescence in the hippocampus of untreated isotype-treated, or anti-PD-1-treated mice. *P*-values were calculated using Student's unpaired t-test. **(F)** Representative immunofluorescence images of glial fibrillary-associated protein (GFAP) expression in the hippocampus of untreated or anti-PD-1-treated mice. Scale bar: 30µm. **(G)** Violin plot showing the quantification of GFAP fluorescence intensity per cell in the hippocampus of untreated or anti-PD-1-treated mice. The *P*-values were calculated using the Mann-Whitney t-test.

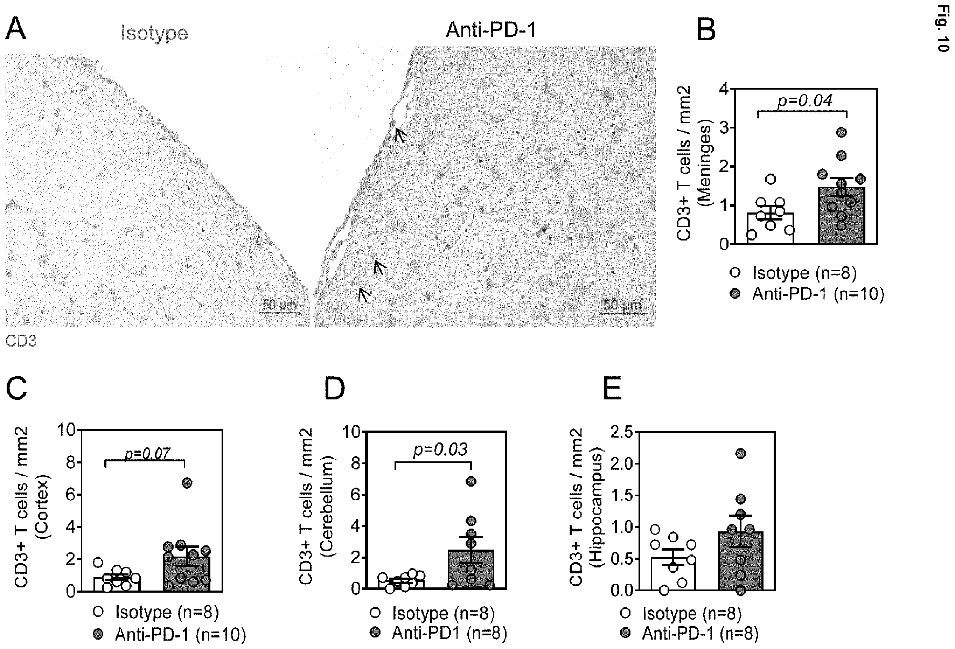

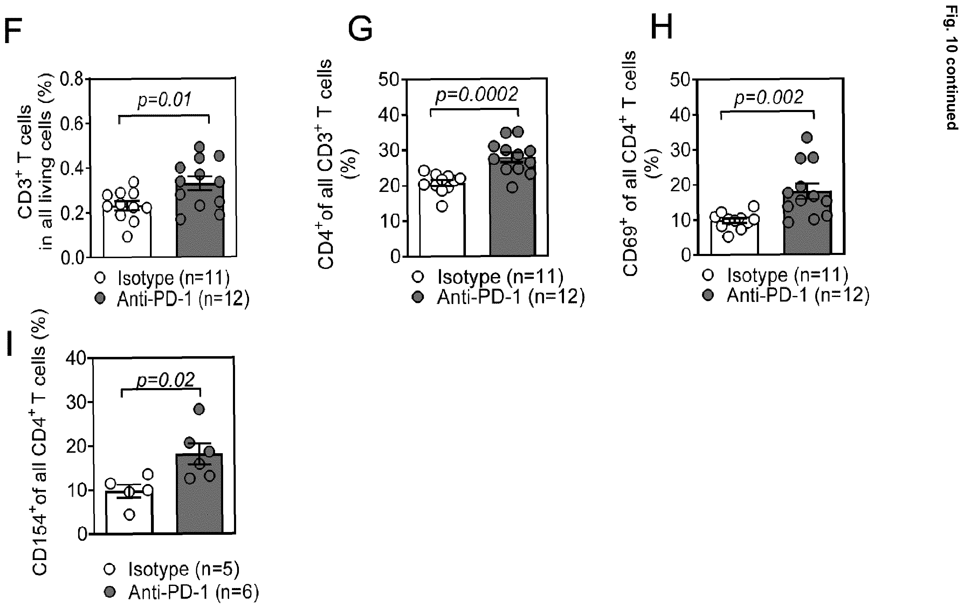

**Figure 10****:** Activated T cells infiltrate the CNS in response to anti-PD-1 therapy. **(A-E)** Histology of brain samples immunostained for CD3⁺ cells from the cortex of mice treated with anti-PD-1 or isotype control. **(A)** Representative images from each group are shown. Scale bars denote 50µm. The scatter plots show the number of CD3⁺ cells (per mm²) in meninges **(B),** cortex **(C),** cerebellum **(D),** and hippocampus **(E).** The experiment was performed two times and the results (mean ± s.e.m.) were pooled. *P*-values were calculated using Student's unpaired *t*-test. **(F-G)** The scatter plot shows the quantification (percentage) of CD3⁺ T cells **(F)** and CD4⁺ T cells **(F)** from the CNS of mice treated with isotype or anti-PD-1. The experiments were performed two times and the results (mean ± s.e.m.) were pooled. *P*-values were calculated using Student's unpaired *t*-test. **(H-I)** The scatter plot shows the quantification (percentage) of CD69⁺ **(H)** and CD154⁺ **(I)** T cells of all CD4⁺ T cells isolated from the CNS of mice treated with isotype control or anti-PD-1. The experiments were performed two times and the results (mean ± s.e.m.) were pooled. *P-*values were calculated using Student's unpaired *t*-test.

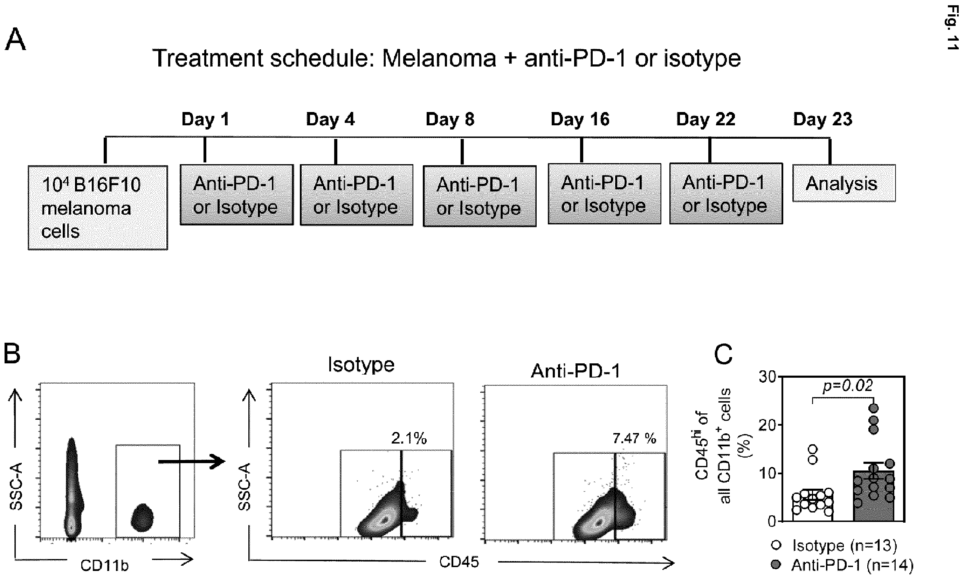

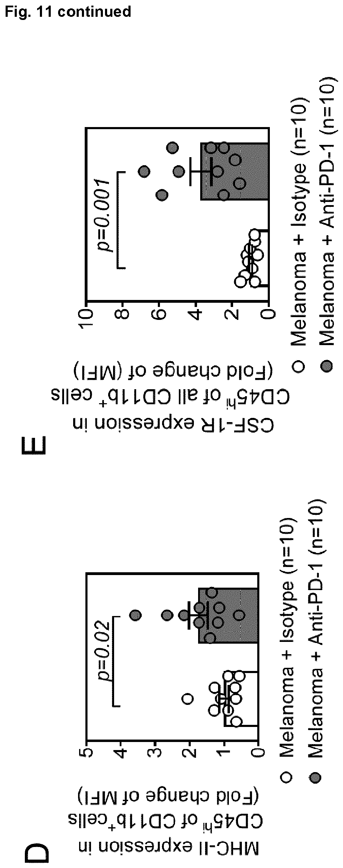

**Figure 11****:** Treatment sketch for CNS-irAE induction in the melanoma mouse model, and CD45^{hi} infiltration. **(A)** The treatment scheme is depicted. Mice were intravenously injected with 10,000 B16F10Luc⁺ GFP⁺ melanoma cells and treated with 250 µg of either anti-PD-1 or isotype antibody at the indicated time points. Analysis was performed on day 23 after melanoma injection. **(B-C)** Flow cytometry for CD45^{hi} cells within all CD11b⁺ cells in the CNS of melanoma bearing mice treated with either isotype antibody or anti-PD-1 antibody. The analysis was performed on day 23 after melanoma injection as indicated. **(B)** Representative flow cytometry plots from each group. **(C)** The scatter plot shows the quantification of CD45^{hi} cells within all CD11b⁺ cells from different groups as indicated. The experiment was repeated 3 times and results (mean ± s.e.m) were pooled. *P* values were calculated using Student's unpaired t-test. **(D-E)** The scatter plot shows the quantification (fold change of MFI) of MHC-II **(D)** and CSF-1R **(E)** expression on CD45^{hi} CD11b⁺ cells isolated from the CNS of melanoma-bearing mice treated with anti-PD-1 antibody or isotype control. The experiments were performed twice and the results (mean ± s.e.m.) were pooled. *P*-values were calculated using Student's unpaired *t*-test.

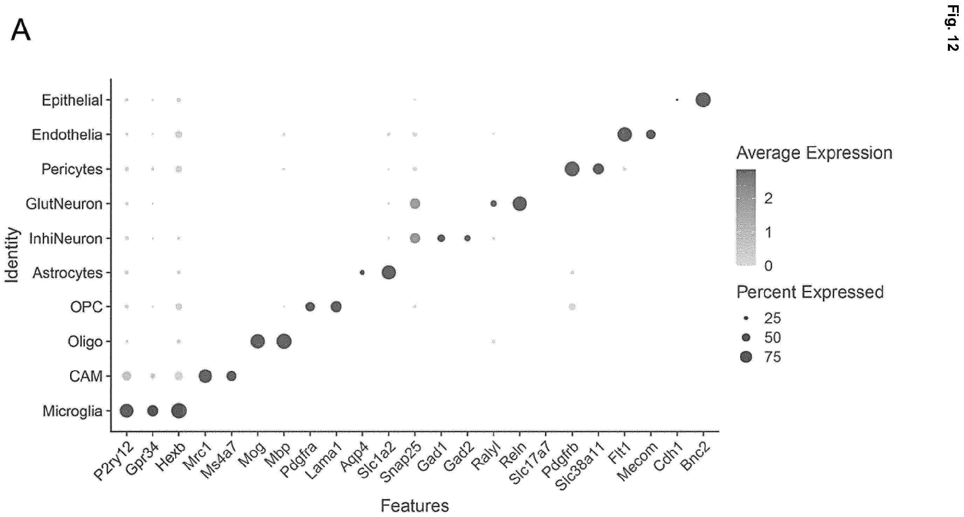

**Figure 12****:** nRNA seq-based marker gene expression in the CNS of melanoma bearing mice. **(A)** The dot plot shows marker gene expression of cells isolated from the CNS of melanoma (B16F10) bearing mice treated with isotype or anti-PD-1. The different cell types were determined by marker gene expression. The experiment was performed once.

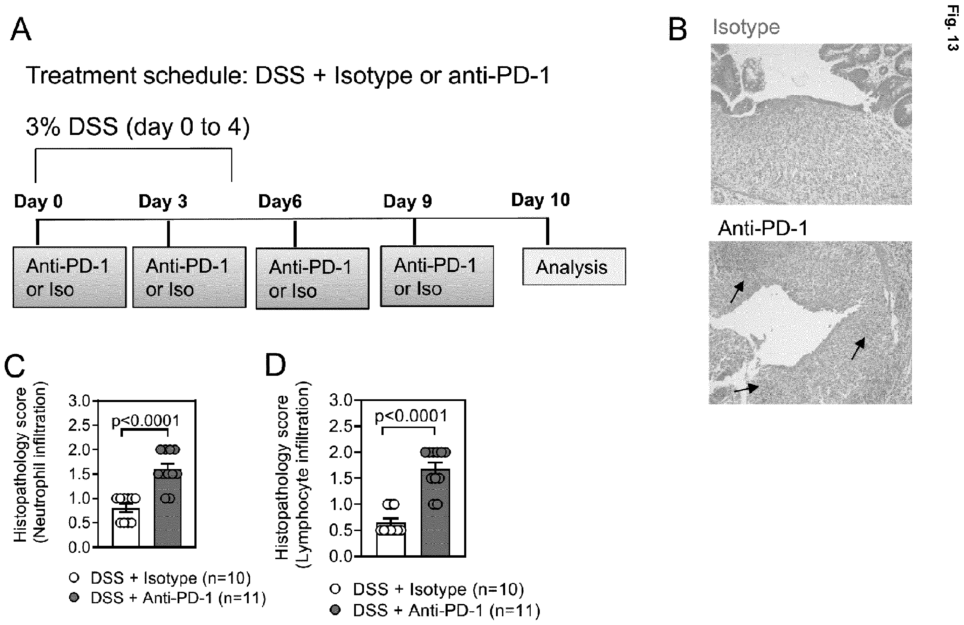

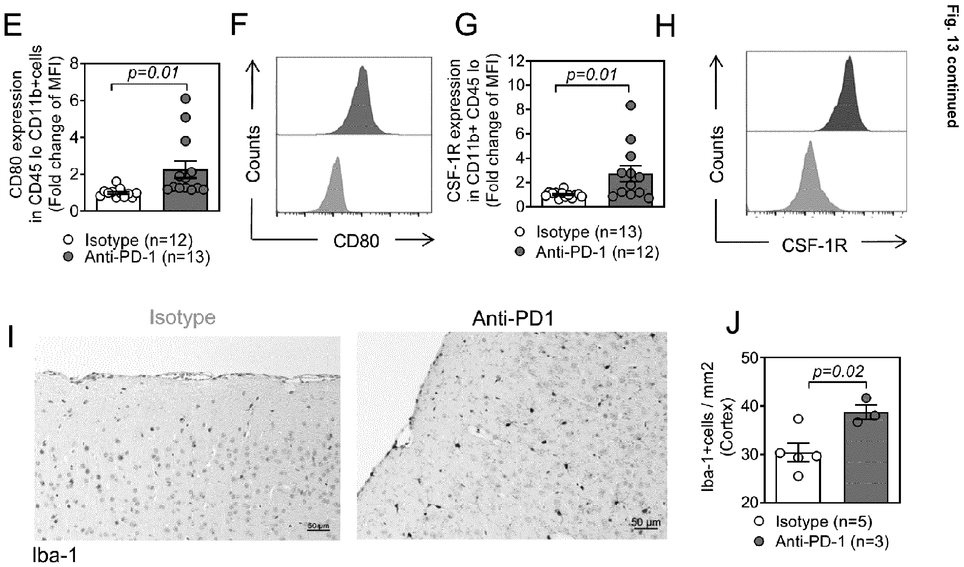

**Figure 13****:** Treatment sketch for DSS colitis model and microglial activation. **(A)** Schematic depicting the schedule of DSS colitis induction and anti-PD-1 or isotype treatments. Mice were treated with 3% DSS from day 0-4 and treated i.p. with 250µg of isotype or anti-PD-1 at indicated time points. Analysis was performed on day 10. **(B)** Representative H&E-stained colon samples. The arrow indicate immune cell infiltration in mice developing colitis of the anti-PD-1 group. **(C-D)** Histology scores representing neutrophil **(C)** and lymphocyte **(D)** infiltration in the colon of DSS-induced colitis mice treated with anti-PD-1 or isotype control. **(E-H)** Scatter plots show the quantification (fold change of MFI) of CD80 **(E)**, CSF-1R **(G)** expression on microglia (CD45^{lo}CD11b⁺) isolated from the CNS of DSS-induced colitis mice treated with anti-PD-1 or isotype control. **(F, H)** Representative flow cytometry plots for CD80 and CSF-1R from each group are shown. The experiments were performed three times and results (mean ± s.e.m.) were pooled. P-values were calculated using Student's unpaired t-test. **(I-J)** Immunostaining for Iba-1⁺ cells from the cortex of DSS-induced colitis mice treated with isotype control or anti-PD-1. **(I)** Representative images from each group. Scale bars: 50µm. **(J)** Scatter plot shows the number of Iba-1⁺ cells (per mm²). The experiment was performed once.

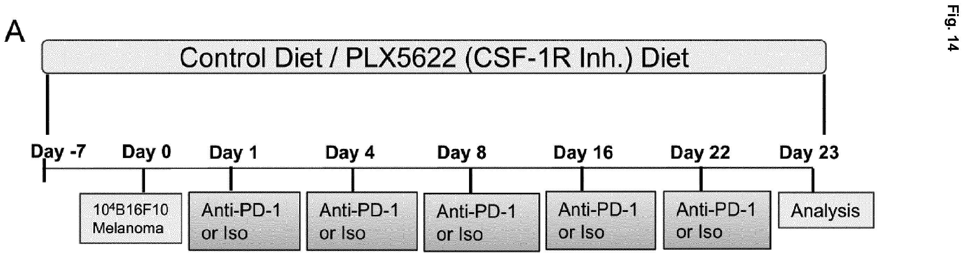

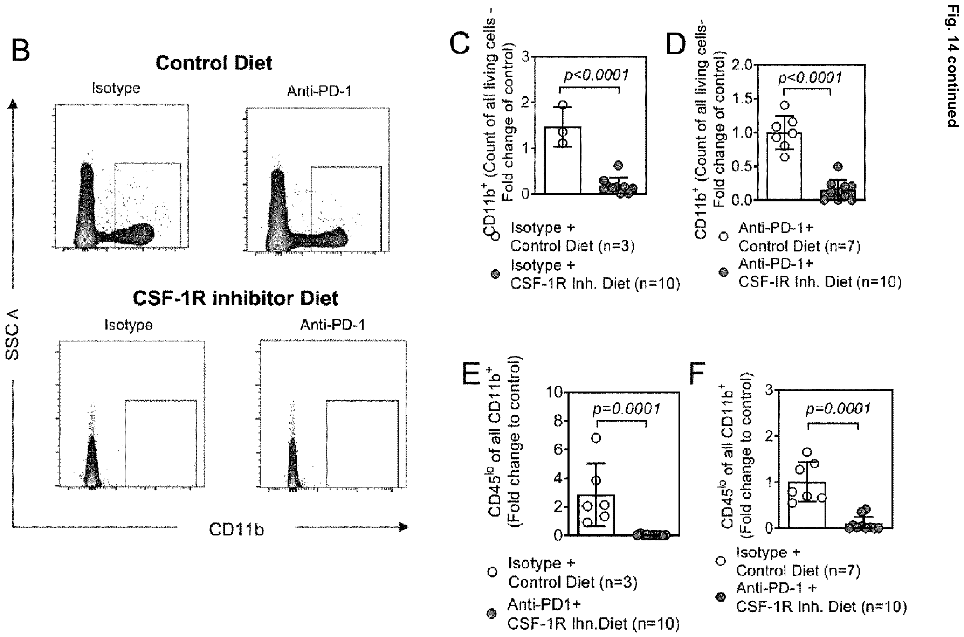

**Figure 14****:** Treatment sketch for Microglia depletion mouse model. **(A)** The treatment scheme is depicted. Mice received a CSF-1R inhibitor diet (PLX5622) or control diet from day-7 until day 23. On day 0, 10,000 B16F10Luc⁺GFP⁺ melanoma cells were injected i.v. and mice were treated with 250 µg of either or isotype or anti-PD-1 at the indicated time points. Analysis was performed on day 23 after melanoma injection. **(B-F)** Representative flow cytometry plots and scatter plots depicting the depletion of CD11b⁺ cells and (CD11b⁺CD45^{lo}) cells from the CNS of melanoma bearing mice treated with either isotype or anti-PD-1 and maintained on CSF-1R inhibitor diet or control diet.

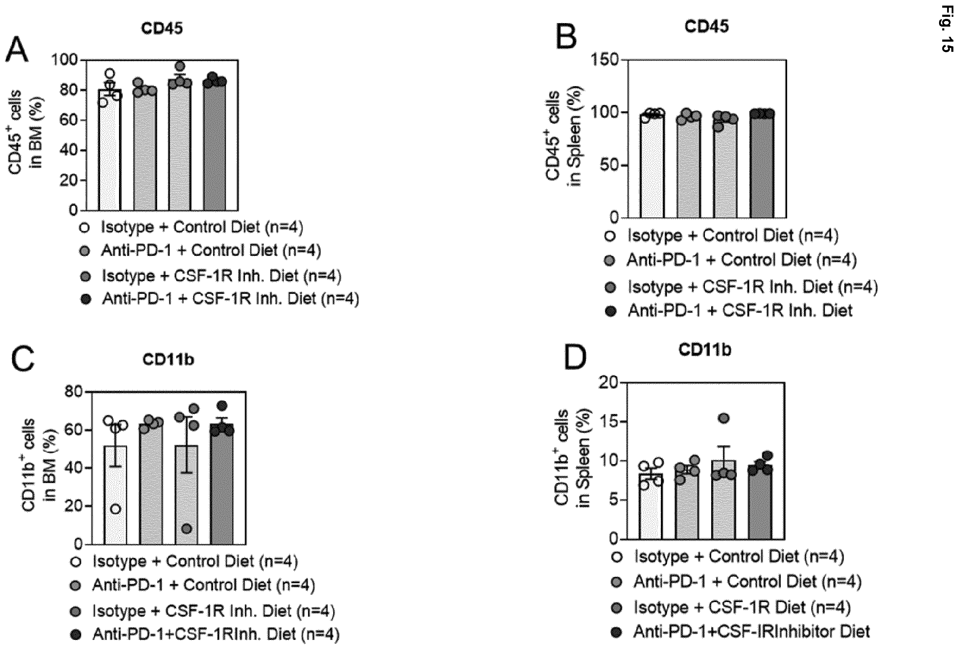

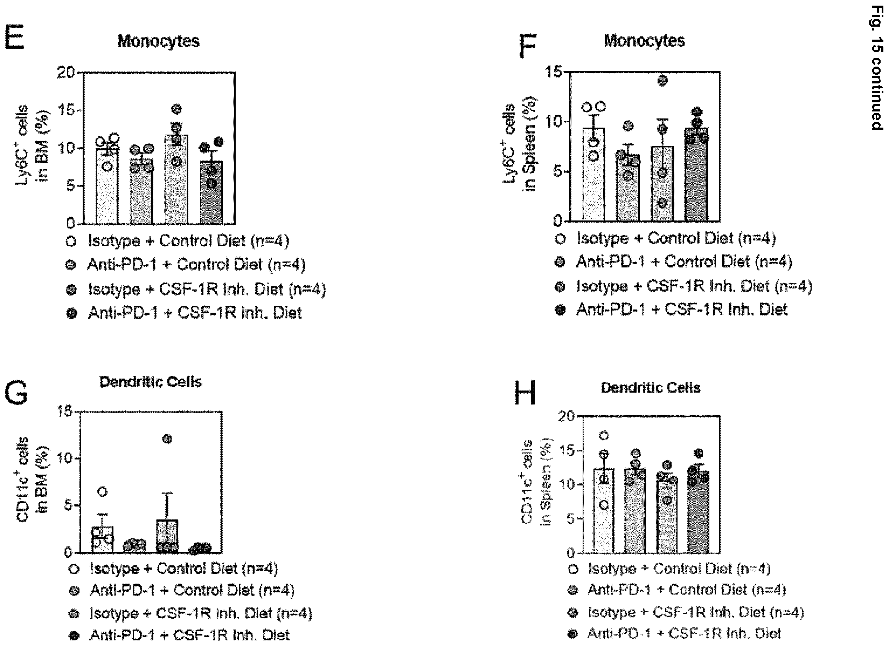

**Figure 15****:** Impact of CSF-1R Inhibition on the peripheral immune cells. **(A-B)** The scatter plot shows the quantification (percentage) of CD45⁺of all living cells isolated from BM **(A)** and spleen **(B)** of melanoma-bearing mice treated with anti-PD-1 or isotype control. The experiment was performed twice and results (mean ± s.e.m.) were pooled. *P*-values were calculated using Student's unpaired *t*-test. **(C-D)** The scatter plot shows the quantification (percentage) of CD11b⁺ of all CD45 cells isolated from BM **(C)** and spleen **(D)** of melanoma-bearing mice treated with anti-PD-1 or isotype control. The experiment was performed twice and the results (mean ± s.e.m.) were pooled. *P*-values were calculated using Student's unpaired *t*-test. **(E-F)** The scatter plot shows the quantification (percentage) of Ly6C⁺ (monocytes) of all CD45 cells isolated from BM **(E)** and spleen **(F)** of melanoma-bearing mice treated with anti-PD-1 or isotype control. The experiment was performed twice and the results (mean ± s.e.m.) were pooled. *P*-values were calculated using Student's unpaired t-test. **(G-H)** The scatter plot shows the quantification (percentage) of CD11c⁺ of all CD11b cells isolated from BM **(G)** and spleen **(H)** of melanoma-bearing mice treated with anti-PD-1 or isotype control. The experiment was performed twice and the results (mean ± s.e.m.) were pooled. *P*-values were calculated using Student's unpaired *t-*test.

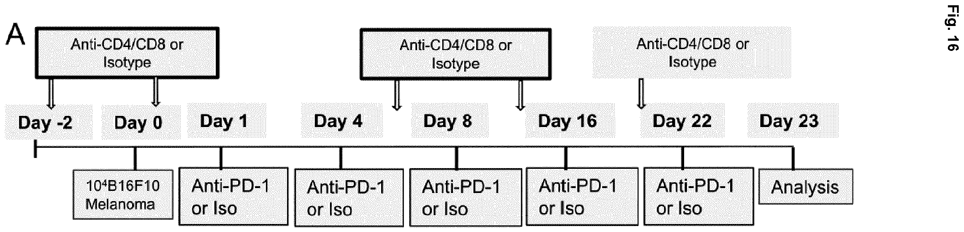

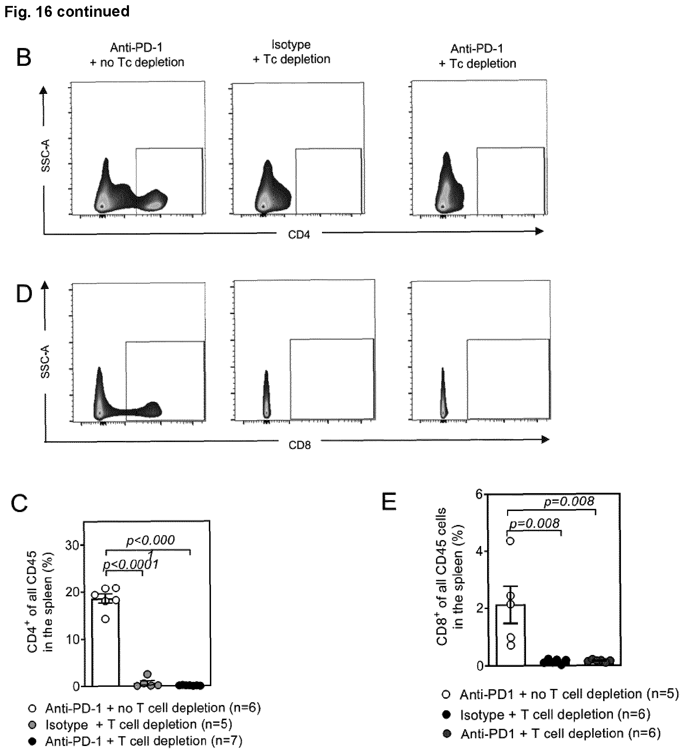

**Figure 16****:** Treatment sketch for T cell depletion mouse model. **(A)** The treatment scheme is depicted. C57BL/6 mice were treated with 250µg of anti-CD4/CD8 or isotype antibodies on day - 2, 0, 5, 15, 21. On day 0, mice received an i.v. injection of 10,000 B16F10Luc⁺GFP⁺ melanoma cells and were treated with 250 µg of either anti-PD-1 or isotype antibody at the indicated time points. Analysis was performed on day 23 after melanoma injection. **(B-E)** Representative flow cytometry plots and scatter plots depicting the frequencies of CD4⁺ and CD8⁺ cells from the spleens of melanoma bearing mice treated with either anti-PD-1 or Isotype control and receiving either anti-CD4/CD8 depleting antibodies or isotype antibodies.

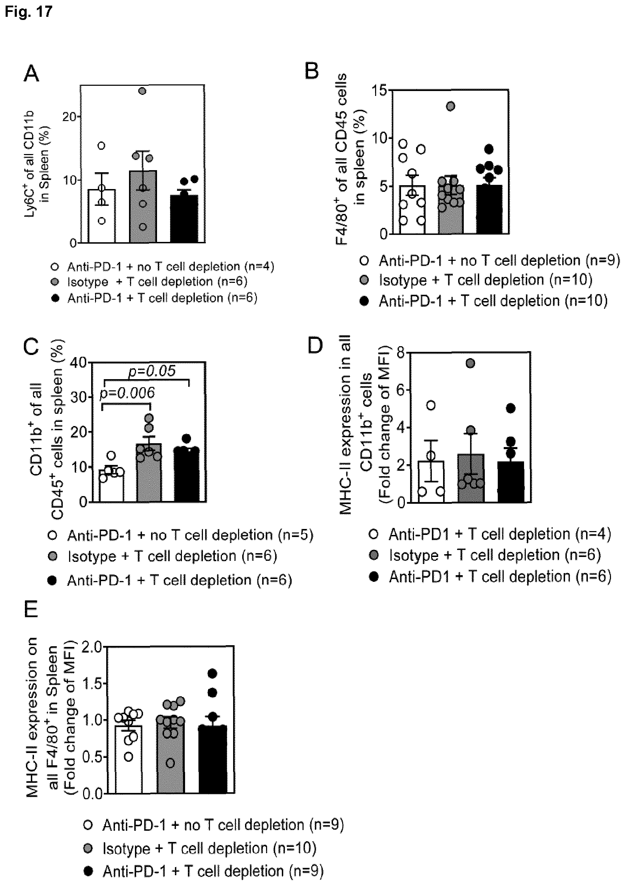

**Figure 17****:** Impact of T cell depletion on peripheral immune cells. **(A-B)** The scatter plot shows the quantification (percentage) of Ly6C⁺ cells of all CD11b⁺ cells **(A)** and F4/80⁺ cells of CD45⁺ cells **(B)** isolated from the spleen of melanoma-bearing mice treated with either anti-PD-1 with no T cell depletion or isotype/anti-PD-1 with T cell depletion antibodies. The experiment was performed once. **(C)** The scatter plot shows the quantification (percentage) of CD11b⁺ cells isolated from the spleen of melanoma-bearing mice treated with either anti-PD-1 with no T cell depletion or isotype/ anti-PD-1 with T cell depletion antibodies. The experiment was performed once. *P*-values were calculated using Student's unpaired *t*-test. **(D-E)** The scatter plot shows the quantification (fold change of MFI) of MHC-II expression from CD11b⁺ **(D)** and F4/80⁺ **(E)** cells isolated from the spleen of melanoma-bearing mice treated with either anti-PD-1 with no T cell depletion or isotype/anti-PD-1 with T cell depletion antibodies. The experiment was performed once.

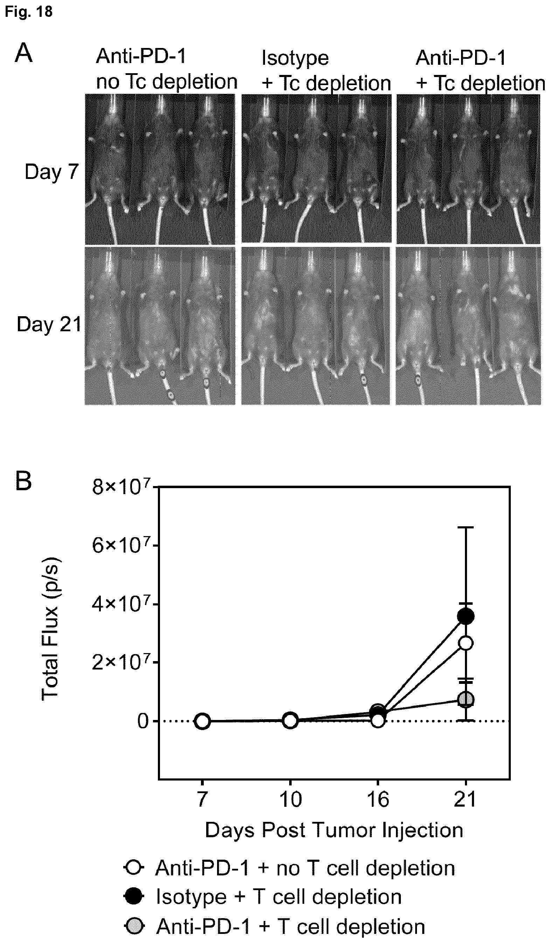

**Figure 18****:** Tumor burden in melanoma bearing mice that received T cell depletion or no T cell depletion. **(A-B)** BLI images **(A)** and quantification of melanoma burden **(B)** from mice treated with either anti-PD1 or isotype antibody and receiving either anti-CD4/CD8 T cell-depleting antibodies or isotype antibodies.

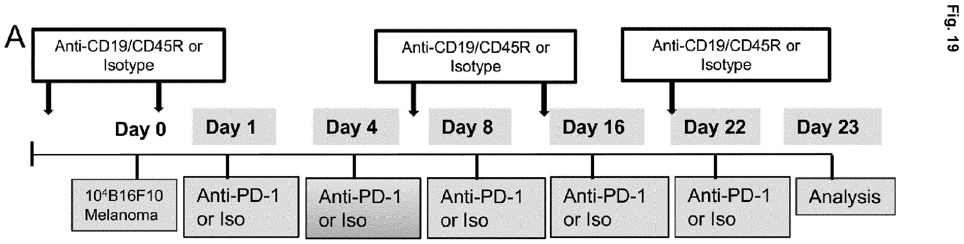

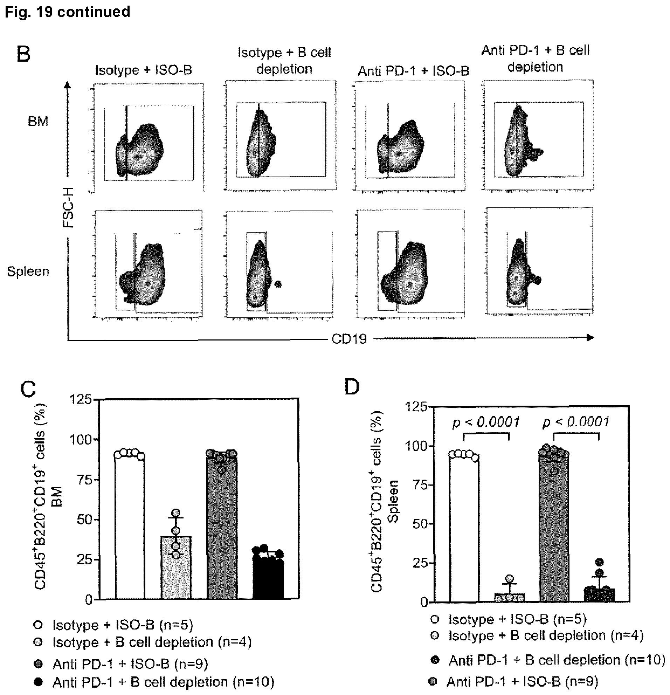

**Figure 19****:** Treatment sketch for B cell depletion mouse model. (A) The treatment scheme is depicted. C57BL/6 mice were treated with 250µg of anti-CD19/CD45R or isotype antibodies on day -2, 0, 5, 15, 21. On day 0, mice received an i.v. injection of 10,000 B16F10Luc⁺GFP⁺ melanoma cells and were treated with 250 µg of either anti-PD-1 or isotype antibody at the indicated time points. Analysis was performed on day 23 after melanoma injection. **(B-D)** Representative flow cytometry plots and scatter plots depicting the frequencies of CD19⁺ cells from the bone marrow (BM) or spleens of melanoma bearing mice treated with either anti-PD-1 or Isotype control and receiving either anti-CD19/CD45R antibodies or isotype antibodies.

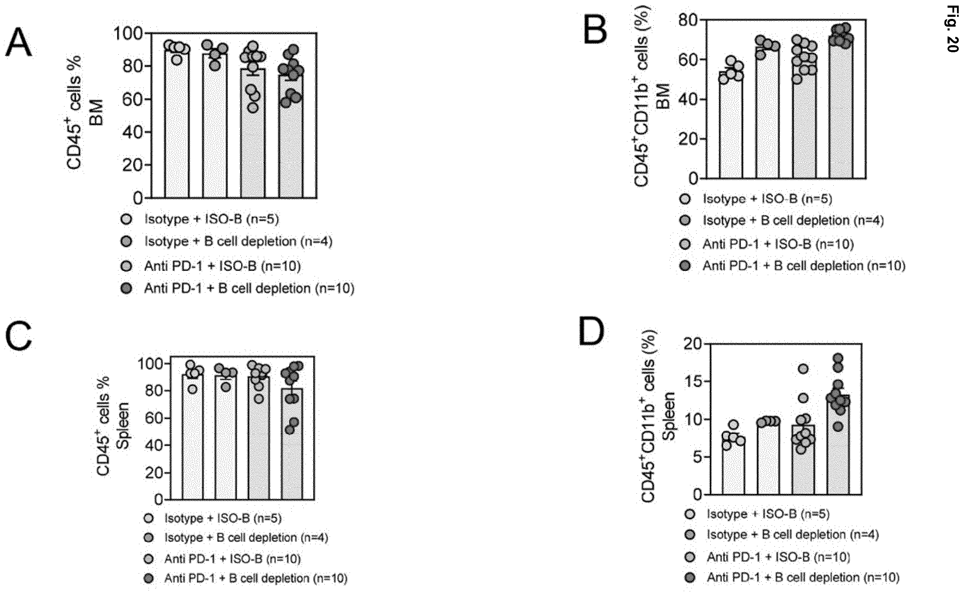

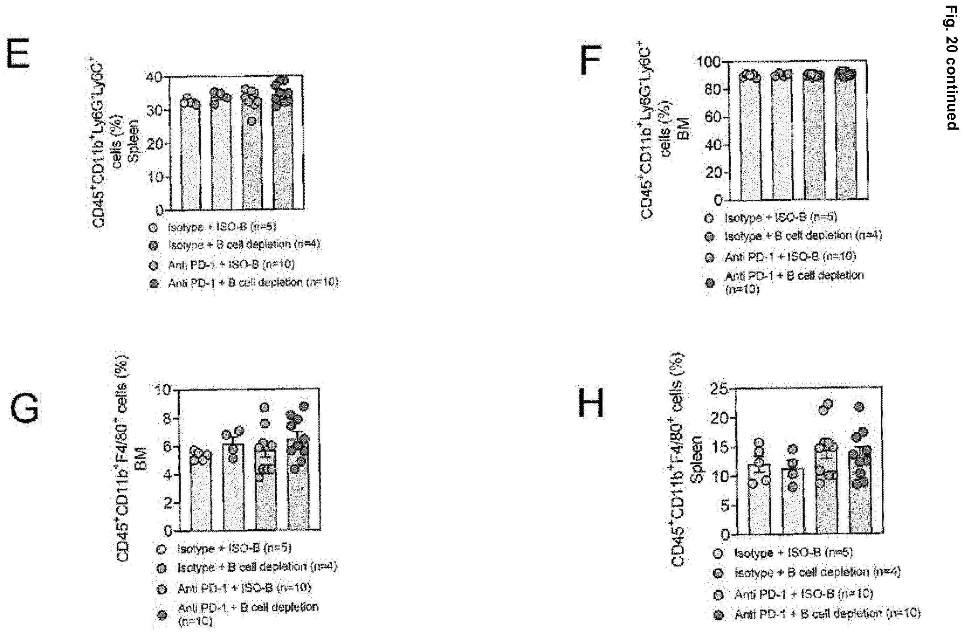

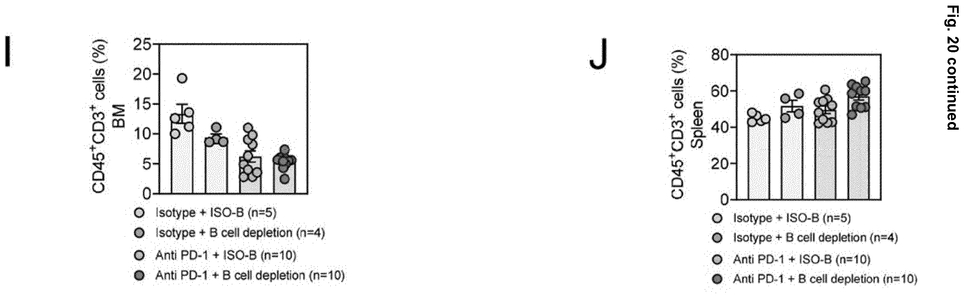

**Figure 20****:** Impact of B cell depletion on peripheral immune cells. **(A-J)** The scatter plot shows the quantification (percentage) of different cell types relative to all cells in the BM or spleen as indicated of melanoma-bearing mice treated with either anti-PD-1 with no B cell depletion or isotype/anti-PD-1 with B cell depletion antibodies. The experiment was performed once. **(A)** The scatter plot shows the quantification (percentage) of CD45⁺ cells of all cells in the BM. **(B)** The scatter plot shows the quantification (percentage) of CD45⁺ CD11b⁺ cells of all cells in the BM. **(C)** The scatter plot shows the quantification (percentage) of CD45⁺ cells of all cells in the spleen. **(D)** The scatter plot shows the quantification (percentage) of CD45⁺ CD11b⁺ cells of all cells in the spleen. **(E)** The scatter plot shows the quantification (percentage) of CD45⁺ CD11b⁺ Ly6G⁻ Ly6C⁺ cells of all cells in the spleen. **(F)** The scatter plot shows the quantification (percentage) of CD45⁺ CD11b⁺ Ly6G⁻ Ly6C⁺ cells of all cells in the BM. **(G)** The scatter plot shows the quantification (percentage) of CD45⁺ CD11b⁺ F4/80⁺ cells of all cells in the BM. **(H)** The scatter plot shows the quantification (percentage) of CD45⁺ CD11b⁺ F4/80⁺ cells of all cells in the spleen. **(I)** The scatter plot shows the quantification (percentage) of CD45⁺ CD3⁺ cells of all cells in the BM. **(J)** The scatter plot shows the quantification (percentage) of CD45⁺ CD3⁺ cells of all cells in the BM.

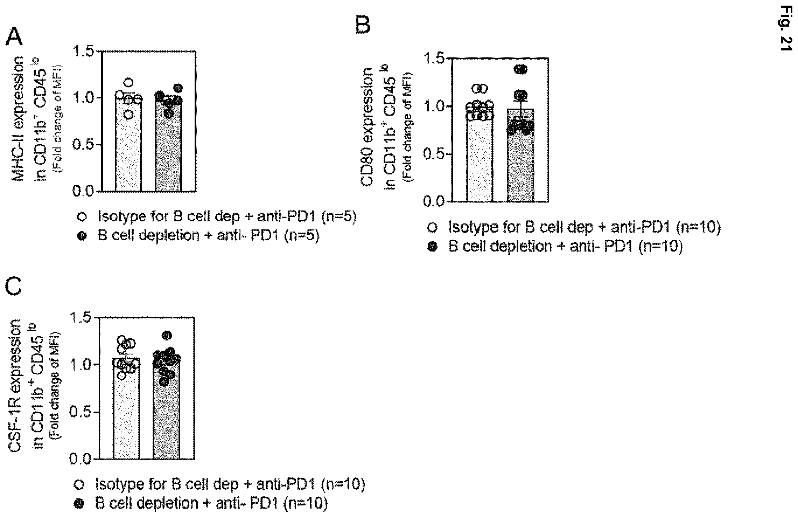

**Figure 21****:** Impact of B cell depletion on MHC II, CD80 and CSF-1R expression on microglia. **(A-C)** The scatter plot shows the quantification (fold change of MFI) of MHC-II (**A**), CD80 (**B**) and CSF-1R (**C**) expression on CD11b⁺ CD45^{low} cells isolated from the CNS of melanoma-bearing mice treated with either anti-PD-1 with no B cell depletion or isotype/anti-PD-1 with B cell depletion antibodies. The experiment was performed two times. *P*-values were calculated using Student's unpaired *t*-test.

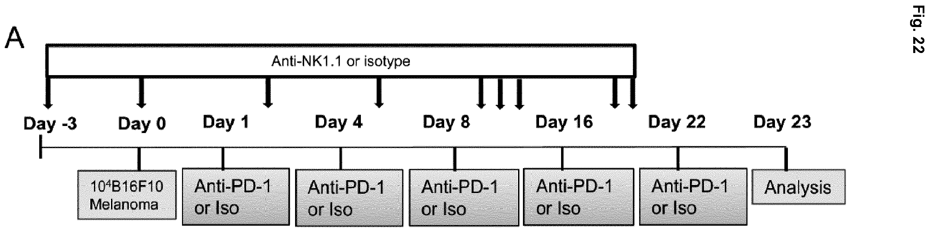

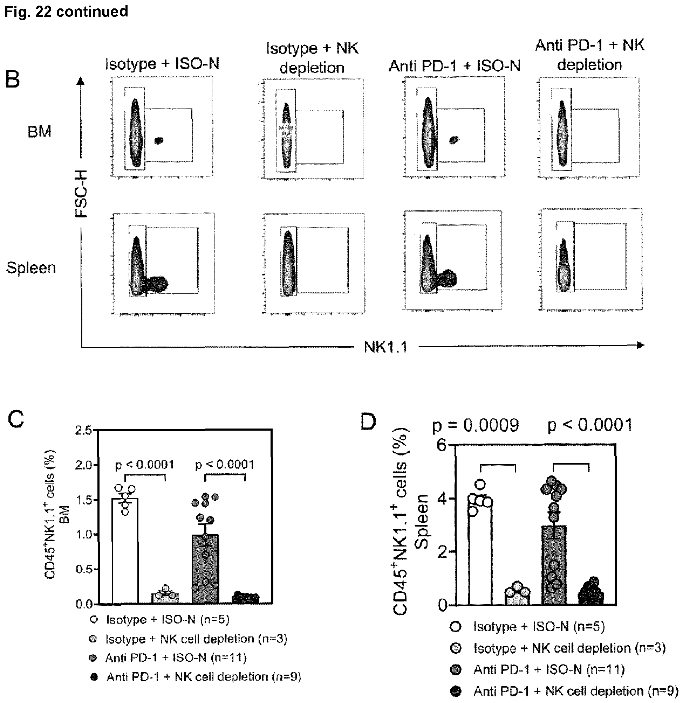

**Figure 22****:** Treatment sketch for NK cell depletion mouse model. **(A)** The treatment scheme is depicted. C57BL/6 mice were treated with 250µg of anti-NK1.1 or isotype antibodies on day -2, 0, 5, 15, 21. On day 0, mice received an i.v. injection of 10,000 B16F10Luc⁺GFP⁺ melanoma cells and were treated with 250 µg of either anti-PD-1 or isotype antibody at the indicated time points. Analysis was performed on day 23 after melanoma injection. **(B-D)** Representative flow cytometry plots and scatter plots depicting the frequencies of CD45⁺ NK1.1⁺ cells from the bone marrow (BM) or spleens of melanoma bearing mice treated with either anti-PD-1 or Isotype control and receiving either anti-NK1.1 antibodies or isotype antibodies.

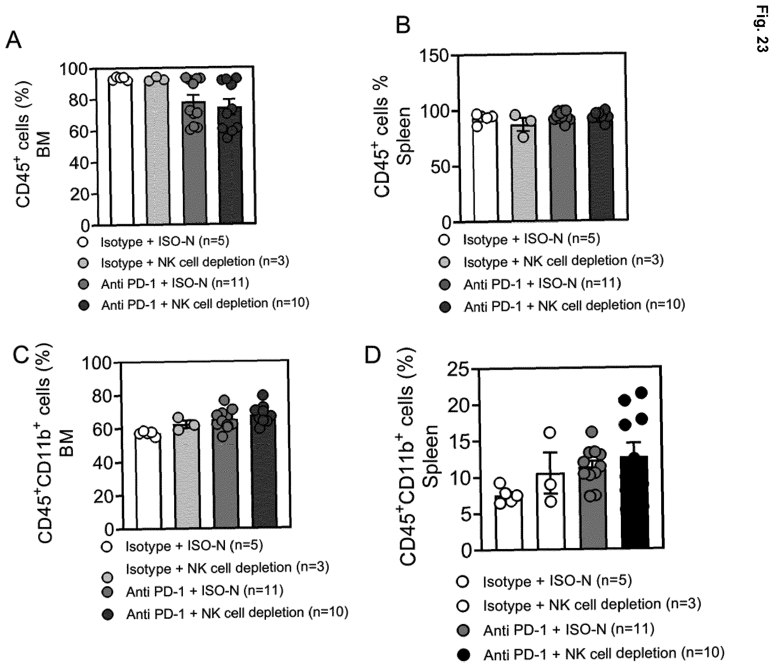

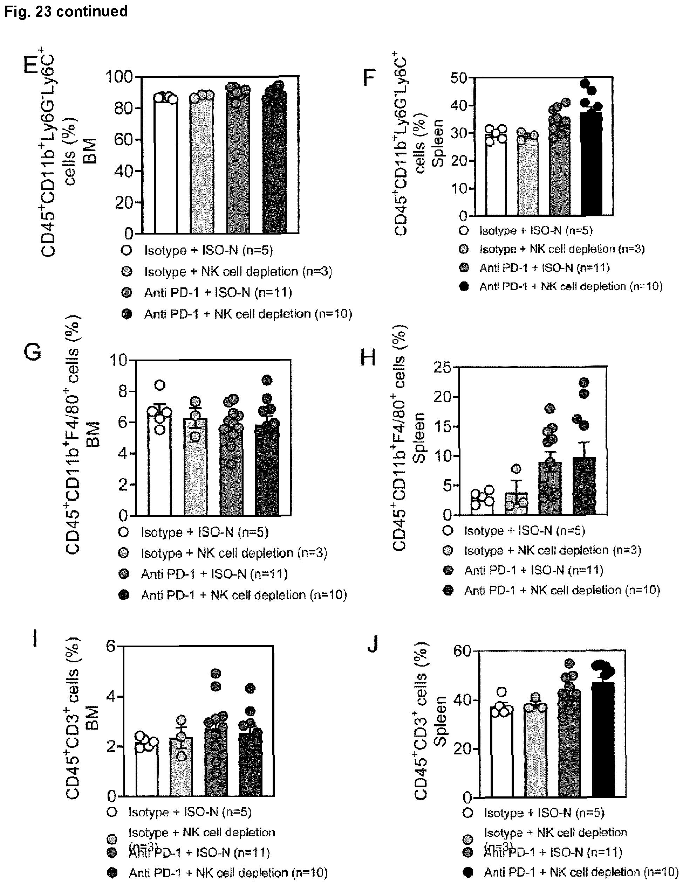

**Figure 23****:** Impact of NK cell depletion on peripheral immune cells. **(A-J)** The scatter plot shows the quantification (percentage) of different cell types relative to all cells in the BM or spleen as indicated of melanoma-bearing mice treated with either anti-PD-1 without NK cell depletion or isotype/anti-PD-1 with NK cell depletion antibodies. The experiment was performed once. **(A)** The scatter plot shows the percentage of CD45⁺ cells of all cells in the BM. **(B)** The scatter plot shows the percentage of CD45⁺ CD11b⁺ cells of all cells in the BM. **(C)** The scatter plot shows the quantification (percentage) of CD45⁺ cells of all cells in the spleen. **(D)** The scatter plot shows the quantification (percentage) of CD45⁺ CD11b⁺ cells of all cells in the spleen. **(E)** The scatter plot shows the quantification (percentage) of CD45⁺ CD11b⁺ Ly6G⁻ Ly6C⁺ cells of all cells in the spleen. **(F)** The scatter plot shows the quantification (percentage) of CD45⁺ CD11b⁺ Ly6G⁻ Ly6C⁺ cells of all cells in the BM. **(G)** The scatter plot shows the quantification (percentage) of CD45⁺ CD11b⁺ F4/80⁺ cells of all cells in the BM. **(H)** The scatter plot shows the quantification (percentage) of CD45⁺ CD11b⁺ F4/80⁺ cells of all cells in the spleen. **(I)** The scatter plot shows the quantification (percentage) of CD45⁺ CD3⁺ cells of all cells in the BM. **(J)** The scatter plot shows the quantification (percentage) of CD45⁺ CD3⁺ cells of all cells in the BM.

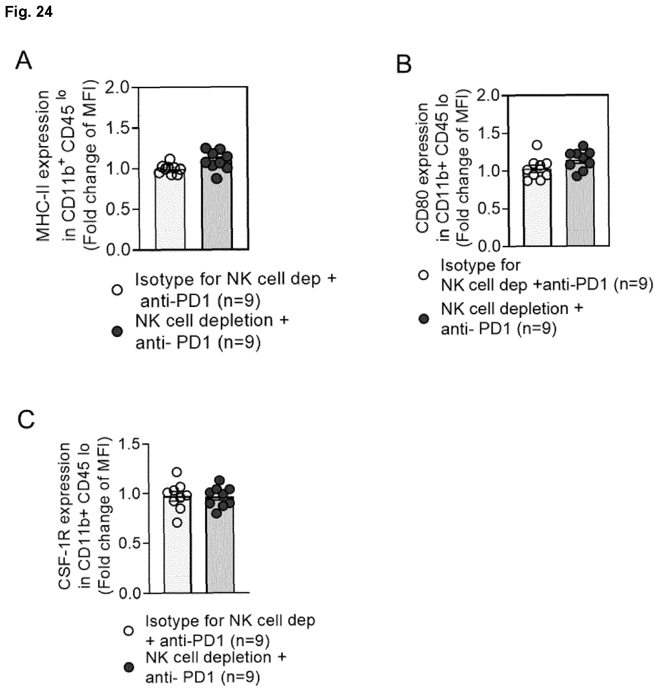

**Figure 24****:** Impact of NK cell depletion on MHC II, CD80 and CSF-1R expression on microglia. **(A-C)** The scatter plot shows the quantification (fold change of MFI) of MHC-II (**A**), CD80 (**B**) and CSF-1R (**C**) expression on CD11b⁺ CD45^{low} cells isolated from the CNS of melanoma-bearing mice treated with either anti-PD-1 with no NK cell depletion or isotype/anti-PD-1 with NK cell depletion antibodies. The experiment was performed two times. *P*-values were calculated using Student's unpaired *t*-test.

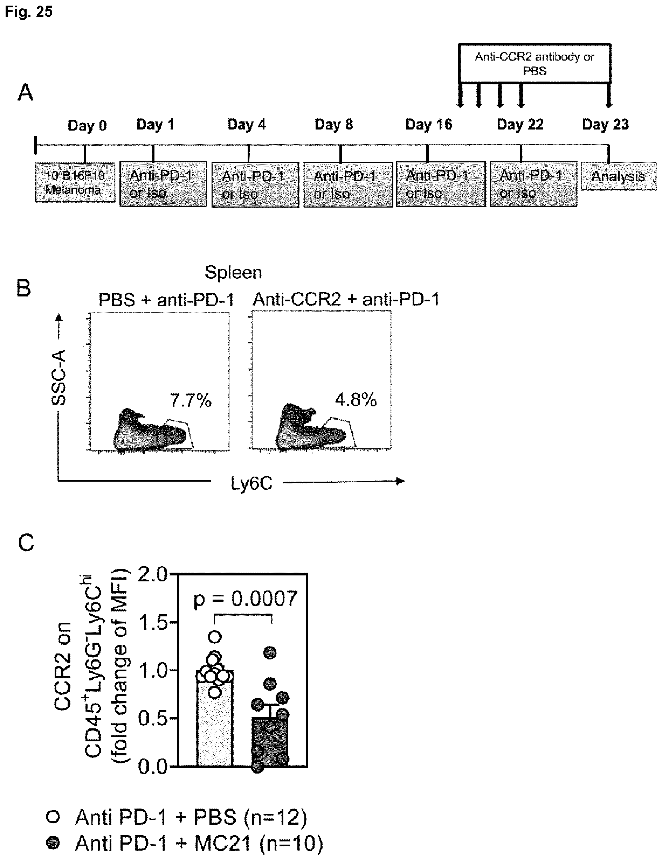

**Figure 25****:** Treatment sketch for CCR2⁺ cell depletion mouse model. **(A)** The treatment scheme is depicted. C57BL/6 mice were treated with 250µg of anti-CCR2 (MC21) or isotype antibodies on day -2, 0, 5, 15, 21. On day 0, mice received an i.v. injection of 10,000 B16F10Luc⁺GFP⁺ melanoma cells and were treated with 250 µg of either anti-PD-1 or isotype antibody at the indicated time points. Analysis was performed on day 23 after melanoma injection. **(B)** Representative flow cytometry plots depicting the frequencies of Ly6C⁺ cells from the spleens of melanoma bearing mice treated with either anti-PD-1 or Isotype control and receiving either anti-CCR2 antibodies or isotype antibodies. **(C)** Scatter plots depicting the MFI for CCR2 on CD45⁺ Ly6G⁻ Ly6C^{high} cells from the spleens of melanoma bearing mice treated with either anti-PD-1 or Isotype control and receiving either anti-CCR2 antibodies or isotype antibodies.

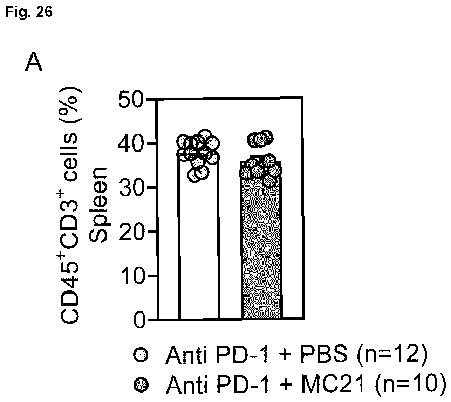

**Figure 26****:** Impact of CCR2⁺ cell depletion on splenic T cells. **(A)** Scatter plots depicting the quantification (percentage) of CD45⁺ CD3⁺ cells from the spleens of melanoma bearing mice treated with either anti-PD-1 or Isotype control and receiving either anti-CCR2 antibodies or isotype antibodies.

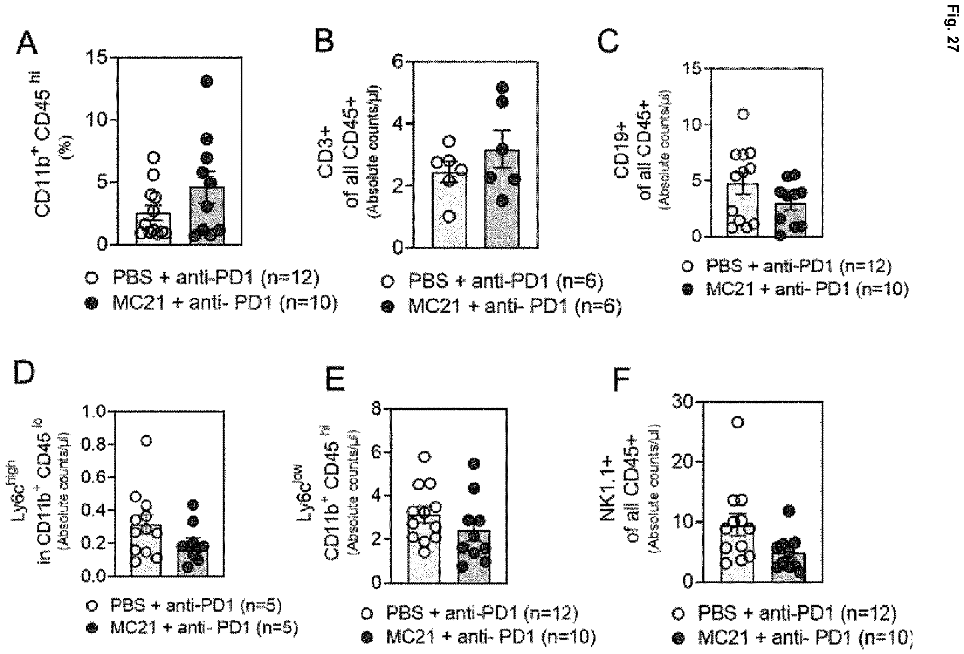

**Figure 27****:** Impact of CCR2⁺ cell depletion on peripheral immune cells. **(A-F)** The scatter plot shows the quantification (percentage) of different cell types relative to all cells in the spleen of melanoma-bearing mice treated with either anti-PD-1 without CCR2 depletion or isotype/anti-PD-1 with anti-CCR2 cell depletion antibodies. **(A)** The scatter plot shows the percentage of CD45⁺ CD11b⁺ cells of all cells in the spleen. **(B)** The scatter plot shows the quantification (absolute counts /µl) of CD3⁺ cells of all CD45⁺ cells in the spleen. **(C)** The scatter plot shows the quantification (absolute counts /µl) of CD19⁺ cells of all CD45⁺ cells in the spleen. **(D)** The scatter plot shows the quantification (absolute counts /µl) of Ly6C^{high} in CD45^{low} CD11b⁺ cells of all cells in the spleen. **(E)** The scatter plot shows the quantification (absolute counts /µl) of Ly6C^{low} in CD45^{high} CD11b⁺ cells of all cells in the spleen. **(F)** The scatter plot shows the quantification (absolute counts /µl) of CD3⁺ cells of all CD45⁺ cells in the spleen.

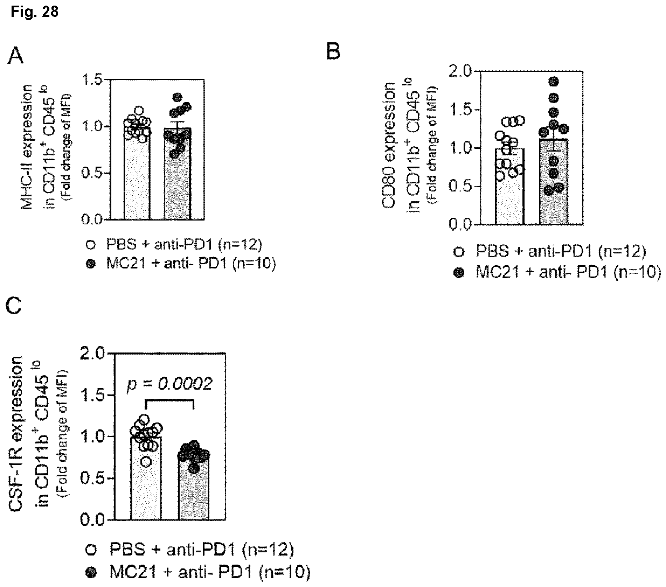

**Figure 28****:** Impact of CCR2⁺ cell depletion on MHC II, CD80 and CSF-1R expression on microglia. **(A-C)** The scatter plot shows the quantification (fold change of MFI) of MHC-II (**A**), CD80 (**B**) and CSF-1R (**C**) expression on CD11b⁺ CD45^{low} cells isolated from the CNS of melanoma-bearing mice treated with either anti-PD-1 without CCR2 depletion or isotype/anti-PD-1 with anti-CCR2 cell depletion antibodies. The experiment was performed two times. *P*-values were calculated using Student's unpaired *t*-test.

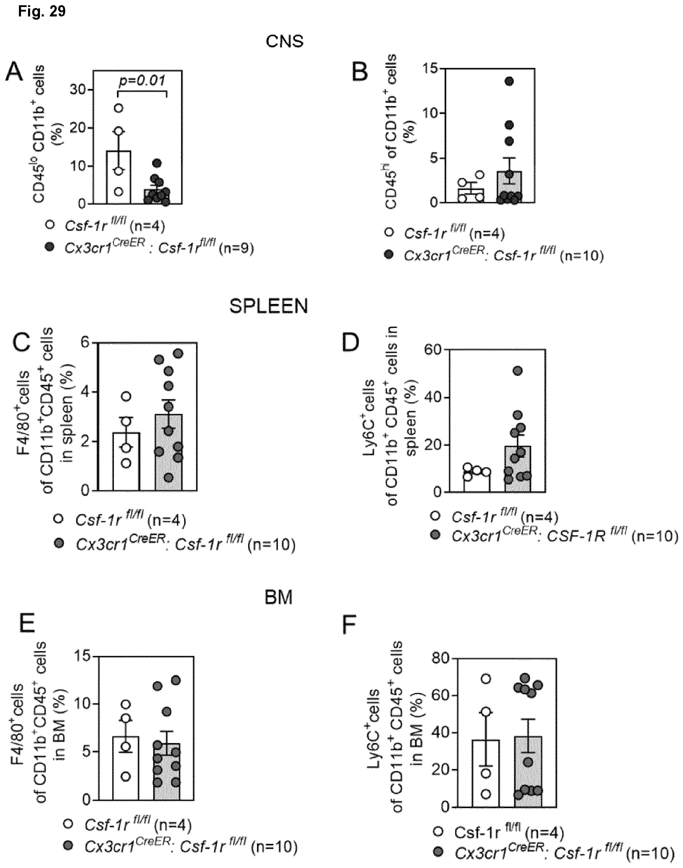

**Figure 29****:** Myeloid cells in CNS, spleen, and BM of *Cx3cr1^{CreER}: Csf-1r*^{*fl*/*fl*} mice. **(A-B)** The scatter plot shows the quantification (percentage) of CD45^{lo} **(A)** and CD45^{hi} **(B)** cells in CD11b⁺ cells isolated from the CNS of either *Cx3cr1^{CreER}: Csf-1r*^{*fl*/}*^{fl} or Csf-1r*^{*fl*/*fl*} melanoma-bearing mice treated with either anti-PD-1. The experiment was performed twice and the results (mean ± s.e.m) were pooled. *P*-values were calculated using Student's unpaired t-test. **(C-D)** The scatter plot shows the quantification (percentage) of F4/80⁺ **(C)** and Ly6C⁺ **(D)** cells isolated from the spleen of *Cx3cr1^{CreER}: Csf-1r*^{*fl*/*fl*} or *Csf-1r*^{*fl*/*fl*} melanoma-bearing mice treated with anti-PD-1. The experiments was performed twice and the results (mean ± s.e.m.) were pooled. P-values were calculated using Student's unpaired *t*-test. **(E-F)** The scatter plot shows the quantification (percentage) of F4/80⁺ **(E)** and Ly6C⁺ **(F)** cells isolated from BM of *Cx3cr1^{CreER}: Csf-1r*^{*fl*/*fl*} or *Csf-*1r^{fl/fl} melanoma-bearing mice treated with anti-PD-1. The experiment was performed twice and the results (mean ± s.e.m.) were pooled. *P*-values were calculated using Student's unpaired t-test.

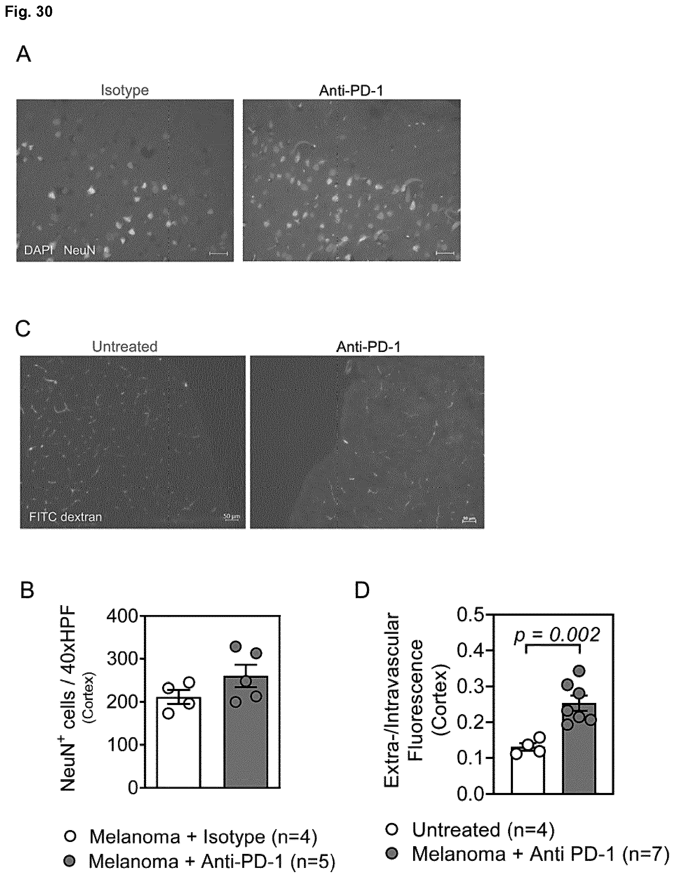

**Figure 30****:** Neuron counts and endothelial barrier permeability in melanoma bearing mice. (A-B) Analysis of the cortex immunostained for NeuN and DAPI derived from melanoma bearing mice that had received isotype or anti-PD-1. **(A)** Representative images are shown. Scale bars: 50µm. **(B)** Scatter plots show the number of NeuN⁺ cells (per mm²). The experiment was performed once. **(C)** Representative immunofluorescence images of the cortex of mice that had received FITC-dextran. BBB leakage correlates with extravasation of FITC-dextran. Melanoma bearing mice that were untreated or treated with anti-PD-1 antibody are shown. Scale bars: 30µm. **(D)** The scatter graph shows the quantification of the ratio of extravascular versus intravascular FITC-Dextran fluorescence in the cortex from untreated or melanoma bearing mice treated with anti-PD-1.The experiment was performed twice. *P*-values were calculated using Student's unpaired t-test.

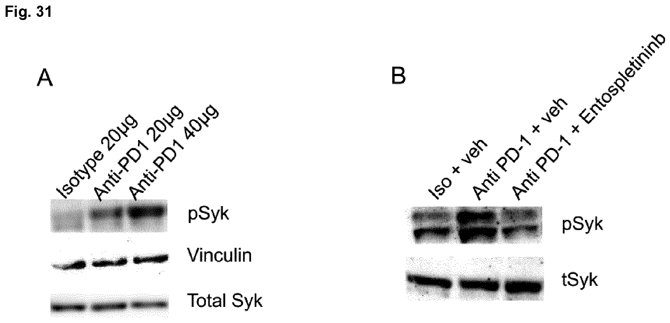

**Figure 31****:** Anti-PD-1 antibody dose dependent effects on Syk activation. **(A)** Representative western blot for total Syk and phosphor-Syk using protein derived from primary murine microglia treated with 20µg, 40µg or 60 µg of anti-PD-1 for 5 min when indicated. **(B)** Representative western blot for total Syk and phosphor-Syk using protein derived from primary murine microglia treated with 40µg of anti-PD-1 alone or with entosplenib for 5 min when indicated.

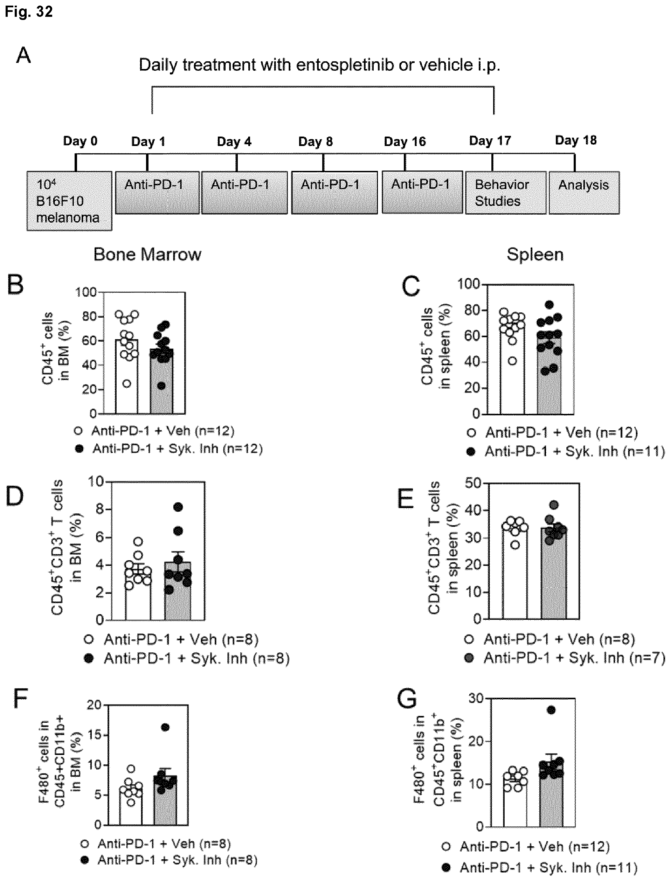

**Figure 32****:** Treatment sketch for the melanoma mouse model including vehicle or Syk inhibitor treatment. **(A)** The treatment schematic is depicted. Mice were injected i.v. with B16F10Luc⁺GFP⁺ melanoma cells on day 0 and then treated with 250µg of anti-PD-1 antibody at the indicated time points. Additionally, vehicle or Syk inhibitor (Entosplenitib, 40mg/kg per i.p. injection) were injected daily from day 1 to day 17. **(B, D, F)** The scatter plot shows the quantification (percentage) of CD45⁺ **(B),** CD3⁺**(D)** and F4/80 **(F)** cells isolated from the BM of melanoma-bearing mice treated with anti-PD-1/isotype and Syk inhibitor/vehicle. The experiment was performed once. *P*-values were calculated using Student's unpaired t-test. **(C, E, G)** The scatter plot shows the quantification (percentage) of CD45⁺ **(C),** CD3⁺ **(E)** and F4/80⁺ **(G)** cells isolated from the spleen of melanoma-bearing mice treated with anti-PD-1/isotype and Syk inhibitor/vehicle. The experiment was performed once. *P*-values were calculated using Student's unpaired *t*-test.

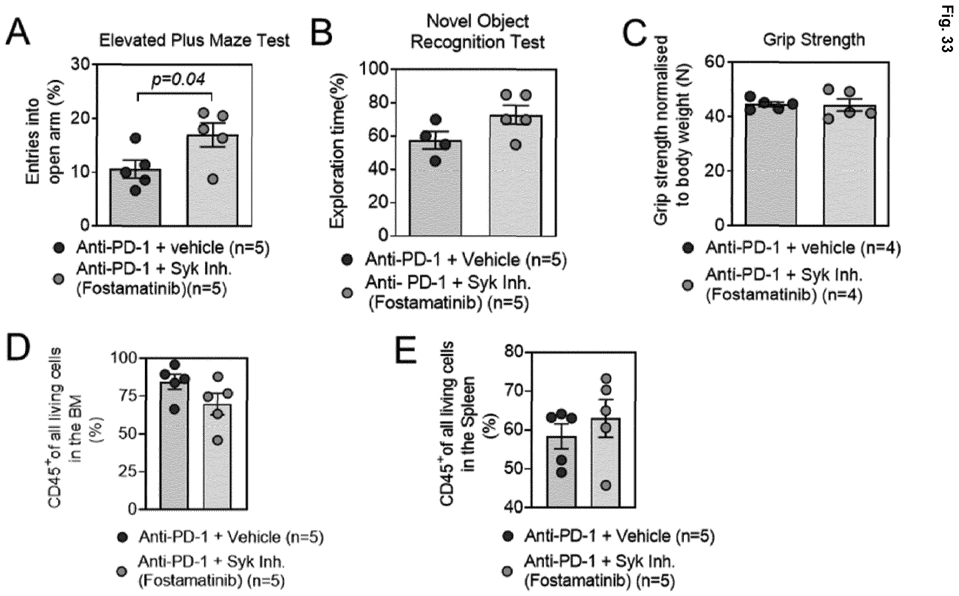

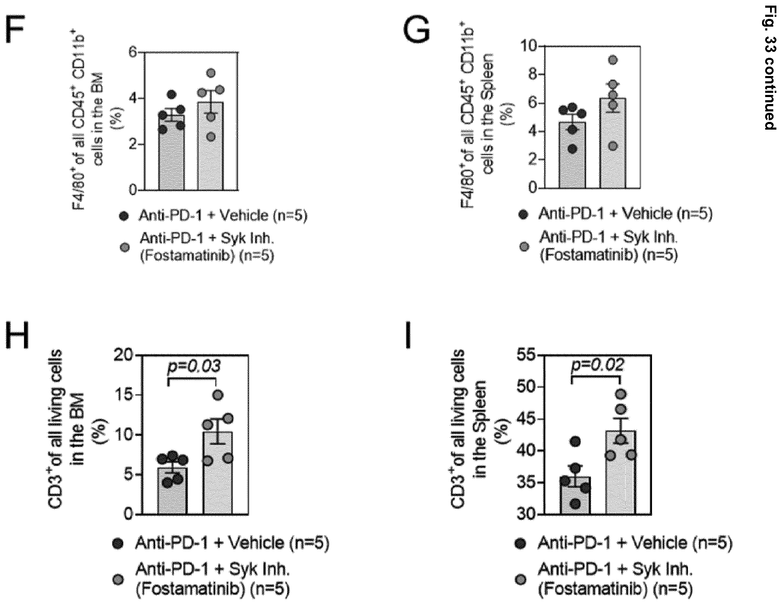

**Figure 33****:** Effects of the second Syk inhibitor (Fostamatinib) treatment. **(A)** The scatter plot shows the entries into open arms during the elevated plus maze of melanoma bearing mice treated with anti-PD-1 and Syk inhibitor or vehicle. **(B)** The scatter plot shows the time spent exploring a novel object with respect to the total time in the novel object recognition test by melanoma bearing mice treated with anti-PD-1 and Syk inhibitor or vehicle. **(C)** The scatter plot shows the grip strength normalized to body weight (N/kg) of melanoma bearing mice treated with anti-PD-1 and Syk inhibitor/vehicle. **(D, F, H)** The scatter plot shows the quantification (percentage) of CD45⁺ **(D),** F4/80⁺ **(F)** and CD3⁺ **(H)** cells isolated from the BM of melanoma-bearing mice treated with anti-PD-1/isotype and Syk inhibitor/vehicle. The experiment was performed once. *P*-values were calculated using Student's unpaired *t*-test. **(E, G, I)** The scatter plot shows the quantification (percentage) of CD45⁺ **(E),** F4/80⁺ **(G)** and CD3⁺ **(I)** cells isolated from the spleen of melanoma-bearing mice treated with anti-PD-1/isotype and Syk inhibitor/vehicle. The experiment was performed once. *P*-values were calculated using Student's unpaired *t*-test.

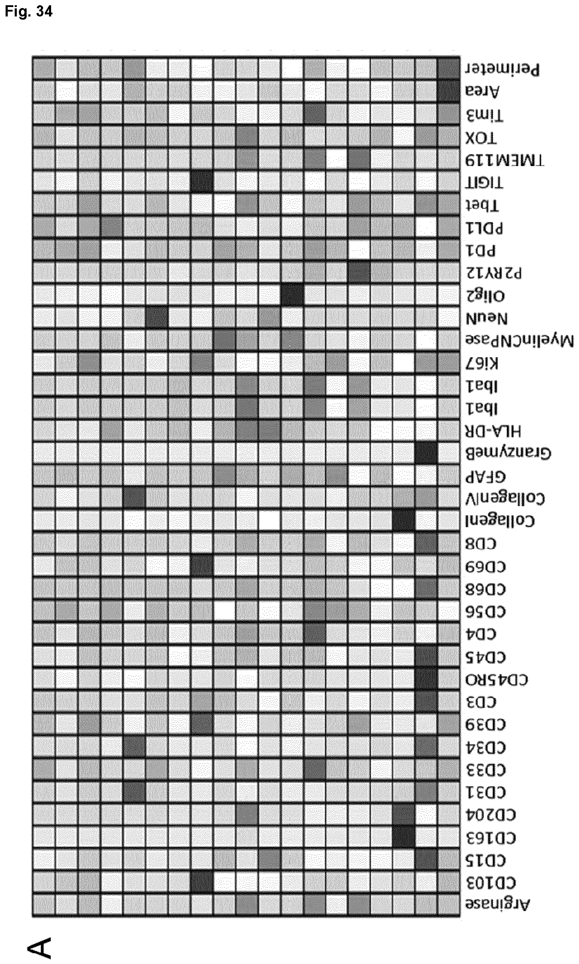

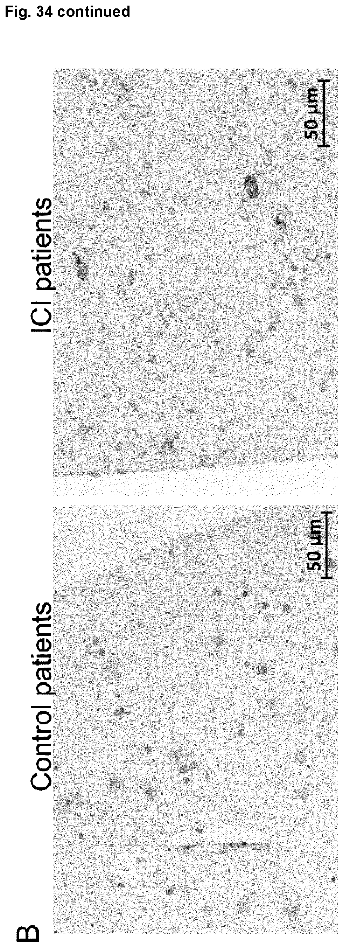

**Figure 34****:** IMC based clustering of myeloid cells and representative images of CD68⁺ cells from frontal cortex samples. **(A)** Heat map representing the clustering of diverse populations generated via CYTOF analysis on human autopsied tissues isolated from ICI treated or control brains. **(B)** Immunohistochemistry for CD68⁺ cells of frontal cortex samples derived from control patients or patients treated with anti-PD-1. Representative images from each group are shown. Scale bars, 50µm.

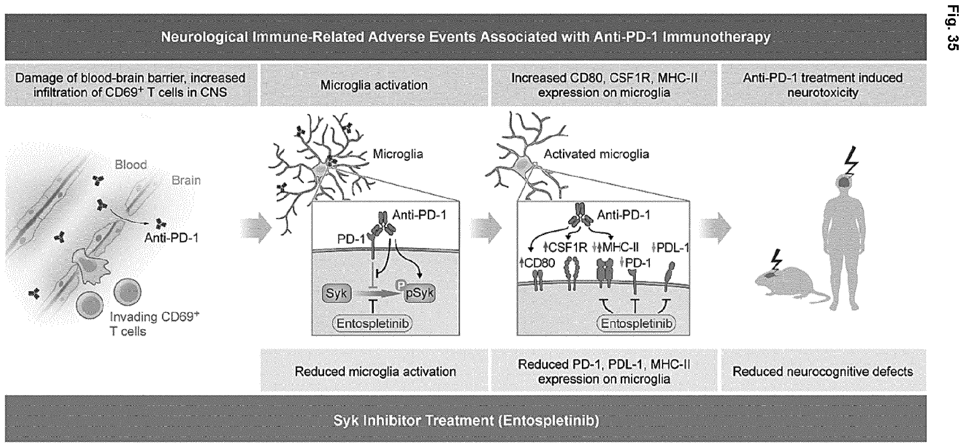

**Figure 35****:** Graphical abstract. Anti-PD-1 treatment led to increased MHC-II expression on microglia. Activated microglia upregulated CSF-1R, CD80, and F4/80. This was associated with cognitive defects. In addition, anti-PD-1 treatment induced Syk activation in microglia. Syk inhibition reduced microglial activation and improved cognitive functions.

### EXAMPLES

The invention is further described by the following examples. These are not intended to limit the scope of the invention but represent preferred embodiments of aspects of the invention provided for greater illustration of the invention described herein.

### Material and methods

### Study Design

To investigate the pathomechanisms of CNS-irAEs, the inventors used different irAE mouse models and studied the contribution of microglia, myeloid cells and T cells to CNS-irAEs. Behavior studies were employed to study the neurocognitive defects in response to anti-PD-1 treatment. The inventors employed single cell sequencing and kinase assays to profile microglia. Genetic and pharmacological depletion/inhibition studies were performed to further support microglial contribution to CNS-irAEs. All experiments were independent of each other and performed two to three times.

### Human tissue analysis and patients

The analysis of human tissue samples was approved by the Ethic committee of the University of Freiburg (protocol number 340/19, Analysis of CNS autopsy material and CSF in patients that received anti-PD-1 immunotherapy). To study microglial activation in ICI patients, post-mortem FFPE brain tissues of ICI cohort and control cohort were analyzed by IHC and IMC.

### Mice

For all experiments, six to twelve-week-old mice were used that were randomized into experimental groups prior to the experiment. The mice were kept under specific pathogen-free conditions in individually ventilated cagesC57BL/6 (H-2Kb, Thy-1.2) and BALB/c (H-2Kd, Thy-1.2) mice were purchased either from Janvier Labs (France) or from the local stock of the animal facility at University of Freiburg. Cx3cr1creER: Csf1 rfl/fl mice were generated by crossing Csf1 rfl/fl and Cx3cr1 creER mice. The Cx3cr1 creER mice had been previously described (17). Mice were used between 6 and 14 weeks of age.

### Anti-PD-1 treatment (no tumor) model

BALB/c or C57BL/6 mice were treated with 250 µg of either anti PD-1 or Isotype control via intra peritoneal injections on Day 1, 4, 8, 11, 15, 18 and 22 (Fig. 9).

### Statistical analysis

For the sample size in the murine experiments a power analysis was performed. A sample size of at least n=8 per group was determined by 80% power to reach a statistical significance of 0.05 to detect an effect size of at least 1.06. Differences in animal survival (Kaplan-Meier survival curves) were analyzed by Mantel Cox test. The experiments were performed in a non-blinded fashion except for the analysis of human tissue specimens and behavioural experiments in knockout mice. For analysis of human brain specimens, samples were allocated to the group after finalization of the analysis. All samples or mice were included in our analysis.

For statistical analysis of 2 groups an unpaired 2 tailed Student's t test was applied. All data were tested for normality applying the Kolmogorov-Smirnov test. If the data did not meet the criteria of normality, the Mann-Whitney U test was applied. If more than 2 groups were analyzed the Kruskal-Wallis-Test was used if non-parametric testing was suggested and a one-way ANOVA was performed in case of normally distributed data. Statistical analysis was performed using GraphPad Prism (GraphPad Software; San Diego, CA). Data are presented as mean and s.e.m. (error bars). Differences were considered significant when the P-value was <0.05.

### Results

### Morphological signs of microglia activation upon anti-PD-1 treatment in the absence of tumor

To characterize the impact of anti-PD-1 treatment on microglia in the absence of tumor-related effects, healthy mice were treated with anti-PD-1 or isotype controls (Fig. 9A). Using Iba-1 as a marker for myeloid cells, the inventors observed increased numbers of lba-1+ cells in the cortex and hippocampus of mice treated with anti-PD-1 compared to isotype controls (Fig. 1A-C). Additionally, the frequency of activated CD11b+ cells with high CD45 expression was increased after anti-PD-1 treatment (Fig. 9B and C). Microglia from anti-PD-1 treated mice exhibited morphological changes with reduced filament length, fewer dendrites, and fewer branching and terminal dendrite points, indicative of a more activated phenotype (Fig. 1D-H). Consistent with CNS entry of anti-PD-1, other investigators had previously found anti-PD-1 in the CNS of patients and in concentrations in cerebrospinal fluid (CSF) that are sufficient to block PD-1 on T cells (25). Accordingly, the inventors observed that the anti-PD-1 treatment disrupted the blood-brain barrier (BBB) as determined by extravasation of FITC-dextran (Fig. 2A and B, Fig. 9D and E) and increased GFAP fluorescence intensity in the hippocampus (Fig. 9F-G). These findings support the concept that anti-PD-1 immunotherapy leads to microglia activation in the absence of tumor cells, causes BBB leakage, and can penetrate the CNS following systemic injection.

### Anti-PD-1 treatment causes increased CSF-1R and MHC-II expression on microglia and neurocognitive deficits

Next, the inventors characterized the impact of anti-PD-1 treatment on the phenotype of microglia in more detail. Microglia (CD11b+CD45lo) exhibited increased colony stimulating factor 1 receptor (CSF-1R, CD115) and MHC-II expression after anti-PD-1 treatment (Fig. 2C-F). To understand the potential contribution of T cells in mediating these inflammatory responses, T cell frequencies in response to anti-PD-1 therapy were quantified. There was a significant increase in the CD3⁺ T cell counts in the meninges, cortex, cerebellum and hippocampus (Fig. 10A-E). In addition, the inventors noted increased frequencies of CD3+, CD4+, CD4+CD69+ and CD4+CD154+ T cells infiltrating the CNS of mice that received anti-PD-1 (Fig. 10F-I). To understand if increased microglia activation was mediated by a direct effect of the PD-1 antibody, primary microglia were exposed to two different anti-PD-1 antibodies (clones RMP1-14 and J43) in vitro. Primary microglia (CD11b+CD45+) exhibited increased MHC-II expression upon anti-PD-1 exposure in vitro (Fig. 2G-J). To explore the impact of anti-PD-1 treatment on neurocognitive function, behavioral studies were performed with mice treated with isotype or anti-PD-1 immunotherapy. The inventors surprisingly observed that memory and exploratory function were reduced, while general strength was intact in mice treated with anti-PD-1 as compared to isotype control (Fig. 2K-M). These findings indicate a direct effect of anti-PD-1 on microglia in vitro, and a negative impact of anti-PD-1 treatment on neurocognitive function in vivo. To clarify if the anti-PD-1 treatment effect on microglia activation is indirect, meaning mediated by T cells, B cells or NK cells, RAG2-/- Cy -/- mice, which lack these cell types, were treated with anti-PD-1 or isotype antibodies. When analyzing microglia morphology it was observed typical signs of microglia activation in the group that received anti-PD-1 treatment compared to the isotype control group (Fig. 2N-R). Additionally, the inventors found that the markers TREM119 and P2RY12, which are markers for resting microglia, declined upon anti-PD-1 treatment compared to the isotype control group (Fig. 2S, T). These findings indicate that microglia activation upon anti-PD-1 treatment occurs independent of T cells, B cells and NK cells.

### Microglia activation by anti-PD-1 treatment occurs in the presence of tumor and colitis and is connected to increased T cell infiltration of the CNS

The inventors next aimed to study the impact of anti-PD-1 treatment on microglia in the presence of growing melanoma, which in a more clinically relevant setting. The inventors therefore used a previously described B16 melanoma model and treated the mice with anti-PD-1 at a previously described dose and schedule (26) for 23 days (Fig. 11A). The inventors observed that anti-PD-1 treated mice exhibited higher numbers of Iba1+ cells in the cortex than mice treated with isotype control (Fig. 3A and B). Additionally, CD45, a marker for microglia/myeloid activation, was increased on CD11b+ cells in anti-PD-1 treated mice compared to mice treated with isotype control (Fig. 11B and C). Increased expression of MHC-II and CSF-1R was observed in CD45hi CD11b+ cells in anti-PD-1 treated mice compared to mice treated with isotype control (Fig. 11D and E). Microglia isolated from anti-PD-1 treated mice exhibited morphological changes with reduced filament length, fewer dendrites, and less branching points and terminal dendrite points (Fig. 3C-G). To understand if anti-PD-1 immunotherapy was connected CNS infiltration by different immune cells, T cell, B cell and NK cell frequencies in the CNS were analyzed. The results showed increased numbers of CD3⁺ T cells, activated CD4+CD69+ and CD4+CD25+ T cells and a trend towards more B cells and NK cells in the brains of melanoma-bearing mice treated with anti-PD-1 immunotherapy compared to isotype control (Fig. 3H-L).

### Anti-PD-1 treatment induces major transcriptional changes in microglia on the single cell level

To characterize the impact of anti-PD-1 treatment on the transcriptional activity of microglia in melanoma bearing mice, mice were treated with anti-PD-1 or isotype control and the CNS was isolated on day 23 after melanoma injection. Nuclei from the cerebral cortex of the CNS were sorted and analyzed using 10x Genomics single-nuclei 3' mRNA sequencing (snRNA Seq). The resulting dataset comprised several sub-clusters of different cell types, including microglia, CNS-associated macrophages (CAMs), oligodendrocytes and others (Fig. 4A), according to their marker gene expression (Fig. 12A). Cell type assignment was conducted using the Azimuth algorithm based on human peripheral blood mononuclear cells with known transcriptomic and cell surface marker profiles (27). Gene expression in the microglia cluster was fundamentally different in the anti-PD-1 group compared to the isotype group (Fig. 4B). To decipher the differential gene expression in more detail, four microglia clusters were identified (Fig. 4C). When assigning these cluster to treatment groups, clusters 1 and 2 were linked to anti-PD-1 treatment, while cluster 3 was mainly found in the isotype control group (Fig. 4D). The clusters 1 and 2 (anti-PD-1 treatment) exhibited increased expression of Syk, C1qa, C1qb, Tyrobp, H2-D1 and Trem2 while cluster 3 (isotype control) exhibited Ikzf1 expression (Fig. 4E and F). At the protein level, microglia isolated from melanoma bearing mice treated with anti-PD-1 exhibited increased expression of MHC-II, F4/80, CD80 and CSF-1R compared to melanoma- bearing mice treated with isotype (Fig. 5A-H). Besides microglia other myeloid cells in the CNS of anti-PD-1 treated mice were quantified, revealing increased frequencies of CD11b+Ly6C+ cells (Fig. 5I-L). To investigate the functional implications of anti-PD-1 treatment, behavioral studies with melanoma-bearing mice treated with isotype or anti-PD-1 were performed. The results showed a specific reduction in memory and exploratory functions, while general strength was intact in mice treated with anti-PD-1 (Fig. 5M-O). These findings indicate that anti-PD-1 treatment increases expression of pro-inflammatory genes in microglia and impairs neurocognitive performance in melanoma-bearing mice. To solidify the link between PD-1 blockade and microglia inflammation, the inventors studied dextran sodium sulfate (DSS)-induced colitis in combination with anti-PD-1 treatment (Fig. 13A). In this model, it was found that anti-PD-1 treatment aggravated colitis as evidenced by increased neutrophil and lymphocyte infiltration into the colon (Fig. 13B-D). Anti-PD-1 treatment induced increased expression of CD80 and CSF-1R on microglia and increased numbers of Iba-1+ cells in mice with colitis compared to isotype treated controls (Fig. 13E-J). Based on these data the inventors concluded that anti-PD-1 treatment induces microglia activation in melanoma and colitis settings. Microglia activation is associated with increased infiltration of activated T cells into the meninges and cortex.

### Anti-PD-1 treatment-induced neurocognitive defects are rescued by depletion of microglia but not T cells

To clarify, whether microglia were protective or disease-promoting in anti-PD-1 treatment induced CNS-irAEs, the CSF-1R inhibitor (PLX5622) chow (28) was supplied to deplete mononuclear phagocytes in the CNS (Fig. 14A-F). CSF-1R inhibitor treatment did not alter the frequencies of CD45, CD11b, Ly6C and CD11c populations in the BM and spleen (Fig. 14A-H). It could be observed that melanoma-bearing mice treated with isotype antibody combined with CSF-1R inhibitor diet or control diet did not develop neurocognitive deficits, as determined in novel object recognition test (Fig. 6A). Cognitive function was improved in anti-PD-1 treated mice receiving CSF-1R inhibition compared to control diet (Fig. 6B). Grip strength was intact independent of anti-PD-1 treatment and CSF-1R inhibitor diet (Fig. 6C and D). To test if these effects were specific to mononuclear phagocytes or may be achieved by reduction of other CNS infiltrating immune cells, anti-CD4/anti-CD8-based T cell depletion in the same model were analyzed (Fig. 16A). This achieved robust depletion of CD4 and CD8 T cells using this approach (Fig. 16B-E), yet T cell depleting antibodies did not affect neurocognitive function in isotype treated mice (Fig. 6E). T cell depletion did not prevent neurocognitive defects in mice treated with anti-PD-1 (Fig. 6F). Grip strength was intact independent of anti-PD-1 treatment and T cell depletion (Fig. 6G and H). Notably, T cell depletion did not alter frequencies of CD11b, F4/80 and Ly6C⁺ cells or MHC-II expression in the spleen (Fig. 17A-E) and melanoma burden was not affected (Fig. 18A and B). Next B cells were depleted to test if microglia activation and reduced cognitive function upon anti-PD-1 treatment can be reversed by this intervention. Anti-CD19/anti-CD45R-based B cell depletion caused robust reduction of CD19/CD45R+ B cells (Fig. 19A-D), without depletion of myeloid cells in spleen or bone marrow (Fig. 20A-J). B cell depletion did not reverse MHC-II, CD80 or CSF-1R expression on microglia induced by anti-PD-1 treatment (Fig. 21A-C) and neurocognitive deficits did not improve upon B cell depletion (Fig. 6I-K). To test the role of NK cells, NK cells were depleted and microglia activation and the cognitive function was analyzed. NK cell depletion caused robust reduction of NK cells (Fig. 22A-D), without depletion of myeloid cells in spleen or bone marrow (Fig. 23A-J). NK cell depletion did not reverse MHC-II, CD80 or CSF-1R expression on microglia induced by anti-PD-1 treatment (Fig. 24A-C) and neurocognitive deficits did not improve upon NK cell depletion (Fig. 6L-N). To reduce the infiltration by CCR2⁺ cells which were previously shown to be monocytes that enter the CNS upon inflammation (29) the inventors used an anti-CCR2 (MC21) antibody-based approach. Anti-CCR2-treatment caused a reduction of CCR2+Ly6Chigh cells in the spleen (Fig. 25A-C), without depletion of T cells (Fig. 26A). In the spleen and bone marrow, anti-CCR2-treatment caused a reduction of CSF1R+CD11b+ cells, which was expected, while multiple other cell types were not affected (Fig. 27A-I). Anti-CCR2-treatment did not reverse MHC-II, CD80 or CSF-1R expression on microglia induced by anti-PD-1 treatment (Fig. 28A-C) and neurocognitive deficits did not improve upon anti-CCR2-treatment (Fig. 6O, P). Next, the inventors more precisely targeted microglia by using a genetic depletion approach, i.e. Cx3cr1 CreER: Csfr1fl/fl mice (Fig. 29A and B), which show no alterations to myeloid cells in the spleen and BM (Fig. 29C-F). The novel object recognition test revealed that Cx3cr1CreER:Csfr1fl/fl mice exhibited improved neurocognitive function compared to Csfr1fl/fl mice (Fig. 6Q). Grip strength was intact independent of the genotype (Fig. 6R). These data indicate that depletion of microglia but not of T cells, B cells, NK cells or CCR2+ cells reduces anti-PD-1 treatment induced neurocognitive defects. To understand if the cognitive deficit was due to neuronal death, the inventors quantified the number of neurons (NeuN+) in the cortex and observed no changes induced by anti-PD-1 treatment (Fig. 30A and B). In addition, increased BBB leakage via extravasation of FITC-dextran was observed when comparing melanoma-bearing anti-PD-1 treated mice with non-tumor bearing untreated mice (Fig. 30C and D), supporting the concept that melanoma contributes to BBB impairment which may allow for the entry of anti-PD-1 into the CNS.

### Anti-PD-1 treatment causes Syk activation in microglia

To characterize the activation of kinases as potential therapeutic targets in microglia following anti-PD-1 immunotherapy, the inventors isolated microglia on day 23 after melanoma injection and isotype or anti-PD-1 treatment (Fig. 11A). Unbiased kinase profiling using the PamChip protein tyrosine kinases (PTK) assay revealed significantly increased phosphorylation (p<0.05) in 18 peptides in anti-PD-1 treated mice compared to isotype controls (Fig. 7A). Key altered tyrosine kinases included Src family kinases (FYN, SRC, LCK, YES, BLK, HCK, LYN), ABL1, and Syk kinase (Fig. 7B). In line with the kinase array results, increased Syk RNA expression in microglia derived from anti-PD-1-treated mice was also observed when analysed by snRNA seq (Fig. 4E). Herein the inventors first confirmed increased Syk phosphorylation in primary microglia following stimulation with anti-PD-1 (Fig. 7C and D), which was dose dependent and could be reduced by Syk-inhibition (Fig. 31A, B). To test the effect of Syk inhibition on microglia activation in a controlled in vitro system, primary microglia were exposed to anti-PD-1 with or without the Syk inhibitor Entospletinib. Thereupon it was observed that expression of PD-1, PD-L1 and MHC-II on primary microglia declined upon Syk inhibitor treatment in vitro (Fig. 7E-G). To study the impact of Syk inhibition on microglia in vivo, mice were treated with anti-PD-1 combined with vehicle or Entospletinib, which is approved for clinical use (Fig. 32A). Microglia isolated from mice treated with anti-PD-1 and Syk inhibitor exhibited lower expression of PD-1, PDL-1 and a trend towards reduced MHC-II expression compared to microglia from mice treated with anti-PD-1 and vehicle (Fig. 7H-J). The inventors observed no changes in the frequency of CD45, CD3, and F4/80+ cells in BM and spleen (Fig. 32B-G). To understand whether Syk inhibition could rescue neurocognitive deficits induced by anti-PD-1, behavior studies were performed. The novel object recognition test revealed that mice treated with anti-PD-1 plus Syk inhibitor exhibited improved memory compared to mice treated with anti-PD-1 plus vehicle (Fig. 7K). The mice treated with anti-PD-1 and Syk-inhibitor were more exploratory in the elevated plus maze test compared to the anti-PD-1 receiving vehicle (Fig. 7L). Grip strength was intact independent of the treatment with vehicle or Syk inhibitor (Fig. 7M). This effect was reproducible using a second Syk-inhibitor (Fostamatinib) (Fig. 33A-C). No changes could be observed in the peripheral myeloid compartment upon Fostamatinib treatment (Fig. 33D-G) and an expansion of CD3+ T cells in the BM and spleen upon Fostamatinib treatment (Fig. 33H and I). Notably, Syk inhibition did not compromise the anti-melanoma effect of anti-PD-1 treatment (Fig. 7N). These findings indicate that anti-PD-1 treatment induces Syk activity in microglia and that inhibition of Syk reduces microglia activation and improves cognitive function.

### Anti-PD-1 treatment causes activation of microglia in patients

To characterize the activation of microglia in humans, the inventors compared patients receiving anti-PD-1 treatment for different malignancies to a control cohort. The inventors observed an increase of Iba-1+ cells in the grey matter of the frontal lobe in patients treated with anti-PD-1 compared to the control patients (Fig. 8A and B). To analyse the grey matter in more depth, imaging mass cytometry (IMC) was performed with a panel of 37 markers and compared the two cohorts (Fig. 8C, Fig. 34A). Microglia with an amoeboid morphology and increased CD68 signal, indicating increased lysosomal activity, could be observed in patients treated with anti-PD-1 compared to controls (Fig. 8C and D). Conversely, a reduced signal of TMEM119 and P2RY12 and a weak signal of CD163 was found in the patient cohort treated with anti-PD-1 immunotherapy compared to the control cohort (Fig. 8C). Additionally, the proportion of activated microglia (Cluster 10) was increased proportionally in the anti-PD-1 patient cohort compared to the control patient cohort (Fig. 8E). The inventors further demonstrated a significant increase in CD68+ cells via immunohistochemistry in the anti-PD-1 patient cohort (Fig. 8F, Fig. 34B).These findings indicate that anti-PD-1 immunotherapy leads to microglia activation in patients, thus corroborating our findings from multiple preclinical models.

### Discussion

Anti-PD-1 immunotherapy induces significant anti-tumor immune responses with clinical success. However, the success of this therapy is complicated by irAEs which include neurological symptoms in 1-12% of all treated patients (1, 3, 4, 8). The pathophysiology of irAEs affecting the CNS following anti-PD-1 treatment is not well understood. In particular, the role of microglia in this setting remains elusive. The inventors observed that mice treated with anti-PD-1 treatment showed morphological signs of microglia activation as well as upregulation of MHC-II and CSF-1R on microglia. Comparable morphological changes and marker expression have been previously reported for microglia in CNS autoimmune inflammation (17) and CNS alloimmune inflammation (18), indicating microglia activation. The inventors observed that anti-PD-1 immunotherapy in a non-tumor setting can mediate neuroinflammation via direct drug interactions. This is in contrast to other immunotherapy induced neuroinflammatory settings, such as that observed with chimeric antigen receptor (CAR) T cells, which is dependent on the presence of tumor cells (30, 31). This could be due to major differences in the mechanisms leading to anti-PD-1 and CAR-related neuroinflammation, the latter of which relies on IL-1β and IL-6 (31), while an increase of these cytokines in microglia or other myeloid cells in the CNS of mice treated with anti-PD-1 was not observed. Microglia may be inflammatory or regulatory depending on the disease (29, 32). Single-cell profiling revealed that microglia subsets are distinctly affected in neuroinflammation (33). Herein the inventors identified a disease-promoting role for microglia in anti-PD-1 induced CNS-irAEs. Depletion of microglia, in contrast to depletion of T-cells, B-cells or NK cells, reduced anti-PD-1-induced neurocognitive deficits. This finding is consistent with the inventors observation that microglia activation by anti-PD-1 occurred independent of T cells in vitro. Our observation that anti-PD-1 directly activates microglia is consistent with a previous report showing that PD-1 deficiency in myeloid cells leads to their activation (34). Additionally, myeloid cell-specific PD-1 ablation induced increased T effector memory cell function, thereby mediating improved antitumor protection, despite preserved PD-1 expression in T cells (34). This supports a role for PD-1 in myeloid cell activation, including microglia (as shown here).

Current therapies used for irAEs after anti-PD-1 immunotherapy are based on expert opinions and small case series but not on prospective clinical trials. These include corticosteroids, mycophenolate, cyclosporine A, etanercept and extracorporeal photopheresis (12-14). Current treatment options for irAEs affecting the CNS are based on non-selective immunosuppression. A targeted therapy based on the biology of anti-PD-1-induced CNS-irAEs is highly desirable, as these immunosuppressive medications may allow malignancies to progress in patients, as reported in recent retrospective studies (1, 35, 36). Additionally, immunosuppressive drugs can cause major side-effects such as invasive infections (37). The present in-depth analysis enabled the inventors to identify Syk inhibition with Entospletinib as a novel targeted approach for CNS-irAEs. This inhibitor is presently in clinical trials for acute myeloid leukemia (ClinicalTrials.gov Identifier: NCT05020665) and chronic lymphocytic leukemia (38, 39) which substantially facilitates necessary investigational steps required for clinical use. The inventors surprising finding that Syk inhibition resulted in reduced microglia activation and improved neurocognitive activity, without blocking the anti-melanoma effects of anti-PD-1 antibody therapy, is highly clinically relevant, since Entospletinib can be tested in a clinical trial on patients with anti-PD-1 induced neurocognitive dysfunction. The inventors observation that Syk is a target for anti-PD-1 induced CNS-irAEs is consistent with a recent characterization of gain-of-function variants in SYK that cause immune dysregulation and systemic inflammation in humans and mice (40). In line with the present finding that CSF-1R inhibition reduced disease activity of CNS-irAEs, this treatment was also shown to attenuate experimental autoimmune encephalomyelitis (41). Additionally, depletion of microglia improves cognitive function following cranial irradiation (42). The depletion of microglia is not associated with major toxicity as microglia can self-renew from residual microglia after acute depletion (43). Translation of the present experimental results is particularly encouraged by the inventors finding of increased microglial activation in anti-PD-1 treated patients as compared to control patients. Additionally, reduced TMEM119 and P2RY12 expression and a weak CD163 signal in microglia of anti-PD-1 treated patients further support microglia activation (44). In summary, herein the inventors describe a novel disease-promoting role for microglia in anti-PD-1 treatment-induced neurotoxicity and identify Syk as a mediator of CNS-irAEs. Mechanistically, Syk-inhibition interfered with PD-1, PD-L1 and MHC-II expression thereby reducing the severity of CNS-irAEs in mice. The present experimental results provide a strong rationale for a pharmacological Syk inhibitor-based approach to interfere with neurocognitive impairment as a severe complication after anti-PD-1 immunotherapy.

### REFERENCES

1. T. Z. Horvat, Adel, N.G., Dang TO, Momtaz P, Postow MA, Callahan MK, Carvajal RD, Dickson MA, D'Angelo SP, Woo KM, Panageas KS, Wolchok JD, Chapman PB., Immune-related adverse events, need for systematic immunosuppression, and effects on survival and time to treatment failure in patients with melanoma treated with ipilimumab at Memorial Sloan Kettering Cancer Center. J Clin Oncol. 33, 3193 (2015).
2. L. Zimmer, Goldinger, S.M., Hofmann L, Loquai C, Ugurel S, Thomas I, Schmidgen MI, Gutzmer R, Utikal JS, Göppner D, Hassel JC, Meier F, Tietze JK, ForschnerA, Weishaupt C, Leverkus M, Wahl R, Dietrich U, Garbe C, Kirchberger MC, EigentlerT, Berking C, Gesierich A, Krackhardt AM, Schadendorf D, Schuler G, Dummer R, Heinzerling LM., Neurological, respiratory, musculoskeletal, cardiac and ocular side-effects of anti-PD-1 therapy. Eur J Cancer 60, 210 (2016).
3. K. L. Reynolds, Guidon, A.C., Diagnosis and Management of Immune Checkpoint Inhibitor-Associated Neurologic Toxicity: Illustrative Case and Review of the Literature. Oncologist 4, 435 (2019).
4. J. Larkin, Chmielowski, B., Lao CD, Hodi FS, Sharfman W, Weber J, Suijkerbuijk KPM, Azevedo S, Li H, Reshef D, Avila A, Reardon DA., Neurologic Serious Adverse Events Associated with Nivolumab Plus Ipilimumab or Nivolumab Alone in Advanced Melanoma, Including a Case Series of Encephalitis. Oncologist 6, 709 (2017).
5. A. M. Haugh, Probasco JC, Johnson DB., Neurologic complications of immune checkpoint inhibitors. Expert Opin Drug Saf. 19, 479 (2020).
6. S. Cuzzubbo, Javeri F, Tissier M, Roumi A, Barlog C, Doridam J, Lebbe C, Belin C, Ursu R, Carpentier AF., Neurological adverse events associated with immune checkpoint inhibitors: Review of the literature. Eur J Cancer 73, 1 (2017).
7. A. Farina, Birzu, C., Elsensohn MH, Picca A, Muñiz-Castrillo S, Vogrig A, Villagrán-García M, Ciano-Petersen NL, Massacesi L, Hervier B, Guégan S, Kramkimel N, Vano Y, Salem JE, Allenbach Y, Maisonobe T, Assaad S, Maureille A, Devic P, Weiss N, PegatA, Maucort-Boulch D, Ricard D, Honnorat J, Psimaras D, Joubert B., Neurological outcomes in immune checkpoint inhibitor-related neurotoxicity. Brain Commun. 5, fcad169 (2023).
8. I. Bot, Blank, C.U., Boogerd, W., Brandsma D., Neurological immune-related adverse events of ipilimumab. Pract Neurol. 13, 278 (2013).
9. J. C. Kao, Liao B, Markovic SN, Klein CJ, Naddaf E, Staff NP, Liewluck T, Hammack JE, Sandroni P, Finnes H, Mauermann ML., Neurological Complications Associated With Anti-Programmed Death 1 (PD-1) Antibodies. JAMA Neurol. 74, 1216 (2017).
10. Y. Cao, Nylander, A., Ramanan S, Goods BA, Ponath G, Zabad R, Chiang VL, Vortmeyer AO, Hafler DA, Pitt D., CNS demyelination and enhanced myelin-reactive responses after ipilimumab treatment. Neurology 86, 1553 (2016).
11. V. Pillonel, Dunet V, HottingerAF, Berthod G, Schiappacasse L, Peters S, Michielin O, Aedo-Lopez V., Multiple nivolumab-induced CNS demyelination with spontaneous resolution in an asymptomatic metastatic melanoma patient. J Immunother Cancer. 7, 336 (2019).
12. B. J. Schneider, Naidoo J, Santomasso BD, Lacchetti C, Adkins S, Anadkat M, Atkins MB, Brassil KJ, Caterino JM, Chau I, Davies MJ, Ernstoff MS, Fecher L, Ghosh M, Jaiyesimi I, Mammen JS, Naing A, Nastoupil LJ, Phillips T, Porter LD, Reichner CA, Seigel C, Song JM, Spira A, Suarez-Almazor M, Swami U, Thompson JA, Vikas P, Wang Y, Weber JS, Funchain P, Bollin K., Management of Immune-Related Adverse Events in Patients Treated With Immune Checkpoint Inhibitor Therapy: ASCO Guideline Update. J Clin Oncol. 39, 4073 (2021).
13. J. B. A. G. Haanen, Carbonnel F, Robert C, Kerr KM, Peters S, Larkin J, Jordan K; ESMO Guidelines Committee., Management of toxicities from immunotherapy: ESMO Clinical Practice Guidelines for diagnosis, treatment and follow-up. Ann Oncol. 29, iv264 (2018).
14. P. Apostolova, Unger, S., Meiss, F., von Bubnoff, D., Aumann, K., Becher, B., Zeiser, R., Extracorporeal photopheresis for severe immune checkpoint inhibitor-induced autoimmune colitis. N Eng J Med 382, 294 (2020).
15. L. Shi, Chen, S., Yang L, Li Y., The role of PD-1 and PD-L1 in T-cell immune suppression in patients with hematological malignancies. J Hematol Oncol. 6, 74 (2013).
16. J. Zhao, Roberts A, Wang Z, Savage J, Ji RR., Emerging Role of PD-1 in the Central Nervous System and Brain Diseases. Neurosci Bull. 37, 1188 (2021).
17. T. Goldmann, Wieghofer, P., Müller, P.F., Wolf, Y., Varol, D., Yona, S., Brendecke, S.M., Kierdorf, K., Staszewski, O., Datta, M., Luedde, T., Heikenwalder, M., Jung, S., Prinz, M., A new type of microglia gene targeting shows TAK1 to be pivotal in CNS autoimmune inflammation. Nat Neurosci. 16, 1618 (2013).
18. N. R. Mathew, Vinnakota, J.M., Apostolova, P., Erny, D., Hamarsheh, S., Andrieux, G., Kim, J., Hanke, K., Goldmann, T., Chappell-Maor, L., El-Khawanky, N., Ihorst, G., Schmidt, D., Duyster, J., Finke, J., Blank, T., Boerries, M., Blazar, B.R., Jung, S., Prinz, M., Zeiser, R., Graft-versus-host disease of the CNS is mediated by TNF upregulation in microglia. The Journal of clinical investigation 130, 1315 (2020).
19. G. Rao, Latha, K., Ott M, Sabbagh A, Marisetty A, Ling X, Zamler D, Doucette TA, Yang Y, Kong LY, Wei J, Fuller GN, Benavides F, Sonabend AM, Long J, Li S, Curran M, Heimberger AB., Anti-PD-1 Induces M1 Polarization in the Glioma Microenvironment and Exerts Therapeutic Efficacy in the Absence of CD8 Cytotoxic T Cells. Clin Cancer Res. 26, 4699 (2020).
20. M. P. Kummer, Ising C, Kummer C, Sarlus H, Griep A, Vieira-Saecker A, Schwartz S, Halle A, Brückner M, Händler K, Schultze JL, Beyer M, Latz E, Heneka MT., Microglial PD-1 stimulation by astrocytic PD-L1 suppresses neuroinflammation and Alzheimer's disease pathology. EMBO J. 40, e108662 (2021).
21. A. Yao, Liu, F., Chen K, Tang L, Liu L, Zhang K, Yu C, Bian G, Guo H, Zheng J, Cheng P, Ju G, Wang J., Programmed death 1 deficiency induces the polarization of macrophages/microglia to the M1 phenotype after spinal cord injury in mice. Neurotherapeutics. 11, 636 (2014).
22. M. E. Weinblatt, Kavanaugh, A., Genovese, M. C., Musser, T. K., Grossbard, E. B., Magilavy, D. B., An oral spleen tyrosine kinase (Syk) inhibitor for rheumatoid arthritis. N Engl J Med 363, 1303 (Sep 30, 2010).
23. F. Morschhauser, Dyer MJS, Walter HS, Danilov AV, Ysebaert L, Hodson DJ, Fegan C, Rule SA, Radford J, Cartron G, Bouabdallah K, Davies AJ, Spurgeon S, Rajakumaraswamy N, Li B, Humeniuk R, Huang X, Bhargava P, Jürgensmeier JM, Salles G., Phase 1b study of tirabrutinib in combination with idelalisib or entospletinib in previously treated B-cell lymphoma. Leukemia 35, 2108 (2021).
24. D. J. Andorsky, Kolibaba KS, Assouline S, Forero-TorresA, Jones V, Klein LM, Patel-Donnelly D, Smith M, Ye W, Shi W, Yasenchak CA, Sharman JP., An open-label phase 2 trial of entospletinib in indolent non-Hodgkin lymphoma and mantle cell lymphoma. Br J Haematol. 184, 215 (2019).
25. J. Portnow, Wang, D., Blanchard MS, Tran V, Alizadeh D, Starr R, Dodia R, Chiu V, Brito A, Kilpatrick J, McNamara P, Forman SJ, Badie B, Synold TW, Brown CE., Systemic Anti-PD-1 Immunotherapy Results in PD-1 Blockade on T Cells in the Cerebrospinal Fluid. JAMA Oncol. 6, 1947 (2020).
26. J. Mastroianni, Stickel, N., Andrlova, H., Hanke K, MelchingerW, Duquesne S, Schmidt D, Falk M, Andrieux G, Pfeifer D, Dierbach H, Schmitt-GraeffA, Meiss F, Boerries M, Zeiser R., miR-146a Controls Immune Response in the Melanoma Microenvironment. Cancer Res 79, 183 (2019).
27. Y. Hao, Hao S, Andersen-Nissen E, Mauck WM 3rd, Zheng S, Butler A, Lee MJ, WilkAJ, Darby C, Zager M, Hoffman P, Stoeckius M, Papalexi E, Mimitou EP, Jain J, Srivastava A, Stuart T, Fleming LM, Yeung B, Rogers AJ, McElrath JM, Blish CA, Gottardo R, Smibert P, Satija R., Integrated Analysis of Multimodal Single-Cell Data. Cell 184, 3573 (2021).
28. J. M. Rosin, Vora SR, Kurrasch DM., Depletion of embryonic microglia using the CSF1R inhibitor PLX5622 has adverse sex-specific effects on mice, including accelerated weight gain, hyperactivity and anxiolytic-like behaviour. Brain Behav Immun. 73, 682 (2018).
29. A. Mildner, Mack, M., Schmidt, H., Brück, W, Djukic, M., Zabel, M.D., Hille, A., Priller, J., Prinz, M., CCR2+Ly-6Chi monocytes are crucial for the effector phase of autoimmunity in the central nervous system. Brain 132, 2487 (2009).
30. V. S. Sheth, GauthierJ., Taming the beast: CRS and ICANS after CAR T-cell therapy for ALL. Bone Marrow Transplant 56, 552 (2021).
31. T. Giavridis, van der Stegen SJC, Eyquem J, Hamieh M, Piersigilli A, Sadelain M., CAR T cell-induced cytokine release syndrome is mediated by macrophages and abated by IL-1 blockade. Nature medicine 24, 731 (2018).
32. S. H. Cho, Sun, B., Zhou, Y., Kauppinen TM, Halabisky B, Wes P, Ransohoff RM, Gan L., CX3CR1 protein signaling modulates microglial activation and protects against plaque-independent cognitive deficits in a mouse model of Alzheimer disease. J Biol Chem. 286, 32713 (2011).
33. M. J. C. Jordão, Sankowski, R., Brendecke, S.M., Sagar, Locatelli G, Tai YH, Tay TL, Schramm E, Armbruster S, Hagemeyer N, Groß O, Mai D, Çiçek Ö, Falk T, Kerschensteiner M, Grün D, Prinz M., Single-cell profiling identifies myeloid cell subsets with distinct fates during neuroinflammation. Science 363, 6425 (2019).
34. L. Strauss, Mahmoud MAA, Weaver JD, Tijaro-Ovalle NM, ChristofidesA, Wang Q, Pal R, Yuan M, Asara J, Patsoukis N, Boussiotis VA., Targeted deletion of PD-1 in myeloid cells induces antitumor immunity. Sci Immunol. 43, eaay1863 (2020).
35. J. Luo, Beattie JA, Fuentes P, Rizvi H, Egger JV, Kern JA, Leung DYM, Lacouture ME, Kris MG, Gambarin M, Santomasso BD, Faleck DM, Hellmann MD., Beyond steroids: immunosuppressants in steroid-refractory/resistant immune related adverse events. J Thorac Oncol. 16, 1759 (2021).
36. D. Marschner, Falk M, Javorniczky NR, Hanke-Müller K, Rawluk J, Schmitt-GraeffA, Simonetta F, Haring E, Dicks S, Ku M, Duquesne S, Aumann K, Rafei-Shamsabadi D, Meiss F, Marschner P, Boerries M, Negrin RS, DuysterJ, Zeiser R, Köhler N., MicroRNA-146a regulates immune-related adverse events caused by immune checkpoint inhibitors. JCI Insight 5, 132334 (2020).
37. M. A. Postow, Sidlow R, Hellmann MD., Immune-Related Adverse Events Associated with Immune Checkpoint Blockade. N Eng J Med 378, 158 (2018).
38. A. S. Kittai, Best, S., Thurlow B, Lam V, Hashiguchi T, Goodyear S, Persky DO, Okada C, Park B, Spurgeon SE, DanilovAV. , Entospletinib and obinutuzumab in patients with relapsed/refractory chronic lymphocytic leukemia and B-cell malignancies. Haematologica 106, 2022 (2021).
39. N. Kutsch, Pallasch C, Tausch E, Böhme V, Ritgen M, Liersch R, Wacker A, Jacobs G, Trappe RU, Dreger P, Fischer K, FinkAM, Stilgenbauer S, Zhai S, Li B, JürgensmeierJM, Rajakumaraswamy N, Bhargava P, Hallek M, Eichhorst BF., Efficacy and Safety of the Combination of Tirabrutinib and Entospletinib With or Without Obinutuzumab in Relapsed Chronic Lymphocytic Leukemia. Hemasphere 6, e692 (2022).
40. L. Wang, Aschenbrenner D, Zeng Z, Cao X, Mayr D, Mehta M, Capitani M, Warner N, Pan J, Wang L, Li Q, Zuo T, Cohen-Kedar S, Lu J, Ardy RC, Mulder DJ, Dissanayake D, Peng K, Huang Z, Li X, Wang Y, Wang X, Li S, Bullers S, Gammage AN, Warnatz K, SchieferAl, Krivan G, Goda V, Kahr WHA, Lemaire M; Genomics England Research Consortium, Lu CY, Siddiqui I, Surette MG, Kotlarz D, Engelhardt KR, Griffin HR, Rottapel R, Decaluwe H, Laxer RM, Proietti M, Hambleton S, Elcombe S, Guo CH, Grimbacher B, Dotan I, Ng SC, Freeman SA, Snapper SB, Klein C, Boztug K, Huang Y, Li D, Uhlig HH, Muise AM., Gain-of-function variants in SYK cause immune dysregulation and systemic inflammation in humans and mice. Nat Genet. 53, 500 (2021).
41. J. C. Nissen, Thompson, K.K., West BL, Tsirka SE., Csf1R inhibition attenuates experimental autoimmune encephalomyelitis and promotes recovery. Exp Neurol. 307, (2018).
42. M. M. Acharya, Green KN, Allen BD, Najafi AR, Syage A, Minasyan H, Le MT, Kawashita T, Giedzinski E, PariharVK, West BL, Baulch JE, Limoli CL, Elimination of microglia improves cognitive function following cranial irradiation. Scientific reports 6, 31545 (2016).
43. Y. Huang, Xu Z, Xiong S, Sun F, Qin G, Hu G, Wang J, Zhao L, Liang YX, Wu T, Lu Z, Humayun MS, So KF, Pan Y, Li N, Yuan TF, Rao Y, Peng B., Repopulated microglia are solely derived from the proliferation of residual microglia after acute depletion. Nat Neurosci. 21, 530 (2018).
44. M. Schwabenland et al., Analyzing microglial phenotypes across neuropathologies: a practical guide. Acta Neuropathol 142, 923 (Dec, 2021).
45. Liu, D., Mamorska-Dyga, A. Syk inhibitors in clinical development for hematological malignancies. J Hematol Oncol 10, 145 (2017). https://doi.org/10.1186/s13045-017-0512-1.
46. Luis A. Carvajal, Michael McKeown, Tressa Hood, Pavan Kumar, Douglas C. Saffran, Jorge DiMartino; SYK Inhibitors, Entospletinib and Lanraplenib, Show Potent Anti-Leukemic Activity in Combination with Targeted Agents. Blood 2022; 140 (Supplement 1): 5932-5933.
47. Mullard, A. FDA approves first-in-class SYK inhibitor. Nat Rev Drug Discov 17, 385 (2018). https://doi.org/10.1038/nrd.2018.96.
48. Kim, M.W.; Choe, K.; Park, J.S.; Lee, H.J.; Kang, M.H.; Ahmad, R.; Kim, M.O.. Cells 2022, 11, 1777. https://doi.org/10.3390/cells11111777.

## Claims

1. An inhibitor of Spleen tyrosine kinase (Syk-inhibitor) for use in the treatment or prevention of immune checkpoint inhibition (ICI)-induced immune-related adverse events (irAEs) in a subject.

2. The Syk-inhibitor for use according to claim 1, wherein irAEs comprise or consist of central nervous system immune-related adverse events (CNS-irAEs).

3. The Syk-inhibitor for use according to any one of the preceding claims, wherein the subject is receiving immune checkpoint blockade therapy.

4. The Syk-inhibitor for use according to any one of the preceding claims, wherein the checkpoint blockade therapy comprises administration of at least one antibody.

5. The Syk-inhibitor for use according to any one of the preceding claims, wherein the at least one antibody is specific for a protein selected from the group comprising cytotoxic T-lymphocyte antigen 4 (CTLA-4), programmed cell death-1 (PD-1) and/or programmed cell death ligand-1 (PD-L1) or Lymphocyte-activation gene 3 (LAG-3).

6. The Syk-inhibitor for use according to any one of the preceding claims, wherein the Syk-inhibitor is administered at least 1 hour before, during and/or at least one hour after administration of immune checkpoint blockade therapy.

7. The Syk-inhibitor for use according to any one of the preceding claims, wherein the administration of the Syk-inhibitor does not interfere with the anti-tumor response induced by the immune checkpoint blockade therapy, which is preferably an anti-PD1 treatment, due to the tissue specific adiponectin induction.

8. The Syk-inhibitor for use according to any one of the preceding claims, wherein the Syk-inhibitor reduces microglial activation in said subject.

9. The Syk-inhibitor for use according to any one of the preceding claims, wherein the microglial activation comprises microglia exhibiting, when compared to microglia cells of a healthy control subject, an elevated expression of MHC-II, F4/80, CD80 and/or CSF-1R, and/or morphological changes comprising reduced filament length, reduced number of dendrites, branching points, terminal dendrite points and/or Syk activation (phosphorylation).

10. The Syk-inhibitor for use according to any one of the preceding claims, wherein the inhibitor is administered to a subject upon occurrence of one or more of cognitive deficits, neurotoxicity, seizures, pathological headaches, increased blood brain barrier (BBB) permeability, CNS infiltration of immune cells, partial or complete loss of eye sight, partial or complete loss of hearing, partial or complete loss of sensation, partial or complete paralysis, muscle weakness, memory loss, numbness in one or more limbs, tremor, paresis, confusion or disorientation, toxicities of cytokine release syndrome (CRS), neurotoxicity and/or neurocognitive defects in said subject.

11. The Syk-inhibitor for use according to any one of the preceding claims, wherein the inhibitor is selected from fostamatinib (R788), entospletinib (GS-9973), lanraplenib (GS-9876), cerdulatinib, R211, R406, R343, R112, BAY-61-3606, GSK143, SKI-G-618, SKI-O-85, ER-27319, YM193306, RO9021 and P505-15 (PRT062607).
